# EUROPEAN PATENT APPLICATION

(11) **EP 4 230 645 A1**
(43) Date of publication of application: **23.08.2023**
(21) Application number: 22158021.0
(22) Date of filing: 22.02.2022
(51) Int. Cl.: C07K 14/47

(54) **PEPTIDIC INHIBITORS OF AMYLOID SELF- AND CROSS-ASSEMBLY**

(71) Applicant: Technische Universität München, 80333 München (DE)
(72) Inventor: The designation of the inventor has not yet been filed
(74) Representative: Engelhard, Markus

(57) **Abstract**

The present invention relates to a peptide, and to a pharmaceutical composition and a heterocomplex comprising the peptide. Furthermore, the present invention relates to the peptide, the pharmaceutical composition, or the heterocomplex for use in a method of preventing or treating Alzheimer's disease and/or for use in a method of preventing or treating type 2 diabetes. The present invention further relates to the peptide, the pharmaceutical composition, or the heterocomplex for use in a method of diagnosing Alzheimer's disease and/or for use in a method of diagnosing type 2 diabetes. The present invention also relates to a kit for the *in vitro* or *in vivo* detection of amyloid fibrils or aggregates, or for the diagnosis of Alzheimer's disease and/or type 2 diabetes in a patient. Moreover, the present invention relates to the use of the peptide or of the heterocomplex in an *in vitro* assay for the detection of monomeric islet amyloid polypeptide (IAPP), monomeric Aβ40(42), amyloid fibrils, or amyloid aggregates.

## Description

### FIELD OF THE INVENTION

The present invention relates to a peptide, and to a pharmaceutical composition and a heterocomplex comprising the peptide. Furthermore, the present invention relates to the peptide, the pharmaceutical composition, or the heterocomplex for use in a method of preventing or treating Alzheimer's disease and/or for use in a method of preventing or treating type 2 diabetes. The present invention further relates to the peptide, the pharmaceutical composition, or the heterocomplex for use in a method of diagnosing Alzheimer's disease and/or for use in a method of diagnosing type 2 diabetes. The present invention also relates to a kit for the *in vitro* or in *vivo* detection of amyloid fibrils or aggregates, or for the diagnosis of Alzheimer's disease and/or type 2 diabetes in a patient. Moreover, the present invention relates to the use of the peptide or of the heterocomplex in an *in vitro* assay for the detection of monomeric islet amyloid polypeptide (IAPP), monomeric Aβ40(42), amyloid fibrils, or amyloid aggregates.

### BACKGROUND OF THE INVENTION

Amyloid self-assembly is linked to numerous devastating cell-degenerative diseases with Alzheimer's disease (AD) and type 2 diabetes (T2D) being two of the most prominent ones. The main component of amyloid plaques in AD brains is the 40(42)-residue peptide Aβ40(42), while pancreatic amyloid of T2D patients consists of fibrillar assemblies of the 37-residue IAPP. IAPP is secreted from pancreatic β-cells and functions as a neuroendocrine regulator of glucose homeostasis. However, formation of cytotoxic IAPP assemblies and amyloid fibrils mediates pancreatic β-cell degeneration in T2D.

Epidemiological studies suggest that T2D patients have an increased risk of AD and *vice versa.* In addition, increasing evidence suggests molecular and pathophysiological links between both diseases. Cross-interactions between Aβ and IAPP could be such molecular links. In fact, polymorphic Aβ/IAPP interactions are able to cross-seed or cross-suppress amyloid self-assembly depending on structures and self-assembly states of the interacting polypeptides. To this end, IAPP and Aβ fibrils act as reciprocal cross-seeds of amyloid self-assembly as shown by both *in vitro* and experimental in *vivo* studies. On the other hand, nanomolar affinity interactions between early pre-fibrillar and non-toxic IAPP and Aβ species redirect both polypeptides into initially non-fibrillar and non-toxic co-assemblies thus delaying amyloid self-assembly. Importantly, Aβ and IAPP were found to colocalize in AD- and T2D-related amyloid deposits both in humans and in mouse models. Aβ/IAPP cross-interactions and putative "hetero-amyloids" could thus be highly relevant to the pathogenesis of both diseases.

Based on the above, molecules targeting amyloid self-assembly and reciprocal cross-seeding effects of IAPP and Aβ are highly promising for anti-amyloid treatments in both AD and T2D. However, so far only few inhibitors of amyloid self-assembly of both polypeptides (termed "cross-amyloid" inhibitors) have been reported and none of them suppressed reciprocal Aβ/IAPP cross-seeding. Moreover, except for a recently approved anti-Aβ amyloid antibody, no anti-amyloid treatments for AD or T2D have yet reached the clinic.

Peptides derived from the IAPP amyloid core IAPP(8-28) as IAPP interaction surface mimics (ISMs) have been designed [1]. ISMs suppressed amyloid self-assembly of Aβ40(42) and/or IAPP by sequestering them into amorphous, non-toxic aggregates. However, none of the reported inhibitors of amyloid self-assembly of Aβ40(42) and/or IAPP has yet advanced to the clinic.

Conclusively, effective inhibitors of amyloid self-assembly are needed. Particularly, there is a need for new inhibitors of amyloid self-assembly of Aβ40(42) and/or IAPP. Furthermore, there remains the need for inhibitors of IAPP and Aβ cross-seeding. There is also a need to provide inhibitors of amyloid self- and cross-assembly that can be used to prevent, treat, and/or diagnose Alzheimer's disease and/or type 2 diabetes.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a peptide having an amino acid sequence according to formula 1

(X₁)ₘ₍X₂)ₙ(X₃)ₚ(X₄)_{q}*FₐF_{b}AE*X₅-X₆X₇X₈-*NKGAII*X₉X₁₀X₁₁*VG(G)ᵣ(V)ₛ(V)ₜ* (formula 1)

wherein,
m, n, p, q, r, s, and t are, independently at each occurrence, selected from 0 and 1;
X₁ is selected from glutamine, asparagine, alanine, glutamic acid, and aspartic acid, preferably is glutamine;
X₂ is selected from lysine, modified lysine e.g. Lys(Ac), Lys(N3), and Lys(biotin), alanine, modified alanine, ornithine, modified ornithine e.g. Orn(Ac), diaminobutyric acid, diaminopropionic acid, arginine, and modified arginine e.g. Arg(Me)2, preferably is lysine; X₃ is selected from leucine, N-methyl-leucine, alanine, N-methyl-alanine, isoleucine, N-methyl-isoleucine, valine, N-methyl-valine, glycine, N-methyl-glycine, norleucine, N-methyl-norleucine, phenylalanine, N-methyl-phenylalanine, tyrosine, and N-methyl-tyrosine, preferably is leucine or N-methyl-leucine;
X₄ is selected from leucine, N-methyl-leucine, alanine, N-methyl-alanine, isoleucine, N-methyl-isoleucine, valine, N-methyl-valine, glycine, N-methyl-glycine, norleucine, N-methyl-norleucine, phenylalanine, N-methyl-phenylalanine, tyrosine, and N-methyl-tyrosine, preferably is valine or N-methyl-valine;
*Fₐ* and *F_{b}* are, independently at each occurrence, selected from phenylalanine, N-methyl-phenylalanine, halogenated phenylalanine, tyrosine, and N-methyl-tyrosine, preferably selected from phenylalanine and N-methyl-phenylalanine;
A is, independently at each occurrence, selected from alanine, N-methyl-alanine, leucine, N-methyl-leucine, valine, N-methyl-valine, glycine, and N-methyl-glycine, preferably is alanine; E is selected from glutamic acid, N-methyl-glutamic acid, aspartic acid, N-methyl-aspartic acid, glutamine, N-methyl-glutamine, asparagine, N-methyl-asparagine, alanine, N-methyl-alanine, glycine, and N-methyl-glycine, preferably is glutamic acid;
X₅ is selected from aspartic acid, glutamic acid, asparagine, aspartic acid, alanine, and glycine, preferably is aspartic acid;
X₆, X₇, and X₈ are, independently at each occurrence, selected from norleucine, leucine, phenylalanine, methionine, tryptophan, tyrosine, threonine, alanine, isoleucine, valine, and modified forms thereof e.g. N-methylated forms thereof, preferably selected from norleucine, leucine, and phenylalanine;
*N* is selected from asparagine, glutamine and alanine, preferably is asparagine;
*K* is selected from lysine, modified lysine e.g. Lys(Ac), Lys(N3), and Lys(biotin), alanine, norleucine, ornithine, modified ornithine e.g. Orn(Ac), diaminobutyric acid, diaminopropionic acid, arginine, and modified arginine e.g. Arg(Me)2, preferably is lysine;
G is, independently at each occurrence, selected from glycine and alanine, preferably is glycine;
I is, independently at each occurrence, selected from isoleucine, leucine, norleucine, and valine, preferably is isoleucine;
X₉ is selected from glycine, alanine, and serine, preferably is glycine;
X₁₀ is selected from leucine, alanine, isoleucine, threonine, norleucine, and valine, preferably is leucine;
X₁₁ is selected from norleucine, leucine, phenylalanine, methionine, tryptophan, tyrosine, threonine, alanine, isoleucine, and valine, preferably is norleucine; and V is, independently at each occurrence, valine;
wherein, preferably, at least one of X₃, X₄, Fₐ, and F_{b} is an N-methylated amino acid;
and pharmaceutically acceptable salts, esters, solvates, polymorphs and modified forms thereof.

In one embodiment, the peptide according to the present invention has an amino acid sequence according to formula 2

(X₁)ₘ(X₂)ₙ(*Lₐ*)ₚ(*Vₐ*)_{q}*FₐF_{b}AE*X₅-X₆X₇X₈*-NKGAII*X₉X₁₀X₁₁*VG(G)ᵣ(V)ₛ(V)ₜ* (formula 2)

wherein,
m, n, p, q, r, s, and t are, independently at each occurrence, selected from 0 and 1;
X₁ is selected from glutamine, asparagine, alanine, glutamic acid, and aspartic acid, preferably is glutamine;
X₂ is selected from lysine, modified lysine e.g. Lys(Ac), Lys(N3), and Lys(biotin), alanine, modified alanine, ornithine, modified ornithine e.g. Orn(Ac), diaminobutyric acid, diaminopropionic acid, arginine, and modified arginine e.g. Arg(Me)2, preferably is lysine; *Lₐ* is leucine or N-methyl-leucine;
*Vₐ* is valine or N-methyl-valine;
*Fₐ* and *F_{b}* are, independently at each occurrence, selected from phenylalanine, N-methyl-phenylalanine, halogenated phenylalanine, tyrosine, and N-methyl-tyrosine, preferably selected from phenylalanine and N-methyl-phenylalanine;
A is, independently at each occurrence, selected from alanine, N-methyl-alanine, leucine, N-methyl-leucine, valine, N-methyl-valine, glycine, and N-methyl-glycine, preferably is alanine;
E is selected from glutamic acid, N-methyl-glutamic acid, aspartic acid, N-methyl-aspartic acid, glutamine, N-methyl-glutamine, asparagine, N-methyl-asparagine, alanine, N-methyl-alanine, glycine, and N-methyl-glycine, preferably is glutamic acid;
X₅ is selected from aspartic acid, glutamic acid, asparagine, aspartic acid, alanine, and glycine, preferably is aspartic acid;
X₆, X₇, and X₈ are, independently at each occurrence, selected from norleucine, leucine, phenylalanine, methionine, tryptophan, tyrosine, threonine, alanine, isoleucine, valine, and modified forms thereof e.g. N-methylated forms thereof, preferably selected from norleucine, leucine, and phenylalanine;
*N* is selected from asparagine, glutamine and alanine, preferably is asparagine;
*K* is selected from lysine, modified lysine e.g. Lys(Ac), Lys(N3), and Lys(biotin), alanine, norleucine, ornithine, modified ornithine e.g. Orn(Ac), diaminobutyric acid, diaminopropionic acid, arginine, and modified arginine e.g. Arg(Me)2, preferably is lysine;
G is, independently at each occurrence, selected from glycine and alanine, preferably is glycine;
I is, independently at each occurrence, selected from isoleucine, leucine, norleucine, and valine, preferably is isoleucine;
X₉ is selected from glycine, alanine, and serine, preferably is glycine;
X₁₀ is selected from leucine, alanine, isoleucine, threonine, norleucine, and valine, preferably is leucine; and
X₁₁ is selected from norleucine, leucine, phenylalanine, methionine, tryptophan, tyrosine, threonine, alanine, isoleucine, and valine, preferably is norleucine;
V is, independently at each occurrence, valine;
wherein at least one of *Lₐ*, *Vₐ*, *Fₐ,* and *F_{b}* is an N-methylated amino acid;
and pharmaceutically acceptable salts, esters, solvates, polymorphs and modified forms thereof.

In one embodiment, the peptide of the invention has an amino acid sequence according to any one of formulae 3-5

QK*L*ₐ*VₐFₐF_{b}*AED-X₆X₇X₈-NKGAIIGLNleVGGVV (formula 3)

*FₐF_{b}*AED-X₆X₇X₈-NKGAIIGLNleVGGVV (formula 4)

QK*LₐVₐFₐF_{b}*AED-X₆X₇X₈-NKGAIIGLNleVG (formula 5)

wherein,
Q is glutamine;
K is, independently at each occurrence, lysine;
*Lₐ* is leucine or N-methyl-leucine;
*Vₐ* is valine or N-methyl-valine;
*Fₐ* and *F_{b}* are, independently at each occurrence, phenylalanine or N-methyl-phenylalanine;
A is, independently at each occurrence, alanine;
E is glutamic acid;
D is aspartic acid;
X₆, X₇, and X₈ are, independently at each occurrence, selected from norleucine, leucine, phenylalanine, methionine, tryptophan, tyrosine, threonine, alanine, isoleucine, and valine, preferably selected from norleucine, leucine, and phenylalanine;
N is asparagine;
G is, independently at each occurrence, glycine;
I is, independently at each occurrence, isoleucine;
L is leucine;
Nle is norleucine;
V is, independently at each occurrence, valine;
wherein at least one of *Lₐ*, *Vₐ*, *Fₐ,* and *F_{b}* is an N-methylated amino acid;
and pharmaceutically acceptable salts, esters, solvates, polymorphs and modified forms thereof.

In an embodiment of the peptide, X₆, X₇, and X₈ are the same amino acid selected from norleucine, leucine, phenylalanine, methionine, tryptophan, tyrosine, threonine, alanine, isoleucine, and valine, preferably selected from norleucine, leucine, and phenylalanine.

In one embodiment, the peptide of the present invention has an amino acid sequence according to any one of formulae 6-8,

QK*LₐVₐFₐF_{b}*AEDNleNleNleNKGAllGLNleVGGVV (formula 6),

QK*LₐVₐFₐF_{b}*AEDNleNleNleNKGAllGLNleVGGVV (formula 7),

QK*LₐVₐFₐF_{b}*AEDNleNleNleNKGAllGLNleVGGVV (formula 8),

wherein,
- Q: is glutamine;
- K: is, independently at each occurrence, lysine;
- *Lₐ*: is leucine or N-methyl-leucine;
- *Vₐ*: is valine or N-methyl-valine;
- *Fₐ* and *F_{b}*: are, independently at each occurrence, phenylalanine or N-methyl-phenylalanine;
- A: is, independently at each occurrence, alanine;
- E: is glutamic acid;
- D: is aspartic acid;
- Nle: is norleucine;
- N: is asparagine;
- G: is, independently at each occurrence, glycine;
- I: is, independently at each occurrence, isoleucine;
- L: is, independently at each occurrence, leucine;
- V: is, independently at each occurrence, valine;
- F: is, independently at each occurrence, phenylalanine;

wherein at least one of *Lₐ*, *Vₐ, Fₐ,* and *F_{b}* is an N-methylated amino acid;
and pharmaceutically acceptable salts, esters, solvates, polymorphs and modified forms thereof.

In an embodiment, two or more of *Lₐ*, *Vₐ, Fₐ,* and *F_{b}* are an N-methylated amino acid;
wherein, preferably, *Lₐ* and *Vₐ; Lₐ* and *Fₐ; Lₐ* and F_{b}; *Vₐ* and *Fₐ; Vₐ* and *F_{b}*; *Fₐ* and *F_{b}*; *Lₐ*, *Vₐ,* and *Fₐ ; Lₐ*, *Vₐ,* and *F_{b}*; *Lₐ*, *Fₐ,* and F_{b}; *Vₐ, Fₐ,* and F_{b}; or *Lₐ*, *Vₐ, Fₐ,* and *F_{b}* are N-methylated amino acids;
wherein, more preferably, at least *Lₐ* and *Fₐ,* or at least *Vₐ* and F_{b}, are an N-methylated amino acid.

In an embodiment, said peptide has an amino acid sequence according to any one of formulae 9-17,

QK(N-methyl-L)V(N-methyl-F)FAEDNleNleNleNKGAIIGLNleVGGVV (formula 9),

QKL(N-methyl-V)F(N-methyl-F)AEDNleNleNleNKGAIIGLNleVGGVV (formula 10),

QK(N-methyl-L)V(N-methyl-F)FAEDLLLNKGAIIGLNleVGGVV (formula 11),

QKL(N-methyl-V)F(N-methyl-F)AEDLLLNKGAIIGLNleVGGVV (formula 12),

QK(N-methyl-L)V(N-methyl-F)FAEDFFFNKGAIIGLNleVGGVV (formula 13),

QKL(N-methyl-V)F(N-methyl-F)AEDFFFNKGAIIGLNleVGGVV (formula 14),

QK(N-methyl-L)(N-methyl-V)(N-methyl-F)(N-methyl-F)AEDFFFNKGAIIGLNleVGGVV (formula 15),

QK(N-methyl-L)(N-methyl-V)(N-methyl-F)(N-methyl-F)AEDLLLNKGAIIGLNleVGGVV (formula 16),

QK(N-methyl-L)(N-methyl-V)(N-methyl-F)(N-methyl-F)AEDNleNleNleNKGAIIGLNleVGGVV (formula 17),

wherein,
- Q: is glutamine;
- K: is, independently at each occurrence, lysine;
- L: is, independently at each occurrence, leucine;
- V: is, independently at each occurrence, valine;
- F: is, independently at each occurrence, phenylalanine;
- A: is, independently at each occurrence, alanine;
- E: is glutamic acid;
- D: is aspartic acid;
- Nle: is, independently at each occurrence, norleucine;
- N: is asparagine;
- G: is, independently at each occurrence, glycine;
- I: is, independently at each occurrence, isoleucine;
and pharmaceutically acceptable salts, esters, solvates, polymorphs and modified forms thereof.

In an embodiment, said peptide consists of a sequence according to any one of formulae 1-17.

In a further aspect, the present invention also relates to a composition comprising a peptide according to the present invention, as defined herein, and a suitable solvent, such as water, and a buffer.

In a further aspect, the present invention also relates to a pharmaceutical composition comprising a peptide according to the present invention, as defined herein, and a pharmaceutically acceptable excipient.

In a further aspect, the present invention further relates to a heterocomplex comprising a peptide according to the present invention, as defined herein, and amyloid-β peptide and/or islet amyloid polypeptide.

In a further aspect, the present invention further relates to a peptide according to the present invention as defined herein, the pharmaceutical composition according to the present invention as defined herein, or the heterocomplex according to the present invention as defined herein, for use in a method of preventing or treating Alzheimer's disease and/or for use in a method of preventing or treating type 2 diabetes.

In an embodiment, said method comprises administering an effective amount of said peptide, of said composition, or of said heterocomplex to a subject in need thereof.

In a further aspect, the present invention further relates to a peptide according to the present invention as defined herein, the pharmaceutical composition according to the present invention as defined herein, or the heterocomplex according to the present invention as defined herein, for use in a method of diagnosing Alzheimer's disease and/or for use in a method of diagnosing type 2 diabetes;

In an embodiment, said method comprises administering an effective amount of said peptide, of said composition, or of said heterocomplex to a subject to be tested for Alzheimer's disease and/or type 2 diabetes.

In one embodiment of such peptide, pharmaceutical composition, or heterocomplex for use, said peptide is linked to or administered together with a suitable reporter molecule that allows detection of Aβ40(42), islet amyloid polypeptide (IAPP), and/or amyloid aggregates or co-aggregates thereof by a suitable detection methodology, such as positron emission tomography (PET), nuclear magnetic resonance (NMR), magnetic resonance imaging (MRI), and PET-MRI and wherein said subject, after administration of said peptide, is subjected to said suitable detection methodology, such as PET, NMR, MRI, PET-MRI.

In a yet a further aspect, the present invention relates to a kit for the *in vitro* or in *vivo* detection of amyloid fibrils or aggregates, or for the diagnosis of Alzheimer's disease and/or type 2 diabetes in a subject, said kit comprising the peptide according to the present invention as defined above, the pharmaceutical composition according to the present invention as defined above, or the heterocomplex according to the present invention as defined above, in a freeze-dried form in a suitable container, a buffered solvent in a separate container for reconstitution of said peptide in solution, and, optionally, means to dispense said peptide once reconstituted in solution, such as a syringe or pipette.

In a yet a further aspect, the present invention also relates to the use of the peptide according to the present invention as defined above or of the heterocomplex according to the present invention as defined above, in an *in vitro* assay, such as an enzyme linked immunosorbent assay (ELISA), a radioimmuno assay (RIA), or a Dot/Slot blot assay, for the detection of monomeric islet amyloid polypeptide (IAPP), monomeric Aβ40(42), amyloid fibrils, amyloid aggregates, and/or amyloid co-aggregates.

In a further aspect, the present invention relates to the use of a peptide according to the present invention as defined above, a pharmaceutical composition according to the present invention as defined above, or a heterocomplex according to the present invention as defined above, for the manufacture of a medicament for the treatment or diagnosis of Alzheimer's disease and/or of type 2 diabetes.

In yet a further aspect, the present invention also relates to a method of prevention, treatment, or diagnosis of Alzheimer's disease and/or type 2 diabetes, wherein said method comprises administering an effective amount of said peptide according to the present invention as defined above, said pharmaceutical composition according to the present invention as defined above, or said heterocomplex according to the present invention as defined above, to a subject in need thereof or to a subject to be tested.

### DETAILED DESCRIPTION

Amyloid self-assembly is linked to numerous devastating cell-degenerative diseases. However, designing inhibitors of this pathogenic process remains a major challenge. Cross-interactions between amyloid-β peptide (Aβ) and islet amyloid polypeptide (IAPP), key polypeptides of Alzheimer's disease (AD) and type 2 diabetes (T2D), link AD with T2D pathogenesis. The present inventors herein show that peptides designed to mimic the amyloid core of Aβ (ACMs) are nanomolar cross-amyloid inhibitors of both IAPP and Aβ42 and effectively suppress reciprocal cross-seeding. Surprisingly, ACMs act by co-assembling with IAPP or Aβ42 into amyloid-like but non-toxic nanofibers and their highly ordered superstructures. Co-assembled nanofibers exhibit various beneficial features including thermolability, proteolytic degradability, and effective cellular clearance which are reminiscent of labile/reversible functional amyloids. ACMs thus provide potent anti-amyloid drugs in both T2D and AD while the supramolecular nanofiber co-assemblies inform the design of novel functional (hetero-)amyloid-based nanomaterials for biomedical/biotechnological applications.

The herein disclosed peptides are derived from the Aβ40 amyloid core Aβ(15-40) as Aβ amyloid core mimics (ACMs) and inhibitors of amyloid self-assembly and cross-seeding interactions of IAPP and Aβ42. In one embodiment, the term "ACM" as used herein, relates to a peptide of the invention. The inhibitor design concept aimed at distorting the pathogenic fibril fold of Aβ(15-40) and stabilize alternative, amyloid-like but non-amyloidogenic folds. The inventors aimed at yielding interaction surfaces with IAPP or Aβ42 and redirecting them into non-fibrillar and non-toxic aggregates. A series of conformationally constrained peptides was synthesized and studied. Unexpectedly, ACMs were non-amyloidogenic and non-cytotoxic, bound IAPP and Aβ42 with nanomolar affinity and fully blocked their cytotoxic amyloid self-assembly. Furthermore, ACMs effectively suppressed reciprocal cross-seeding effects. Surprisingly, ACMs exerted their inhibitory function by co-assembling with IAPP or Aβ42 into amyloid-like nanofibers and their diverse highly ordered superstructures. For their characterization, a spectrum of biophysical, biochemical, and advanced microscopy methods including CLSM, STED, 2PM and FLIM-FRET was applied. In addition, *in vitro* and *ex vivo* cell-based assays were used. In strong contrast to IAPP or Aβ42 fibrils (fIAPP or fAβ42), co-assembled nanofibers were "ThT-invisible", non-cytotoxic, and seeding-incompetent. Moreover, they were thermolabile, easily degradable by proteinase K (PK), and became efficiently phagocytosed *in vitro* by primary macrophages and cultured microglial cells. Conclusively, the peptides of the present invention are highly useful in the prevention and treatment of Alzheimer's disease and type 2 diabetes. ThT is a dye which typically stains pathogenic amyloid fibrils but not the new type of heterocomplexes, e.g. hetero-nanofibers, which are generated by interaction of ACMs with IAPP or Abeta42. The fact that the heterocomplexes do not bind ThT supports the observed differences in their properties and functions (e.g. the heterocomplexes being non-toxic, becoming easier proteolytically degraded, phagocytosed etc). The products of the interactions of ACMs with IAPP and Abeta42 have the advantage that they can be removed by proteolytic degradation, in contrast to IAPP and Abeta42 fibrils. Furthermore, the differential staining properties are useful for diagnostic assays.

The present invention provides highly effective inhibitors of amyloid self-assembly of Aβ40(42) and/or IAPP and amyloid cross-assembly. Surprisingly, the peptides of the invention inhibits not only Aβ40(42) and/or IAPP self-assembly, but also Aβ40(42) and IAPP cross-assembly. Thus, the present invention provides peptides, and compositions and heterocomplexes comprising these peptides, which are effective inhibitors of amyloid self- and cross-assembly that can be used to prevent, treat, and/or diagnose Alzheimer's disease and/or type 2 diabetes.

In one embodiment, the peptide of the invention has an amino acid sequence according to formula 1

(X₁)ₘ₍X₂)ₙ(X₃)ₚ(X₄)_{q}*FₐF_{b}AE*X₅-X₆X₇X₈-NKGAIIX₉X₁₀X₁₁*VG(G)ᵣ(V)ₛ(V)ₜ* (formula 1)

wherein,
m, n, p, q, r, s, and t are, independently at each occurrence, selected from 0 and 1;
X₁ is selected from glutamine, asparagine, alanine, glutamic acid, and aspartic acid, preferably is glutamine;
X₂ is selected from lysine, modified lysine e.g. Lys(Ac), Lys(N3), and Lys(biotin), alanine, modified alanine, ornithine, modified ornithine e.g. Orn(Ac), diaminobutyric acid, diaminopropionic acid, arginine, and modified arginine e.g. Arg(Me)2, preferably is lysine;
X₃ is selected from leucine, N-methyl-leucine, alanine, N-methyl-alanine, isoleucine, N-methyl-isoleucine, valine, N-methyl-valine, glycine, N-methyl-glycine, norleucine, N-methyl-norleucine, phenylalanine, N-methyl-phenylalanine, tyrosine, and N-methyl-tyrosine, preferably is leucine or N-methyl-leucine;
X₄ is selected from leucine, N-methyl-leucine, alanine, N-methyl-alanine, isoleucine, N-methyl-isoleucine, valine, N-methyl-valine, glycine, N-methyl-glycine, norleucine, N-methyl-norleucine, phenylalanine, N-methyl-phenylalanine, tyrosine, and N-methyl-tyrosine, preferably is valine or N-methyl-valine;
*Fₐ* and *F_{b}* are, independently at each occurrence, selected from phenylalanine, N-methyl-phenylalanine, halogenated phenylalanine, tyrosine, and N-methyl-tyrosine, preferably selected from phenylalanine and N-methyl-phenylalanine;
A is, independently at each occurrence, selected from alanine, N-methyl-alanine, leucine, N-methyl-leucine, valine, N-methyl-valine, glycine, and N-methyl-glycine, preferably is alanine;
E is selected from glutamic acid, N-methyl-glutamic acid, aspartic acid, N-methyl-aspartic acid, glutamine, N-methyl-glutamine, asparagine, N-methyl-asparagine, alanine, N-methyl-alanine, glycine, and N-methyl-glycine, preferably is glutamic acid;
X₅ is selected from aspartic acid, glutamic acid, asparagine, aspartic acid, alanine, and glycine, preferably is aspartic acid;
X₆, X₇, and X₈ are, independently at each occurrence, selected from norleucine, leucine, phenylalanine, methionine, tryptophan, tyrosine, threonine, alanine, isoleucine, valine, and modified forms thereof e.g. N-methylated forms thereof, preferably selected from norleucine, leucine, and phenylalanine;
*N* is selected from asparagine, glutamine and alanine, preferably is asparagine;
*K* is selected from lysine, modified lysine e.g. Lys(Ac), Lys(N3), and Lys(biotin), alanine, norleucine, ornithine, modified ornithine e.g. Orn(Ac), diaminobutyric acid, diaminopropionic acid, arginine, and modified arginine e.g. Arg(Me)2, preferably is lysine;
G is, independently at each occurrence, selected from glycine and alanine, preferably is glycine;
I is, independently at each occurrence, selected from isoleucine, leucine, norleucine, and valine, preferably is isoleucine;
X₉ is selected from glycine, alanine, and serine, preferably is glycine;
X₁₀ is selected from leucine, alanine, isoleucine, threonine, norleucine, and valine, preferably is leucine;
X₁₁ is selected from norleucine, leucine, phenylalanine, methionine, tryptophan, tyrosine, threonine, alanine, isoleucine, and valine, preferably is norleucine; and
V is, independently at each occurrence, valine;
wherein, preferably, at least one of X₃, X₄, *Fₐ,* and *F_{b}* is an N-methylated amino acid;
and pharmaceutically acceptable salts, esters, solvates, polymorphs and modified forms thereof.

In a preferred embodiment of the peptide according to formula 1, at least one of X₃, X₄, *Fₐ,* and *F_{b}* is an N-methylated amino acid, e.g. any one, two, three, or all of X₃, X₄, *Fₐ,* and *F_{b}* are an N-methylated amino acid. In one embodiment, two or more of X₃, X₄, *Fₐ,* and *F_{b}* are an N-methylated amino acid, for example X₃ and *Fₐ;* X₄ and *F_{b}*; or X₃, X₄, *Fₐ,* and *F_{b}* are an N-methylated amino acid. In a preferred embodiment, two or more of X₃, X₄, *Fₐ,* and *F_{b}* are an N-methylated amino acid. In one embodiment, X₃ and X₄; X₃ and *Fₐ;* X₃ and *F_{b}*; X₃ and *Fₐ;* X₃ and *F_{b}*; *Fₐ* and *F_{b}*; X₃, X₄, and *Fₐ* ; X₃, X₄, and *F_{b}*; X₃, *Fₐ,* and *F_{b}*; X₄, *Fₐ,* and *F_{b}*; or X₃, X₄ *Fₐ,* and *F_{b}* are N-methylated amino acids. In one embodiment, at least X₃ and *Fₐ,* or at least X₄ and *F_{b}*, are an N-methylated amino acid.

In a preferred embodiment of the peptide according to any one of formulae 2-8, at least one of *Lₐ*, *Vₐ, Fₐ,* and *F_{b}* is an N-methylated amino acid, e.g. any one, two, three, or all of *Lₐ*, *Vₐ, Fₐ,* and *F_{b}* are an N-methylated amino acid. In one embodiment, two or more of *Lₐ*, *Vₐ, Fₐ,* and *F_{b}* are an N-methylated amino acid, for example *Lₐ* and Fₐ; *Vₐ* and F_{b}; or *Lₐ*, *Vₐ, Fₐ,* and *F_{b}* are an N-methylated amino acid. In a preferred embodiment, two or more of *Lₐ*, *Vₐ, Fₐ,* and *F_{b}* are an N-methylated amino acid. In one embodiment, *Lₐ* and *Vₐ; Lₐ* and *Fₐ*; *Lₐ* and F_{b}; *Lₐ* and *Fₐ; Lₐ* and *F_{b}*; *Fₐ* and *F_{b}*; *Lₐ*, *Vₐ,* and *Fₐ ; Lₐ*, *Vₐ,* and *F_{b}*; *Lₐ*, *Fₐ,* and F_{b}; *Vₐ, Fₐ,* and *F_{b}*; or *Lₐ*, *Vₐ, Fₐ,* and *F_{b}* are N-methylated amino acids. In one embodiment, at least *Lₐ* and *Fₐ,* or at least *Vₐ* and F_{b}, are an N-methylated amino acid.

In one embodiment of any of formulae 1-2, as defined herein, each of m, n, p, q, r, s, and t is any selected from 0 and 1. In one embodiment, the peptide of any of formulae 1-2, as defined herein comprises any combination of m, n, p, q, r, s, and t being independently selected from 0 and 1. In one embodiment of any of formulae 1-2, as defined herein, m, n, p, q, r, s, and t are all 0 or 1. In an alternative embodiment of any of formulae 1-2, as defined herein, m and t are 0 and n, p, q, s, and r are 1. In an alternative embodiment of any of formulae 1-2, as defined herein, m, n, s and t are 0 and p, q, and r are 1. In an alternative embodiment of any of formulae 1-2, as defined herein, m and n are 0 and s, t, p, q, and r are 1. Other combinations of m, n, p, q, r, s, and t being independently selected from 0 and 1 are understood by the person skilled in the art. The peptide of the invention can be provided with any combination of m, n, p, q, r, s, and t being independently selected from 0 and 1. In a preferred embodiment of any of formulae 1-2, as defined herein, m, n, p, and q are 0 and r, s, and t are 1, or m, n, p, and q are 1 and r, s, and t are o. In a further preferred embodiment of any of formulae 1-2, as defined herein, m and n are 0 and p, q r, s, and t are 1; m, n and p are 0 and q, r, s, and t are 1; m, n, p and q are o and r, s, and t are 1; m, n, p, and q are 1 and r, s, and t are 0; m, n, p, q and r are 1 and s and t are 0; or m, n, p, q, r and s are 1 and t is o.

In one embodiment, the peptide of the invention, particularly the peptide according to any of formulae 1-8 as defined herein, comprises a sequence *FₐF_{b}*AE, NKGAII, VG, and/or VGGVV, e.g. *FₐF_{b}*AE, NKGAII, and VGGVV; wherein *Fₐ* and *F_{b}* are, independently at each occurrence, selected from phenylalanine and N-methyl-phenylalanine; A is alanine; E is glutamic acid; N is asparagine; K is lysine; G is, independently at each occurrence, is glycine; I is, independently at each occurrence, isoleucine; and V is, independently at each occurrence, valine.

In one embodiment of the peptide according to any one of formulae 1-5 as defined herein, the substituents represented by X₆, X₇, and X₈ are, independently at each occurrence, selected from norleucine, leucine, phenylalanine, methionine, tryptophan, tyrosine, threonine, alanine, isoleucine, valine, and modified forms thereof e.g. N-methylated forms thereof, preferably selected from norleucine, leucine, and phenylalanine. Modified forms of the respective amino acids are, e.g., N-methylated forms of the respective amino acids and/or D-amino acids. In an embodiment of the peptide, X₆, X₇, and X₈ are any combination of amino acids selected from norleucine, leucine, phenylalanine, methionine, tryptophan, tyrosine, threonine, alanine, isoleucine, and valine, preferably selected from norleucine, leucine, and phenylalanine; for example, three different amino acids (e.g. Nle-Leu-Phe), two amino acids of the same kind and one amino acid different therefrom (e.g. Nle-Nle-Phe), or three of the same amino acids (e.g. Nle-Nle-Nle). In a preferred embodiment of the peptide, X₆, X₇, and X₈ are the same amino acid selected from norleucine, leucine, phenylalanine, methionine, tryptophan, tyrosine, threonine, alanine, isoleucine, and valine, preferably selected from norleucine, leucine, and phenylalanine. In one embodiment, X₆, X₇, and X₈ are an amino acid triplet comprising three amino acids of the same kind.

In one embodiment, the term "modified form", as used herein in the context of amino acids, relates to a modified form of an amino acid in which the amino acid chain is derivatized (or a derivative of the amino acid side chain), e.g. an ester or an amino acid comprising a protecting group such as BOC, to a modified form of an amino acid in which the side chain of the amino acid comprises one or more substituents, to a D-amino acid of the respective amino acid, and/or to an N-methylated form or an N-alkylated form of the respective amino acid. In one embodiment, the amino acids defined for the amino acid residues of any one of formulae 1-17 can be present in a modified form of the amino acid, e.g. any of the substituents recited for X₁, X₂, X₃, X₄, *Fₐ, F_{b}*, *A,* E, X₅, X₆, X₇, X₈, *N, K, G, I,* X₉, X₁₀, X₁₁, *Lₐ*, and *Vₐ* can be a modified amino acid, e.g. an amino acid comprising a side chain with at least one substituent, a D-amino acid, and/or an N-methylated amino acid. For example, lysine may be present, as modified lysine e.g. Lys(Ac), Lys(N3), and Lys(biotin). When referring to modified amino acids by referring to an amino acid three-letter-code in combination with a modification specified in brackets, e.g. e.g. Lys(Ac), Lys(N3), Lys(biotin), e.g. Orn(Ac), and Arg(Me)2, such indication of the modification in brackets is meant to be understood as referring to a modification of the side chain of the amino acid represented by the three-letter-code. For example, Lys(Ac) is lysine with an acetylated sidechain, Lys(N3) is lysine with an azide functional group at the sidechain, Lys(biotin) is lysine with a biotinylated sidechain, Orn(Ac) is ornithine with an acetylated sidechain, and Arg(Me)2 is arginine with the sidechain comprising two methyl groups. In one embodiment, the term "Lys(N3)" relates to N-epsilon-azido-lysine.

The amino acids mentioned in the appended claims, particularly any amino acid present in a peptide of the invention, may be present in a modified form of the respective amino acid, e.g. in an N-methylated form thereof, as an acetylated form thereof e.g. lysine in the form of Lys(Ac), as an biotinylated form thereof e.g. lysine in the form of Lys(biotin), and/or as a D-amino acid. In one embodiment, the peptide of the invention has an amino acid sequence as defined by any of formulae 1-17, as defined above.

In one embodiment of the peptide according to any one of formulae 1-2 as defined herein, X₁₁ is selected from norleucine, leucine, phenylalanine, methionine, tryptophan, tyrosine, threonine, alanine, isoleucine, and valine, preferably is norleucine. For example, a peptide according to any one of formulae 1-2 as defined herein, may comprise X₁₁ being selected from norleucine, leucine, phenylalanine, methionine, tryptophan, tyrosine, threonine, alanine, isoleucine, and valine, preferably being norleucine; and may further comprise a sequence *FₐF_{b}*AE, NKGAII, VG, and/or VGGVV, e.g. *FₐF_{b}*AE, NKGAII, and VGGVV; wherein *Fₐ* and *F_{b}* are, independently at each occurrence, selected from phenylalanine and N-methyl-phenylalanine; A is alanine; E is glutamic acid; N is asparagine; K is lysine; G is, independently at each occurrence, is glycine; I is, independently at each occurrence, isoleucine; and V is, independently at each occurrence, valine; wherein, preferably, at least one of X₃,X₄,*Fₐ*, and *F_{b}* of formula 1 is an N-methylated amino acid and at least one of *Lₐ*, *Vₐ, Fₐ,* and *F_{b}* of formula 2 is an N-methylated amino acid.

In one embodiment, the peptide of the invention is an amyloid inhibitory peptide that preferably binds to Aβ40(42) and/or to islet amyloid polypeptide (IAPP), or said peptide is a peptide that binds to Aβ40(42) and/or to islet amyloid polypeptide (IAPP), but is not necessarily an amyloid inhibitory peptide. It should be noted that in preferred embodiments, where reference is made to a "peptide" in general, such peptide may also be referred to as an "amyloid inhibitory peptide". An "amyloid inhibitory peptide" is a peptide that functions as or can be used as an "amyloid inhibitor". In one embodiment, the peptide of the invention comprises or consists of 19 to 28 amino acids, preferably 22 to 28 amino acids, more preferably comprises or consists of 26 amino acids.

In one embodiment, said peptide is linked to a reporter molecule that allows detection of Aβ40(42), islet amyloid polypeptide (IAPP) and/or amyloid aggregates or co-aggregates thereof by a detection methodology, such as positron emission tomography (PET), nuclear magnetic resonance (NMR), magnetic resonance imaging (MRI), and PET-MRI, preferably a dye or a quantum dot.

In one embodiment, the term "modified form", as used herein in the context of a peptide, refers to modified forms of peptides known to the person skilled in the art, e.g. peptides having a protected N-terminus and/or C-terminus. In one embodiment, the N-terminus and/or the C-terminus of the peptides according to the present invention are protected. Suitable protecting groups are manifold and are known to a person skilled in the art. For example, the N-terminus may be acetylated or formylated, or there may be an even longer chain attached such as palmitoyl. The C-terminus could be protected via formation of an amide or carbonic acid ester.

In one embodiment, the C-termini of the peptide(s) according to the present invention, in particular the peptides according to any one formulae 1-17 according to the present invention, are in their free carboxy-form, are present in the form of an ester, and/or are protected by an amide. In one embodiment, the C-terminus is in its free carboxy-form; in another embodiment, it is in esterified form. In particularly preferred embodiments, the C-termini of the peptide(s) according to the present invention, in particular of the amyloid inhibitory peptides according to the present invention, more particularly of the peptides according to formulae 1-17 according to the present invention, are in their carboxy-form, and the respective N-termini of the peptides are in their NH2-form or NH₃⁺-form. In one embodiment, the peptide of the invention is modified by an N-terminal acetylation, by formylation, by being coupled with a label e.g. fluorescent label or biotin label, by being coupled to any compound via a linker, and/or by C-terminal amidation.

Without wishing to be bound by any theory, the present inventors believe that the amyloid inhibitory effect of an amyloid inhibitory peptide is mediated by its binding to key amyloid polypeptides, in particular to Aβ40(42) and/or to islet amyloid polypeptide (IAPP). Hence, the term "amyloid inhibitory" as used herein in the context of a peptide, refers to the capability of such peptide to block or inhibit amyloid self-assembly, cross-assembly, and/or amyloidogenesis, preferably of the key amyloid polypeptides, in particular of Aβ40(42) and/or of islet amyloid polypeptide (IAPP). Amyloid inhibitory peptides in accordance with the present invention are useful as amyloid inhibitors, and may thus be used for therapeutic and/or diagnostic purposes, i.e. they may be used for treatment and/or diagnosis of diseases involving amyloid self-assembly, cross-assembly, or amyloidogenesis, in particular of Alzheimer's disease and/or of type 2 diabetes. In other embodiments, a peptide in accordance with the present invention may have the capability to bind to key amyloid polypeptides, but may not necessarily be capable of functioning as an amyloid inhibitor. Such peptides may herein also sometimes be referred to as "amyloid binding peptides". They bind to key amyloid peptides, but are not capable of blocking or inhibiting amyloid self-assembly or amyloidogenesis. They may nevertheless be useful for detection purposes, in cases where detection of amyloid peptides may be desirable.

The present invention also relates to a pharmaceutical composition comprising a peptide according to the present invention and a pharmaceutically acceptable excipient. In such pharmaceutical composition, the peptide may occur as such, or it may be linked to other entities/molecules that endow the peptide with a specific functionality. For example, there may be a tag attached to increase blood-brain-barrier permeability, or it may be attached to a specific reporter molecule, such as a dye or a quantum dot, allowing the detection in diagnostic methods (preferably whilst retaining the therapeutic functionality of the peptide - "theranostic applications"). The use of quantum dots may be particularly useful in various imaging technologies, such as MRI, PET, PET-MRI, or specifically quantum-dot-based brain imaging methodologies. In certain embodiments, the peptide may be attached to a nanoparticle, to a suitable carrier molecule, to a targeting entity or other functional molecule.

Because the peptides according to the present invention are highly likely to be capable of passing the blood-brain-barrier, the present invention also relates to the use of a peptide according to the present invention, as defined herein, as a carrier for molecules, substances or compounds to pass the blood-brain-barrier. In certain embodiments, the molecule to pass the blood-brain-barrier is linked, preferably covalently linked, to a peptide according to the present invention as defined herein.

The peptides of the invention bind to Aβ40(42) and IAPP and inhibit amyloid self-assembly and cross-assembly, and are thus highly useful for the use in the prevention, treatment, and diagnosis of AD and/or T2D. In one embodiment, the term "treatment" or "treating", as used herein, encompasses both prophylactic treatment and therapeutic treatment. In a preferred embodiment, it specifically refers to therapeutic treatment. The term "subject", as used herein, relates to an individual, e.g. a human or an animal, preferably a human. In one embodiment, the subject is a patient having or being at risk of acquiring AD and/or T2D. The present inventors have managed to design peptidic inhibitors of amyloid self-assembly of both Aβ40(42) and IAPP, as well as of cross-assembly of Aβ40(42) and IAPP, which can be used in the prevention, treatment, and diagnosis of AD and/or T2D.

The present invention also relates to a kit for the *in vitro* or in *vivo* detection of amyloid fibrils or aggregates, or for the diagnosis of Alzheimer's disease and/or type 2 diabetes in a subject, said kit comprising the peptide according to the present invention as defined above, the pharmaceutical composition according to the present invention as defined above, or the heterocomplex according to the present invention as defined above, in a freeze-dried form in a suitable container, a buffered solvent in a separate container for reconstitution of said peptide in solution, and, optionally, means to dispense said peptide once reconstituted in solution, such as a syringe or pipette. Alternatively, the kit may contain the peptide according to the present invention as defined above, the pharmaceutical composition according to the present invention as defined above, or the heterocomplex according to the present invention as defined above, in an already reconstituted, ready-to-use form.

The present invention also relates to the use of the peptide according to the present invention as defined herein or of the heterocomplex according to the present invention as defined herein, in an *in vitro* assay, such as an enzyme linked immunosorbent assay (ELISA), a radioimmuno assay (RIA), or a Dot/Slot blot assay, for the detection of monomeric islet amyloid polypeptide (IAPP), monomeric Aβ40(42), amyloid fibrils, amyloid aggregates, and/or amyloid co-aggregates. Such use may, in certain embodiments, involve the analysis of blood, cerebrospinal fluid, or brain biopsies, and may also further involve the use of suitable reporter molecules to which the peptide may be attached or with which the peptide may be used together.

The present inventors have provided peptides which function as nanomolar inhibitors of amyloid self-assembly and cross-assembly of Aβ40(42) and IAPP, and which therefore have manifold applications. Moreover, the peptides bind with high affinity to Aβ40(42) and/or IAPP monomers and/or amyloid aggregates and co-aggregates thereof.

The term "heterocomplex", as used herein, relates to a peptide of the present invention that is associated with an amyloid-β peptide and/or islet amyloid polypeptide, for example, a co-assembly comprising a peptide of the invention and amyloid-β peptide and/or islet amyloid polypeptide. In such heterocomplex, the peptide of the invention is associated with amyloid-β peptide and/or islet amyloid polypeptide by noncovalent binding. For example, a heterocomplex can be a heteromeric supramolecular nanofiber co-assembly comprising a peptide of the present invention, and amyloid-β peptide and/or islet amyloid polypeptide. Surprisingly, the heterocomplex of the invention, in contrast to fibrous IAPP or Abeta homo-assemblies, is non-cytotoxic, soluble, and does not act as an accelerator (seed) of amyloid formation of IAPP or Abeta. Furthermore, the heterocomplex of the invention is easily proteolytically degradable, and becomes easier recognized and phagocytosed by macrophages and microglia compared to fibrous IAPP or Abeta homo-assemblies. The peptides of the invention are highly advantageous in that they associate with IAPP and Abeta such that these do not convert into cytotoxic aggregates, co-aggregates, and amyloid. The peptides of the invention are advantageous in that they convert IAPP and Abeta into heterocomplexes having the above mentioned beneficial properties: no cytotoxic effect on cell viability, solubility, thermolability, proteolytic degradability, and effective cellular clearance. Furthermore, the purpose of the invention have the advantage that they convert IAPP and Abeta into heterocomplexes such that IAPP and Abeta are unable to act as seeds or cross-seeds of amyloid self-assembly. The fibrous co-assemblies, particularly the heterocomplexes of the invention, have thus a function; they are functional co-assemblies in that they act as a vehicle to prevent cytotoxic amyloid formation of IAPP and Abeta. By contrast, amyloid fibrils usually are badly soluble, cytotoxic, thermostable, proteolytically non-degradable, not efficiently phagocytosed by macrophages and microglia thereby contributing to cell- and neurodegeneration in AD and T2D, and act as accelerators/seeds of amyloid self-assembly and cross-seeds of IAPP and Abeta. In one embodiment, the terms "heterocomplex", "hetero-assembly", and "co-assembly" are used interchangeably. In one embodiment, the hetercomplex is a heteromeric nanofiber co-assembly, comprising a peptide of the present invention and an amyloid-β peptide and/or islet amyloid polypeptide.

In one embodiment, said heterocomplex is linked to a reporter molecule that allows detection of Aβ40(42), islet amyloid polypeptide (IAPP) and/or amyloid aggregates or co-aggregates thereof by a detection methodology, such as positron emission tomography (PET), nuclear magnetic resonance (NMR), magnetic resonance imaging (MRI), and PET-MRI, preferably a dye or a quantum dot.

In the present application, use is made of the one-letter-code for amino acid residues and the three-letter-code for amino acid residues. Hence, amino acid residues are typically designated herein by reference to their respective one-letter-code or three-letter-code. Accordingly, alanine is A or Ala; arginine is R or Arg; asparagine is N or Asn; aspartic acid is D or Asp; cysteine is C or Cys; glutamine is Q or Gln; glutamate is E or Glu; glycine is G or Gly; histidine is H or His; isoleucine is I or Ile; leucine is L or Leu; lysine is K or Lys; methionine is M or Met; phenylalanine is F or Phe; proline is P or Pro; serine is S or Ser; threonine is T or Thr; tryptophan is W or Trp; tyrosine is Y or Tyr; valine is V or Val; norleucine is Nle.

However, additionally in some instances, reference is made to a modified amino acid one-letter-code wherein some of the letters of the aforementioned "normal" one-letter-code are represented in italics. Such letters in italics refer to amino acid residues whose definition does not only comprise the respective amino acid usually represented by the one-letter-code as outlined in the preceding paragraph (e.g. F representing phenylalanine etc.), but which definition further comprises alternative amino acids, such as conservative substitutions of the respective amino acid represented by the one-letter-code (e.g. tyrosine being a conservative substitution of phenylalanine) and modified forms thereof (e.g. N-methyl-phenylalanine or halogenated phenylalanine). The amino acid residues represented by letters in italics are defined in the appended claims and should be understood to comprise all residues specified for the respective amino acid residue in the appended claims.

In one embodiment, the amino acids present in a peptide of the invention are L-amino acids or D- amino acids, or a mixture of L-amino acids and D- amino acids. In a peptide of the invention, some of the residues in the sequence may be L-amino acids and others may be D-amino acids. Sometimes, in this application, reference to amino acid sequences is made by reciting the individual residues as free amino acids, such as "glycine", "glutamic acid", etc., notwithstanding the fact that these residues appear in the respective amino acid sequence in their respective covalently linked form, i.e. with the individual residues linked by appropriate peptide bonds, i.e. amide bonds, between them.

The term "N-methyl" or "N-methylated", as used herein, refers to a methyl group that is attached to the nitrogen in the amide bond between two amino acid residues. In one embodiment, the terms "N-methyl" and "N-methylated", as used herein in the context of amino acids, are used interchangeably. In one embodiment, where an amino acid, e.g. phenylalanine, in an amino acid sequence is indicated as "N-methyl amino acid", e.g. "N-methyl-phenylalanine", this means that a methyl group is attached to the amide nitrogen forming the amide bond between phenylalanine and the amino acid that precedes phenylalanine in the amino acid sequence.

It should be noted that subscripts in the form of numbers, as used in the context of amino acids "X" in peptide sequences, are meant to denote the position of the individual residue within the sequence and not the total number of such residue in such position. As an example, the sequence X₆X₇X₈ is meant to denote a part of formula 1 with three individual amino acid residues defined by X₆, X₇, and X₈.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.

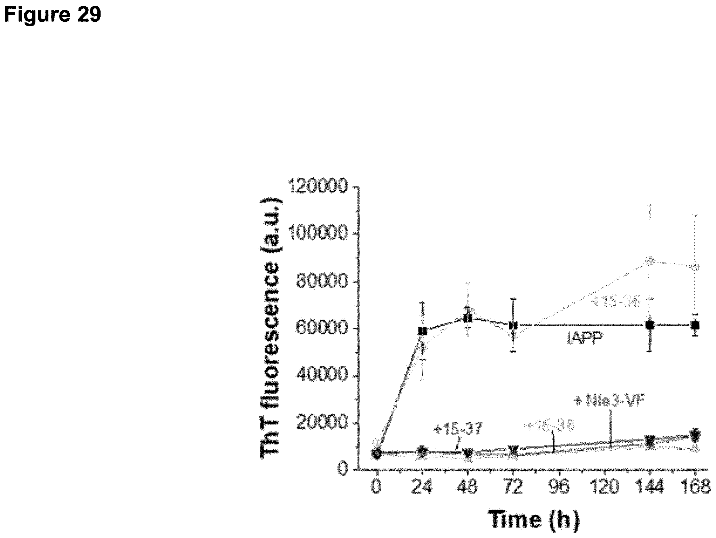

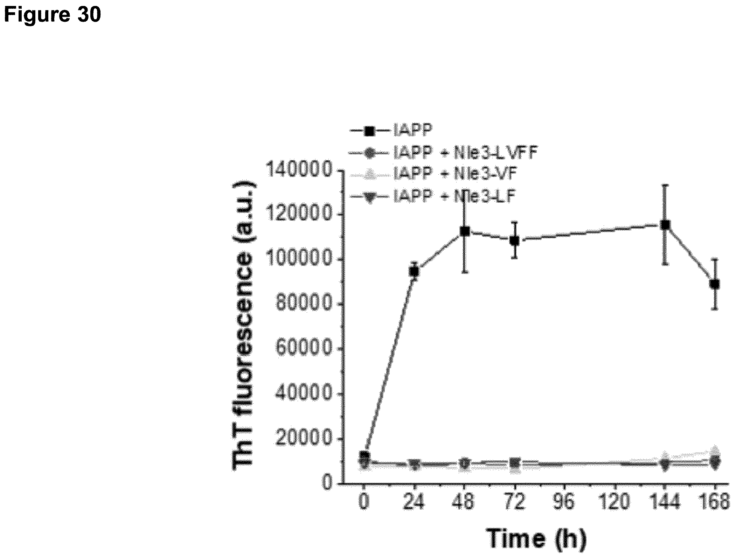

All methods mentioned in the figure descriptions below were carried out as described in detail in the examples.

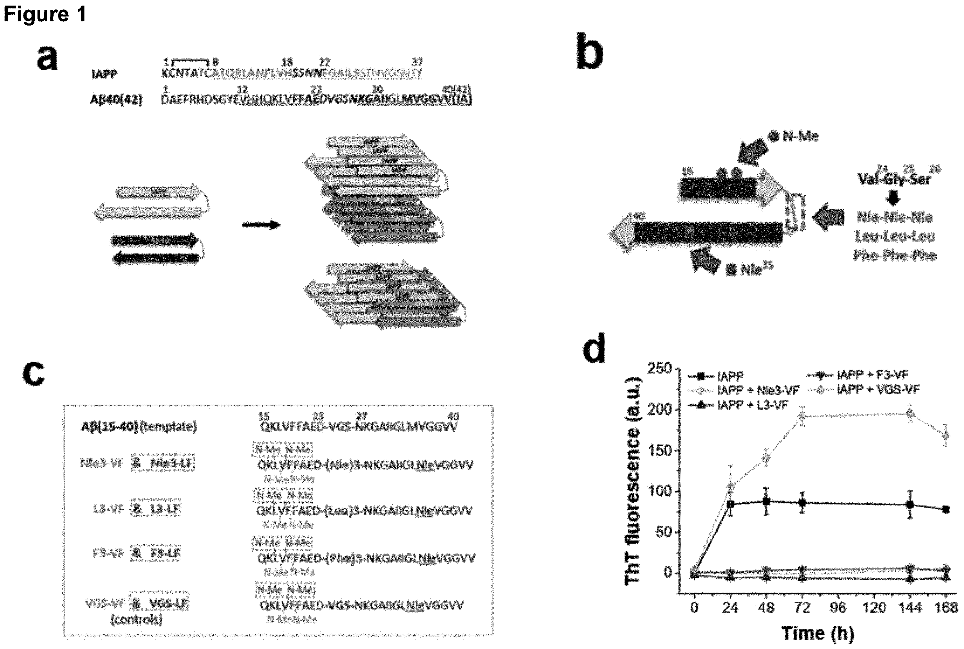

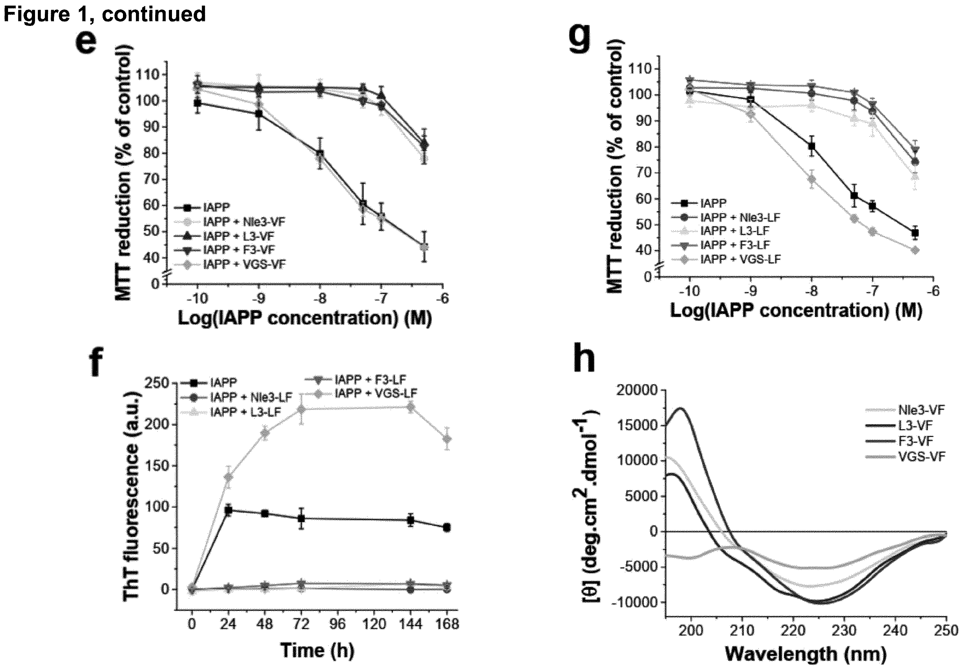

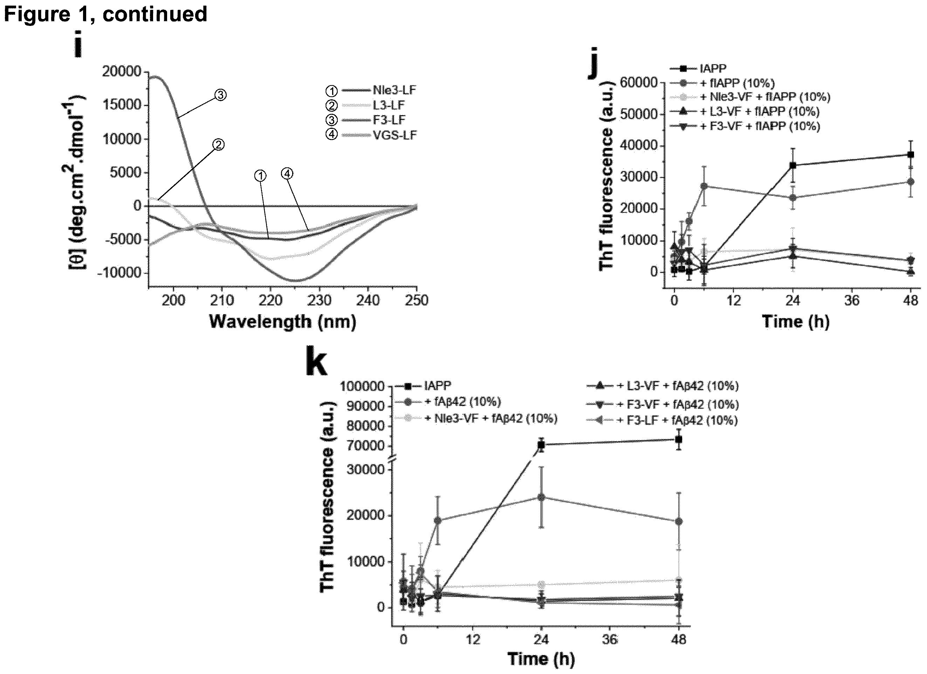

**Figure 1** shows the ACM design concept, their effects on IAPP amyloid self-assembly and cytotoxicity, and ACM secondary structures. **a** Sequences of IAPP and Aβ40(42), proposed models of fIAPP and fAβ40 folds, and hypothetical IAPP/Aβ4.0 "hetero-amyloids" (b-strands, pink & underlined; "hot segments" of self-/cross-interactions, bold; loop residues, italics). **b** ACM inhibitor design strategy. Template Aβ(15-40) in a b-strand-loop-b-strand fold proposed for fAβ40 is modified via (a) N-methylations in Aβ(17-20), (b) substitution of Aβ(24-26) by hydrophobic tripeptides, and (c) Met35 substitution by Nle. **c** Sequences of the six ACMs and negative controls VGS-VF and VGS-LF (Table 3). Each sequence corresponds to two different ACMs which contain the same LTS but a different couple of N-methylated residues (dashed boxes). Color code as in **a;** green or violet for peptide names and corresponding N-methylated residues. **d** Nle3-VF, L3-VF, and F3-VF block IAPP amyloid self-assembly. Fibrillogenesis of IAPP (16.5 µM) alone or with ACMs or VGS-VF assessed via ThT binding (IAPP/peptide 1/2) (means ± SD, 3 assays). **e** Nle3-VF, L3-VF, and F3-VF suppress formation of toxic IAPP assemblies. Solutions of **d** (7 day-aged (VFS-VF 24 h)) added to RIN5fm cells; cell viability determined via MTT reduction (means ± SD, 3 assays, n=3 each). **f** Nle3-LF, L3-LF, and F3-LF block IAPP amyloid self-assembly. Assay as in **d** (IAPP/peptide 1/2 except L3-LF (1/2.5)) (means ± SD, 3 assays). **g** Nle3-LF, L3-LF, and F3-LF suppress formation of toxic IAPP assemblies. Solutions of f (7 day-aged (VGS-LF 24 h)) added to RIN5fm cells; cell viability determined via MTT reduction (means ± SD, 3 assays, n=3 each). **h & i** Secondary structure of ACMs. Far-UV CD spectra of ACMs of **d** and **f** *versus* non-inhibitors (5 µM, pH 7.4). **j** ACMs inhibit seeding of IAPP by preformed fIAPP. Fibrillogenesis of IAPP (12 µM) w/o or with fIAPP seeds (10%) and seeded IAPP/ACM mixtures assessed via ThT binding (IAPP/ACM 1/2) (means ± SD, 3-9 assays). **k** ACMs inhibit fAP42-mediated cross-seeding of IAPP. Fibrillogenesis of IAPP with and w/o fAβ42 seeds (10%) *versus* IAPP/ACM mixtures (IAPP 12 µ M, IAPP/ACM 1/2) (means ± SD, 3-6 assays).

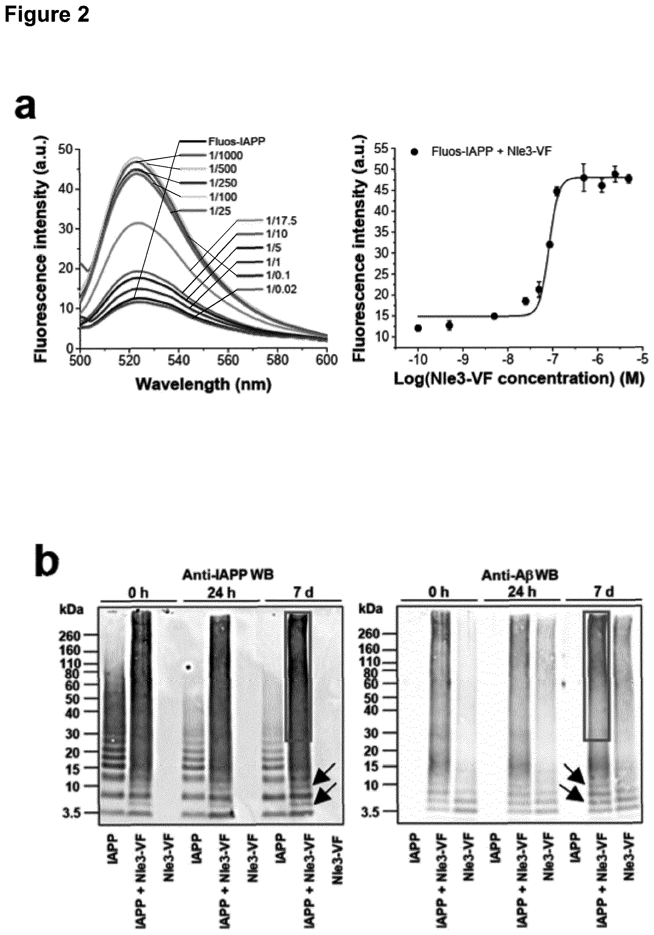

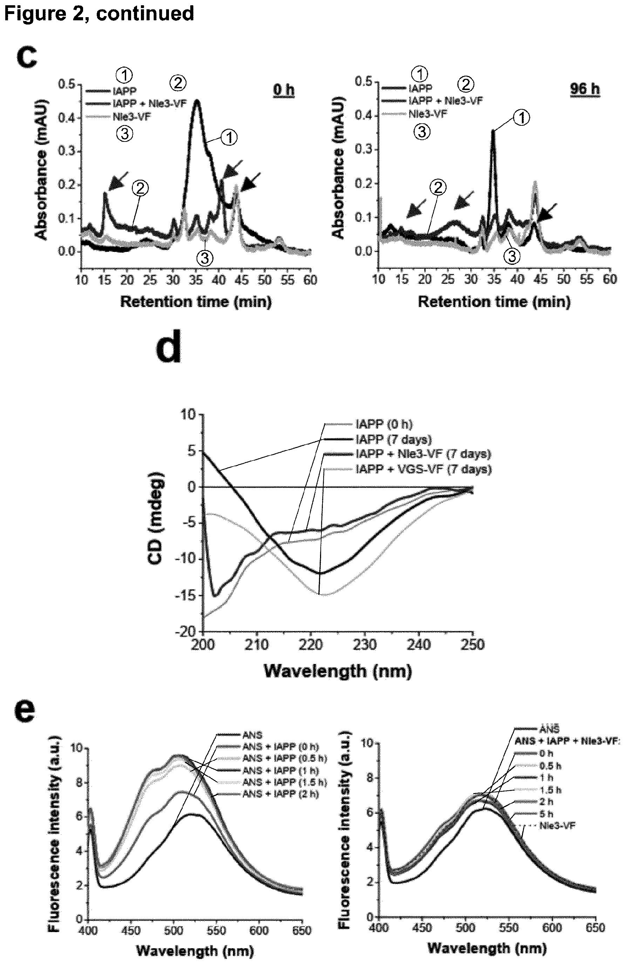

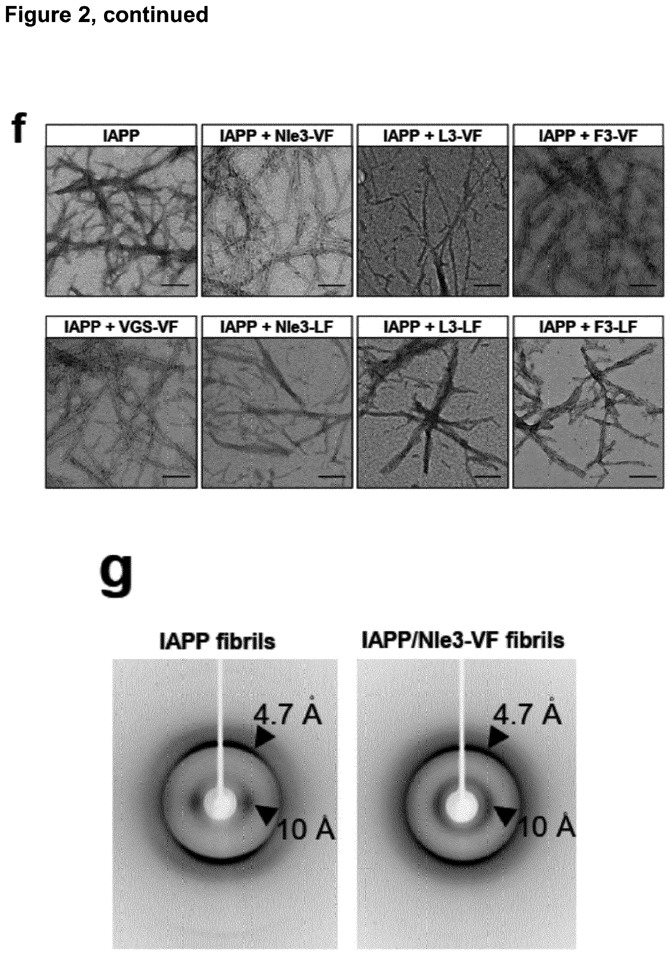

**Figure** 2 shows nanomolar affinity IAPP/ACM interactions yield amyloid-like but ThT-invisible nanofibers. **a** Nanomolar affinity IAPP/ACM interactions as determined by fluorescence spectroscopy. Fluorescence spectra of Fluos-IAPP (5 nM) and its mixtures with Nle3-VF (pH 7.4) at indicated molar ratios (data from one representative binding assay (n=3)). Inset, binding curve; data are means ± SD from 3 assays. **b** IAPP/ACM interactions result in hetero-dimers and medium-to-high MW hetero-assemblies. Characterization of IAPP/Nle3-VF hetero-complexes via cross-linking at different time points and NuPAGE and Western blot using anti-IAPP (left) or anti-Aβ (right) antibodies. IAPP/Nle3-VF mixtures (1/2; IAPP, 30 µM) were cross-linked with glutaraldehyde; orange box indicates medium-to-high MW hetero-assemblies (major species); arrows indicate hetero-di-/-tri-/tetramers. **c** Characterization of IAPP/Nle3-VF hetero-assemblies via size exclusion chromatography (SEC). Chromatograms of IAPP alone (16.5 µM) or its mixtures with Nle3-VF (1/2) at 0 h and at 96 h. Black arrow indicates IAPP monomers and Nle3-VF dimers; blue arrows indicate IAPP/Nle3-VF hetero-dimers and medium-to-high MW hetero-assemblies. **d** IAPP/Nle3-VF co-assemblies are more disordered than b-sheet-rich IAPP assemblies. Far-UV CD spectra of 7 day-aged IAPP (16.5 µM; pH 7.4) and its mixtures (1/2) with the Nle3-VF or VGS-VF (non-inhibitor) are shown; for comparison, the spectrum of freshly dissolved IAPP (0 h) is also shown. **e** IAPP/Nle3-VF co-assembly blocks surface-exposure of hydrophobic clusters occurring at early steps of IAPP amyloid self-assembly as determined by anilinonaphthalene 8-sulfonate (ANS) binding. Fluorescence emission spectra of ANS alone/with IAPP (2 µM) (left) and of ANS alone/with IAPP/ACM (1/2) mixtures (right) (pH 7.4) were measured at various time points of self- or co-assembly as indicated. **f** IAPP/ACM interactions results into ThT-invisible fibrils of indistinguishable appearance to fLAPP by TEM. TEM images of 7 day-aged IAPP (16.5 µM) and its mixtures (1/2) with ACMs or VGS-VF (non-inhibitor) (solutions from Fig. **1d****,f).** Scale bars: 100 nm. **g** fLAPP and fibrils in IAPP/Nle3-VF mixture exhibit the amyloid cross-b structure signature. X-ray fiber diffraction patterns of fIAPP and fibrils present in aged IAPP/Nle3-VF mixture (1/2) showed major meridional and equatorial reflections at ~4.7 and ~10 Å.

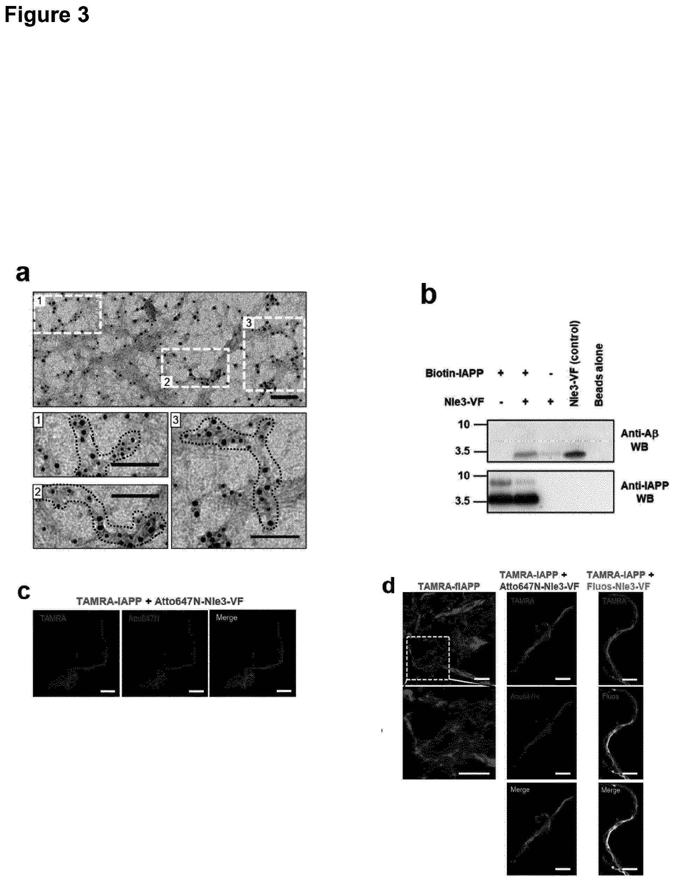

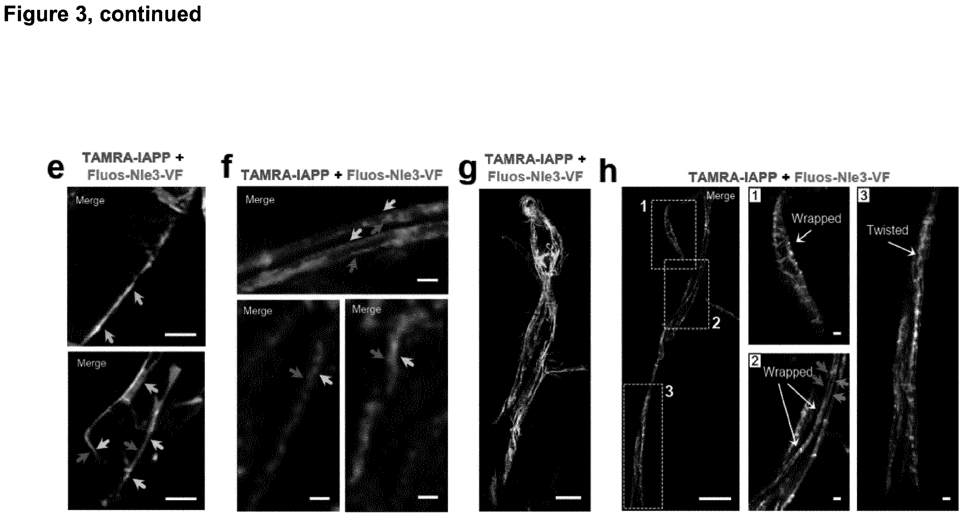

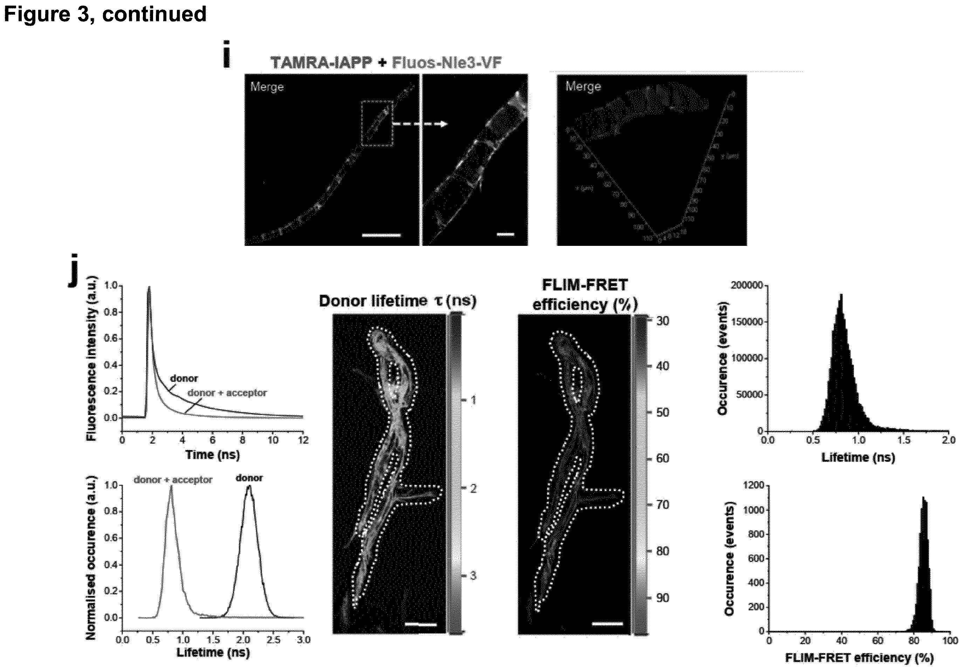

**Figure 3** shows evidence for supramolecular IAPP/ACM nanofiber co-assembly. **a** Immunogold TEM images of aged IAPP/Nle3-VF (1/2; IAPP, 16.5 µM) reveals fibrils which bind to both anti-fLAPP and anti-Aβ antibodies (LAPP, 5 nm gold; Nle3-VF, 10 nm gold). Scale bars: 100 nm. **b** Heteromeric nature of ThT-invisible fibrils in aged Biotin-IAPP/Nle3-VF (1/2; Biotin-LAPP, ₁6.₅ µM) as assessed by a biotin pull-down assay. Components were revealed by WB with anti-Aβ (upper part) and anti-LAPP (lower part) antibodies; lane "Nle3-VF (control)", Nle3-VF directly loaded onto the gel (w/o beads). **c** STED images of supramolecular heteromeric nanofiber bundles in aged LAPP/Nle3-VF (1/2; IAPP(total), ₁6.₅ µM) containing TAMRA-IAPP and Atto647-Nle3-VF (10%). Scale bars: 5 µm. **d** 2-Photon microscopy (2PM) images of nanofiber bundles in aged IAPP containing TAMRA-IAPP (10%) (left), aged IAPP/Nle3-VF containing TAMRA-IAPP and Fluos-Nle3-VF (10%) (middle), and aged IAPP/Nle3-VF containing TAMRA-IAPP and Atto647N-Nle3-VF (10%) (right) (1/2; IAPP(total), ₁6.₅ µM). Scale bars: 10 µm. **e-h** 2PM images of heteromeric fibrous superstructures in aged TAMRA-IAPP/Fluos-Nle3-VF (1/2) (TAMRA-IAPP ₁6.₅ mM). Short colored arrows indicate nanofiber bundles parallel or intertwined (red, TAMRA-IAPP; green, Fluos-Nle3-VF) or overlaying (yellow); long white arrows indicate twists or wrapping. Scale bars: panel **(e)** 5 µm, (**f**) upper part, 5 mm & lower parts, 1 mm, **(g)** 50 mm, **(h)** 50 mm (insets 5 mm). **i** 2PM image of a huge nanotube-like co-assembly found in aged TAMRA-LAPP/Fluos-Nle3-VF (1/2; TAMRA-IAPP, ₁6.₅ µM) (upper panel) and 3D-reconstruction of z-stacks (lower panel). Scale bars: 100 µm (inset 10 µm). **j** FLIM-FRET analysis of TAMRA-IAPP/Fluos-Nle3-VF co-assembly of **(g)** indicates a very close (<5.5 nm) donor-acceptor proximity. Left panel, fluorescence decay curves (top) and lifetimes (bottom) of donor (Fluos-Nle3-VF) without or with acceptor (TAMRA-IAPP); a strong shift of donor lifetime in the presence of acceptor is observed. Middle panel/left side, FLIM image showing donor life time; life time range 0 ns (dark blue) to 3.5 ns (red); scale bar, 50 µm. Middle panel/right side, FLIM-FRET efficiency (%); efficiency range 30% (dark blue) to 95% (red); scale bar, 50 µm. Right panel, donor lifetime (<1 ns) and FLIM-FRET efficiency distributions (>80%).

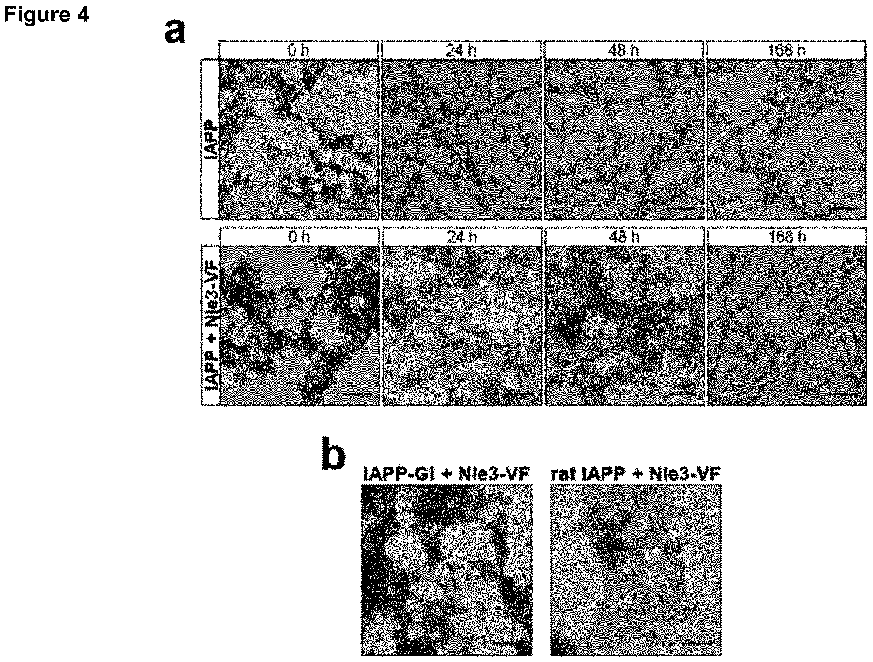

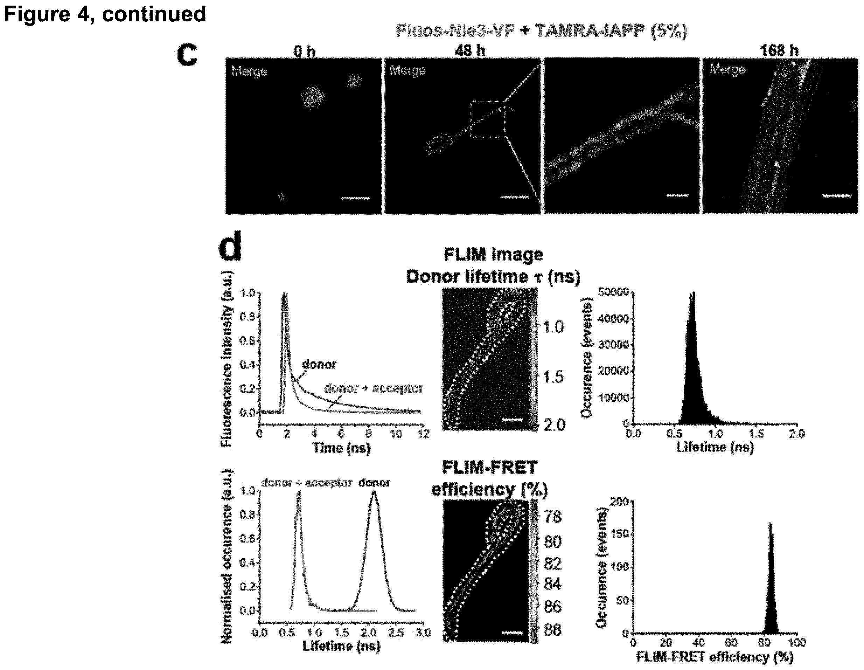

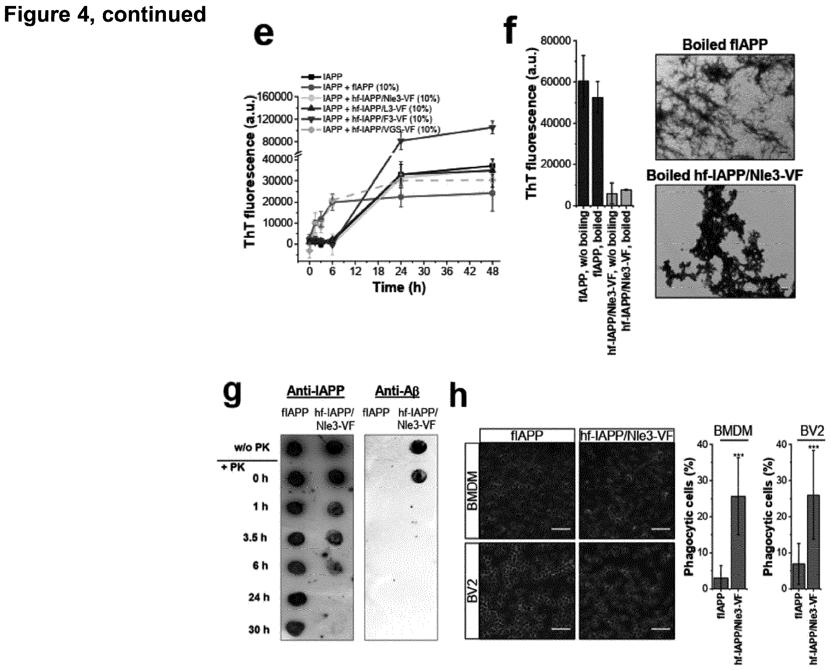

**Figure 4** shows mechanism of formation and properties of IAPP/ACM nanofiber co-assemblies. **a** Evolution of hf-IAPP/ACM from amorphous co-aggregates. TEM images of IAPP (16.5 µM) and IAPP/Nle3-VF mixtures (1/2) between 0 and 7 days of incubation. Scale bars: 100 nm. **b** TEM images of 7 day-aged IAPPGI/Nle3-VF or rat APP/Nle3-VF (1/2) shows amorphous aggregates. Scale bars: 100 nm. **c** IAPP monomers/prefibrillar species template nanofiber co-assembly. 2PM images of Fluos-Nle3-VF (33 µM) "cross-seeded" with freshly made TAMRA-IAPP (5%). Scale bars: 10 µm; inset, 1 µm. **d** FLIM-FRET of nanofiber co-assembly of **c** at 48 h reveals identical FLIM-FRET properties to hf-TAMRA-IAPP/Fluos-Nle3-VF (1/2; 7 day-aged) from Fig. **3j****.** Left panel, fluorescence decay curves (top) and lifetimes (bottom) of Fluos-Nle3-VF without or with TAMRA-IAPP shows a strong shift of donor lifetime in the presence of acceptor. Middle panel/upper part, FLIM image showing donor life time; range as indicated; scale bar, 5 µm. Middle panel/lower part, FLIM-FRET efficiency (%); range as indicated; scale bar, 5 µm. Right panel, distributions donor lifetime (< 1 ns) and FLIM-FRET efficiency (>80%). **e** hf-IAPP/ACM are seeding incompetent. IAPP (12 µM) fibrillogenesis alone or with 10% hf-IAPP/ACM, fIAPP, or IAPP/VGS-VF was followed by ThT binding (means ± SD, 3-4 assays). **f** Thermostability of hf-IAPP/ACM *versus* fIAPP. Left panel, ThT binding of fIAPP and hf-IAPP/Nle3-VF before/after boiling (5 min); means ± SD, 3 assays. Right panel, TEM images after boiling; scale bars: 100 nm. **g** Degradation of hf-IAPP/ACM *versus* fIAPP by proteinase K (PK) followed by dot blot. fIAPP or hf-IAPP/Nle3-VF were subjected to PK digestion (37°C); quantification by anti-fIAPP and anti-Aβ antibodies. Representative membranes from 3-6 assays. **h** Phagocytosis of hf-IAPP/ACMs *versus* fIAPP by primary murine BMDMs and cultured murine BV2 microglia. Left panel, representative microscopic images of cells following incubation (6 h, 37°C) with TAMRA-fIAPP (3.3 µM) or hf-TAMRA-IAPP/Nle3-VF (3.3 µM); red dots, TAMRA-IAPP; scale bars, 100 µm. Mid and right panels, amounts of phagocytic cells (% of total). Data means ± SD from 15-18 peptide preparations analyzed in 5 cell assays with each assay well analyzed in 3 fields of view; ^{∗∗∗} P<o.ooi (unpaired t-test).

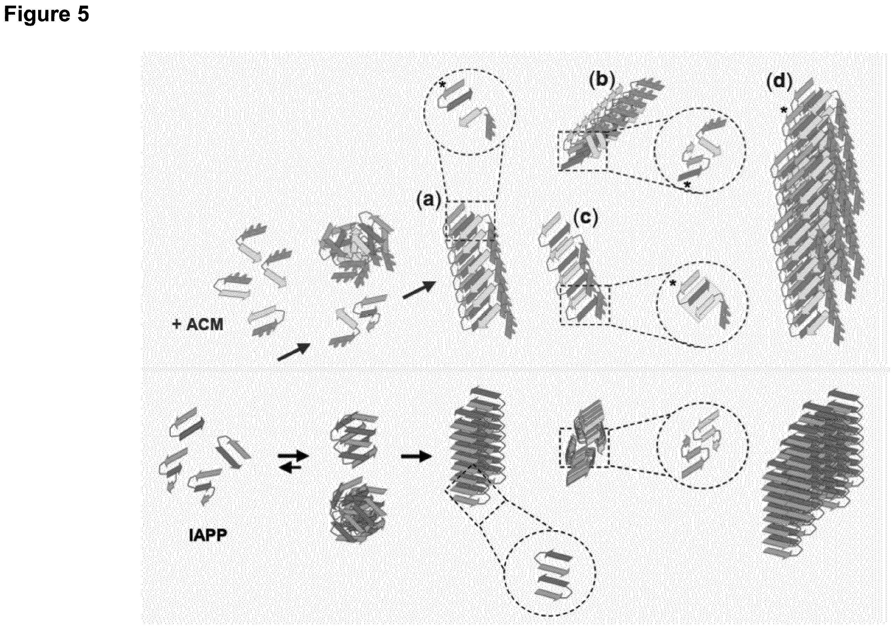

**Figure 5** shows proposed mechanism and hypothetical models of IAPP/ACM nanofiber co-assembly *versus* IAPP amyloid self-assembly. Lower part, IAPP self-assembly into toxic oligomers and amyloid fibrils. Upper part, in the presence of ACMs, IAPP monomers/prefibrillar species are redirected into initially amorphous and non-toxic hetero-assemblies which convert into amyloid-like but ThT-invisible and non-toxic heteromeric nanofibers and their fibrous superstructures. Shown are hypothetical models of heteromeric nanofibers **(a-c)** and supramolecular co-assemblies thereof **(d)** generated by lateral **(a,b,d)** or axial **(c)** co-assembly of the ACM with two of the previously suggested fIAPP folds or variants thereof (indicated by "^{∗}"). The ACM is shown in Aβ amyloid core-mimicking strand-loop-strand folds; blue dots indicate N-methyl rests.

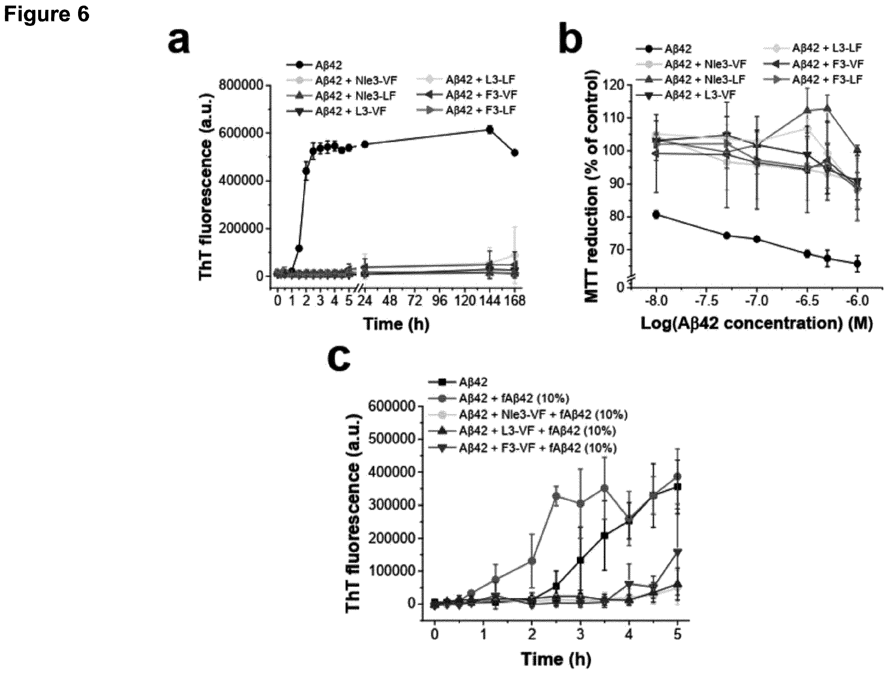

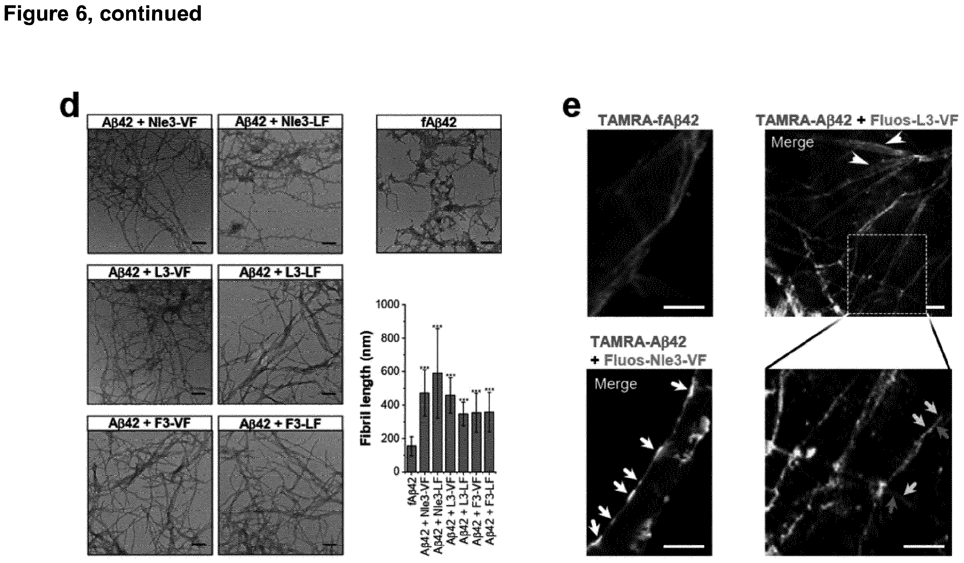

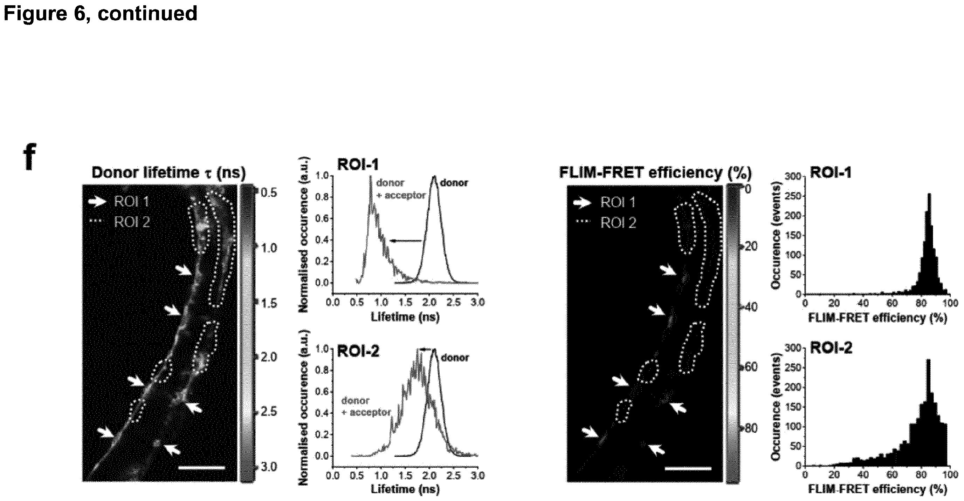

**Figure 6** shows inhibition of Aβ42 amyloid self-assembly via ThT-invisible and non-toxic Aβ42/ACM nanofiber co-assembly. **a** ACMs inhibit Aβ42 amyloid self-assembly. Fibrillogenesis of Aβ42 (5 µM) and Aβ42/ACM (1/1) followed by ThT binding (means ± SD, 3 assays). **b** ACMs suppress Aβ42 cytotoxicity. Aged Aβ42 (5 µM) or Aβ42/ACM (1/1) (6 days) (w/o ThT) were added to PC12 cells; cell damage determined via MTT reduction (means ± SD, 3 assays, n=3 each). **c** ACMs suppressed seeding of Aβ42 by fAβ42. Fibrillogenesis of Aβ42 (5 µM) without or with fAβ42 seeds (10%) and of fAβ42-seeded Aβ42/ACM (1/1) followed by ThT binding (means ± SD, 3 assays). **d** Aged Aβ42/ACM consists of ThT-invisible fibrils (hf-Aβ42/ACMs). TEM images of Aβ42 (fAβ42) and Aβ42/ACM mixtures (1/1) (from **b**; 6 day-aged) are shown; scale bars, 100 nm. Bottom right, bar diagram showing fibril lengths; ^{∗∗∗}P<0.001 (one-way ANOVA & Bonferroni; n=15-23). **e** 2PM images of fAβ42 and hf-Aβ42/ACMs. Fibrillar co-assemblies in aged Aβ42 containing TAMPA-Aβ42 (50%) (2 h; fibrillogenesis plateau), aged Aβ42/Fluos-Nle3-VF containing TAMRA-Aβ42/Fluos-Nle3-VF (50%) (4 days), and aged AP42/Fluos-L3-VF containing TAMRA-AP42/Fluos-L3-VF (50%) (6 days). White arrowheads indicate ribbon- or nanotube-like co-assemblies (yellow); white arrows indicate large "node"-like parts (yellow); colored arrows indicate TAMPA-Aβ42 (red) and Fluos-L3-VF (green) "building units"; scale bars, 10 µm. **f** FLIM-FRET of hf-TAMRA-Aβ42/Fluos-Nle3-VF of **e** indicates regions of high proximity (<5.5 nm) of the two polypeptides. Left panel/left side, FLIM image showing Fluos-Nle3-VF life times in the two regions of interest (ROIs); life time range, 0 ns (dark blue) to 3 ns (red); scale bar, 10 µm. White arrows indicate ROI-1 (node-like) while dotted lines indicate ROI-2 (cable-like). Left panel/right side, diagrams showing lifetimes of donor without or with acceptor in ROI-1 or ROI-2; a pronounced reduction of donor fluorescence lifetime in the presence of acceptor is observed; the shift is stronger in ROI-1. Right panel/left side, distribution of FLIM-FRET efficiency (%); efficiency range 0% (dark blue) to 95% (red); scale bar, 10 µm. Right panel/right side, bar diagrams showing FLIM-FRET efficiency (%) distribution in ROI-1 (>80%) and ROI-2 (60-100%).

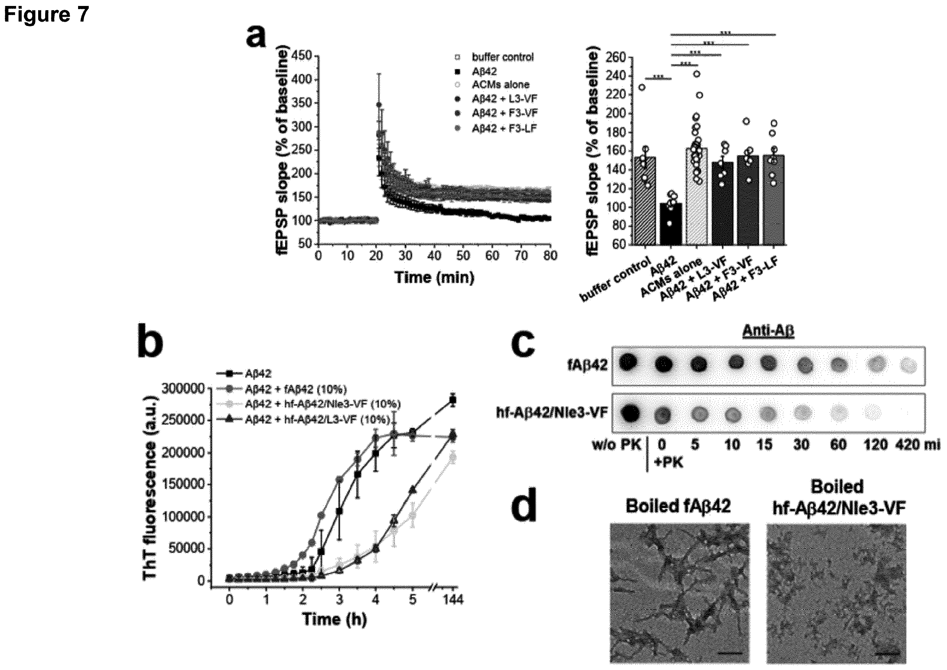

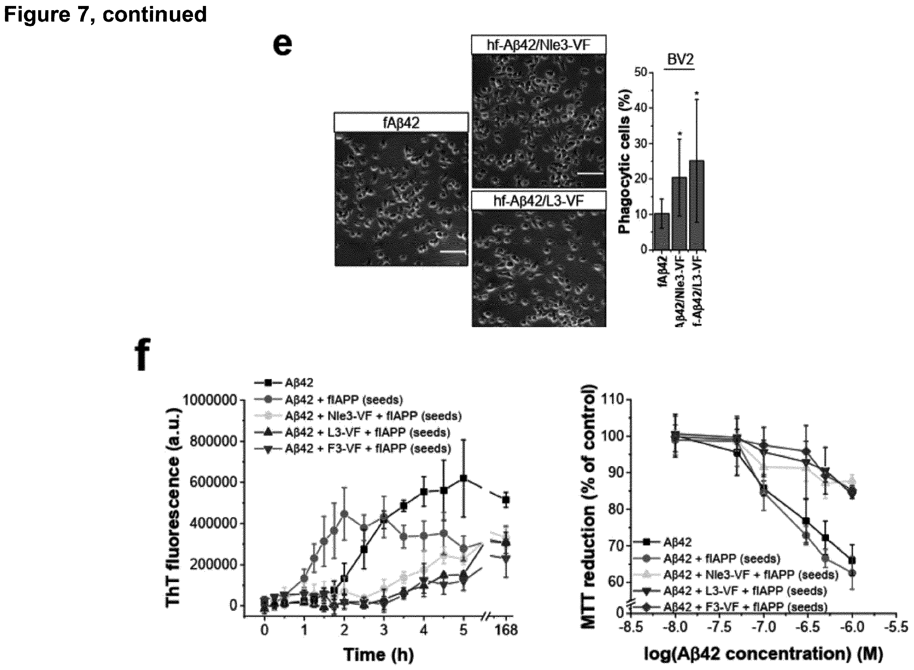

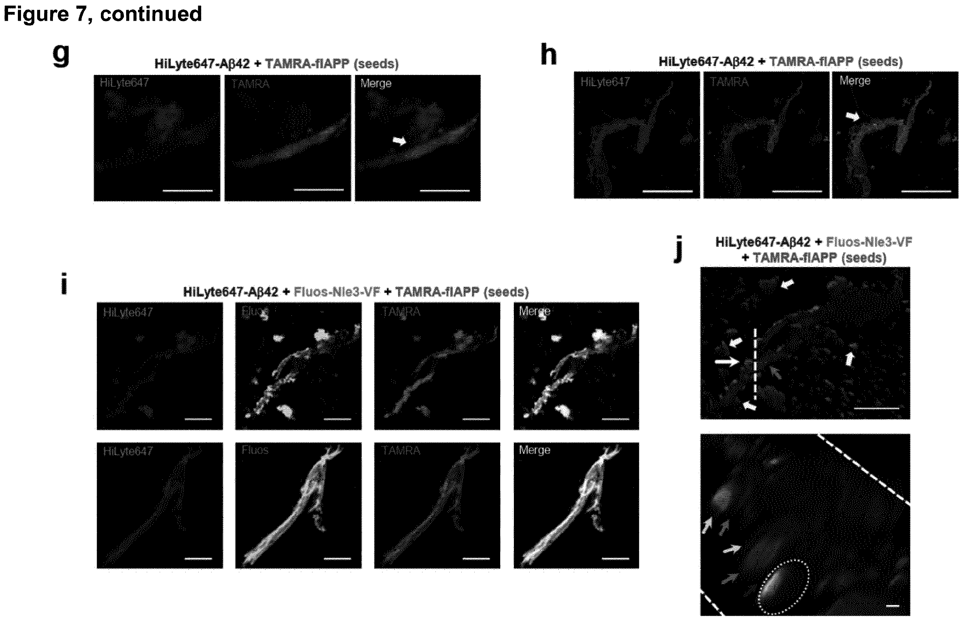

**Figure 7** shows properties and functions of Aβ42/ACM co-assemblies. **a** Aβ42/ACM co-assembly ameliorates Aβ42-mediated LTP impairment in murine hippocampal slices *ex vivo. Left,* time course of synaptic transmission; means ± SEM (n=7-8 for Aβ42/ACM (1/10), n=8 for Aβ42 (50 nM) and buffer controls, and n=36 for ACMs alone (500 nM)). *Right,* LTP values: averages from the last 10 min of recording; data, means ± SEM (n, see above); ^{∗∗∗}P<0.001 *versus* Aβ42 (one-way ANOVA & Bonferroni). **b** hf-Aβ42/ACM are seeding incompetent. Aβ42 (5 µM) fibrillogenesis alone or seeded with fAβ42, hf-Aβ42/Nle3-VF, or hf-Aβ42-L3-VF (10%) determined by ThT binding (means ± SD, 3 assays). **c** Degradation of hf-Aβ42/Nle3-VF and fAβ42 by PK (37°C) followed by dot blot; Aβ42 quantification by Aβ(1-17)-specific antibody. Representative membranes from 3 assays. **d** Thermolability of hf-Aβ42/ACM *versus* fAβ42. TEM images of boiled fAβ42 (15 min) *versus* hf-Aβ42/Nle3-VF (5 min); scale bars: 100 nm. **e** Phagocytosis of hf-Aβ42/ACM *versus* fAβ42 by cultured murine BV2 microglia. Left and mid panels, representative microscopic images of cells after incubation (6 h, 37°C) with TAMRA-fAβ42, hf-TAMRA-Aβ42/Nle3-VF, and hf-TAMRA-Aβ42/L3-VF (1 µM); red dots indicated TAMPA-Aβ42; scale bars, 100 µm. Right panel, amounts of phagocytic cells (% of total). Data means ± SD from 8-10 peptide preparations analyzed in 2 cell assays, each assay well analyzed in 3 fields of view; ^{∗}P<0.05 (unpaired t-test). **f** Effects of ACMs on fIAPP-mediated cross-seeding of Aβ42 fibrillogenesis (left panel) or cytotoxicity (right panel). Left panel, fibrillogenesis of Aβ42 (10 µM) or Aβ42/ACM (1/2) mixtures following cross-seeding with fIAPP (20%) and of Aβ42 w/o fIAPP seeds (10 µM) determined by ThT binding (means ± SD, n=4-8). Right panel, solutions (made as for left panel w/o ThT; 1.5 h aged) were added to PC12 cells; cell damage determined via MTT reduction (means ± SD, 3 assays, n=3 each). **g-j** 2PM characterization of supramolecular co-assemblies in Aβ42 solutions after cross-seeding with fIAPP (20%) in the absence **(g,h)** or presence of ACM **(ij). g & h** 2PM images of TAMRA-fIAPP-cross-seeded Aβ42 containing HiLyte647-Aβ42 (50%) (1.5 h; incubations as in **f)** show clusters of Aβ42 assemblies bound to/branching out of fIAPP surfaces; yellow arrow, Aβ42-fIAPP "contact site"; scale bars: 10 µm **(g)** and 100 mm **(h). i** 2PM images of fibrillar co-assemblies in TAMRA-fIAPP-cross-seeded Aβ42/Nle3-VF mixtures containing HiLyte647-Aβ42/Fluos-Nle3-VF (50%) (1.5 h; incubations as in **f);** scale bars: 10 µm. Upper panel, fIAPP covered byAβ42, Nle3-VF, and Aβ42/Nle3-VF (co-)assemblies and surrounded by amorphous or round/elliptical co-assemblies (see also **j).** Lower panel, huge ternary nanofiber co-assembly. **j** 3D reconstruction of z-stacks/still images of fibrous co-assemblies shown in **i**/upper panel. White arrow and dashed line in image on the top indicate view of the section shown below; yellow arrows, round/elliptical co-assemblies; red arrow, fIAPP; blue & green arrows, Aβ42 & Nle3-VF bound to fIAPP; encircled area indicates Aβ42/Nle3-VF co-assembly bound to fIAPP. Scale bars, 10 µm (top), 1 µm (bottom).

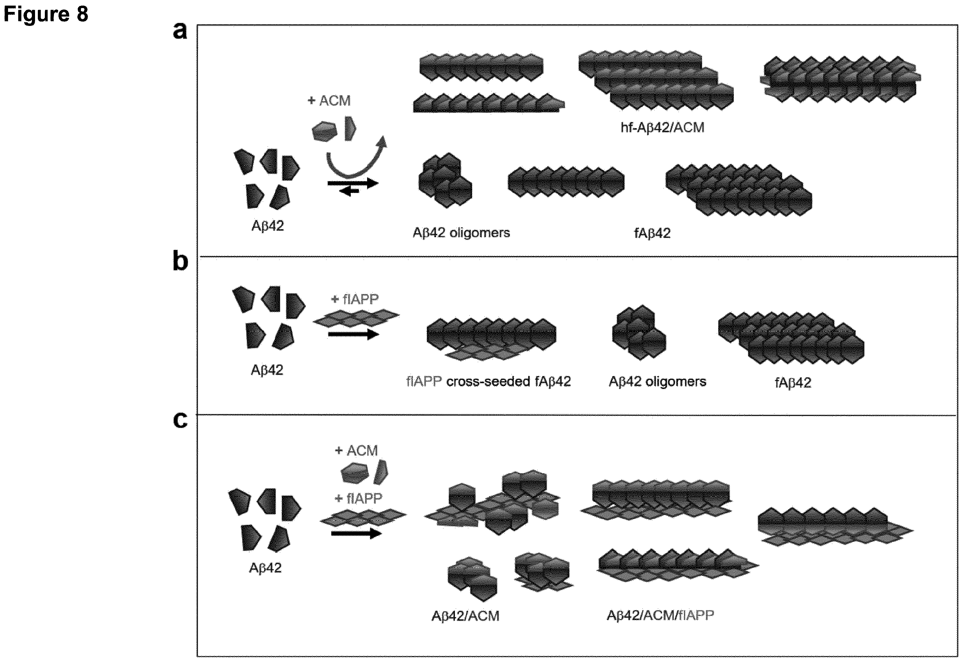

**Figure 8** shows schematic overview of identified co-assemblies and proposed mechanisms of ACM-mediated suppression of Aβ42 amyloid self-assembly **(a)** and its cross-seeding by fIAPP **(b,c). a** Lower row, Aβ42 self-assembles into toxic oligomers and fAβ42. Upper row, non-toxic ACMs bind with low nanomolar affinity Aβ42 and redirect it into heteromeric nanofiber co-assemblies (hf-Aβ42/ACM) which are non-toxic, seeding incompetent, and thermolabile and become easier degraded and more effectively phagocytosed than fAβ42. hf-Aβ42/ACM which may form by lateral or axial co-assembly are shown. **b** Cross-seeding of Aβ42 by fIAPP yields via 2ndary nucleation fAβ42/fIAPP co-assemblies, fAβ42, and toxic Aβ42 oligomers. **c** ACM-mediated inhibition of cross-seeding of Aβ42 by fIAPP. Non-toxic ACMs and ACM/Aβ42 co-assemblies (both fibrillar and amorph) bind to fIAPP yielding non-toxic and cross-seeding-incompetent fibrous co-assemblies.

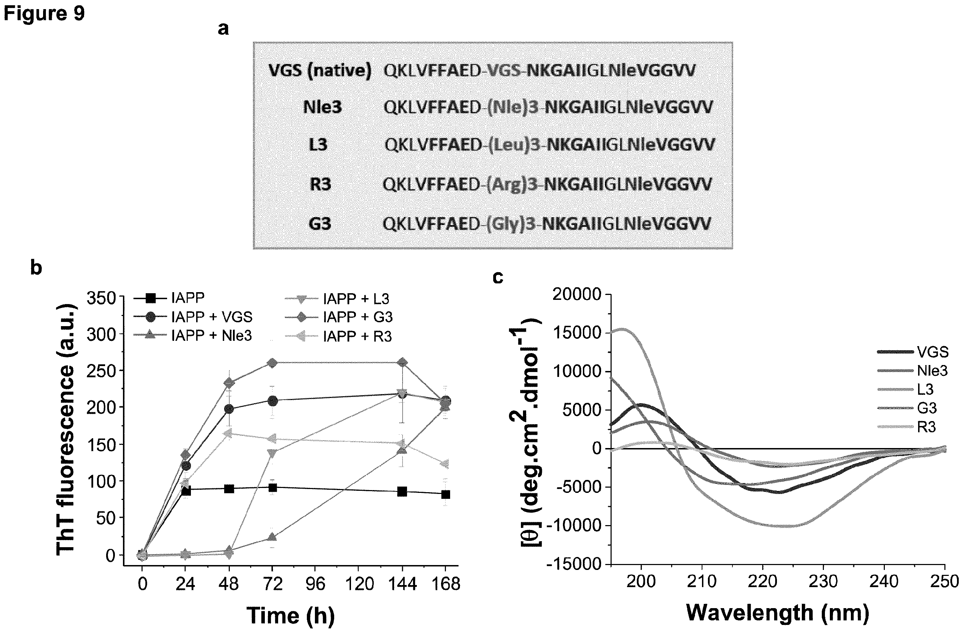

**Figure 9** shows an identification of best-suited LTS for inhibitor design. **a** Sequences of Aβ(15-40) (abbreviated "VGS") and designed analogs thereof (red: LTS; blue: "hot segments"; purple: Met35Nle substitution). **b** Effects of VGS and analogs on IAPP fibril formation: fibrillogenesis of IAPP (16.5 µM) with or without the peptides determined by the ThT binding assay (IAPP/peptide 1/2) (means ± SD, 3 assays). **c** Far-UV CD spectra of VGS and its analogs (5 µM, pH 7.4).

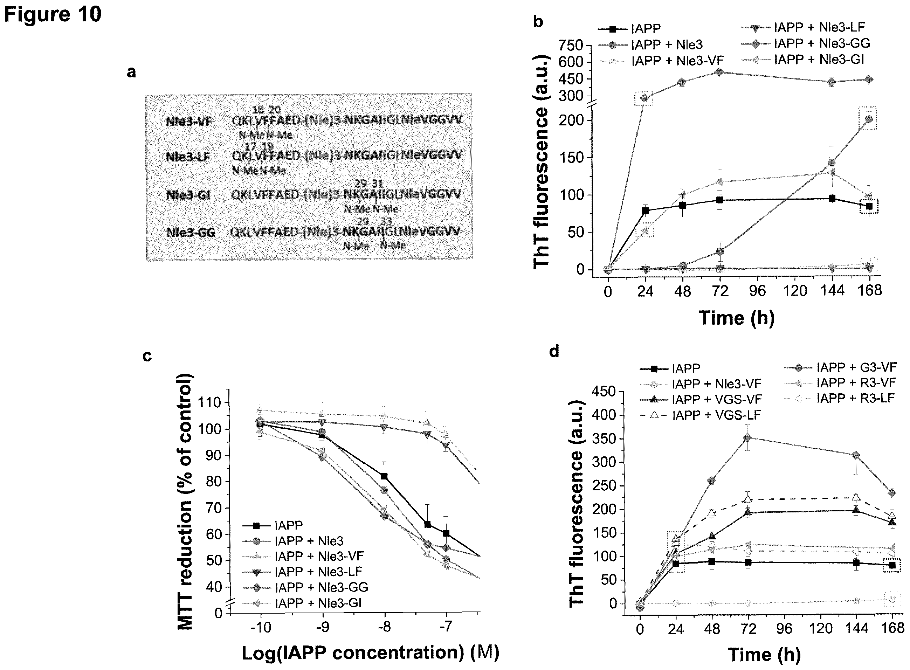

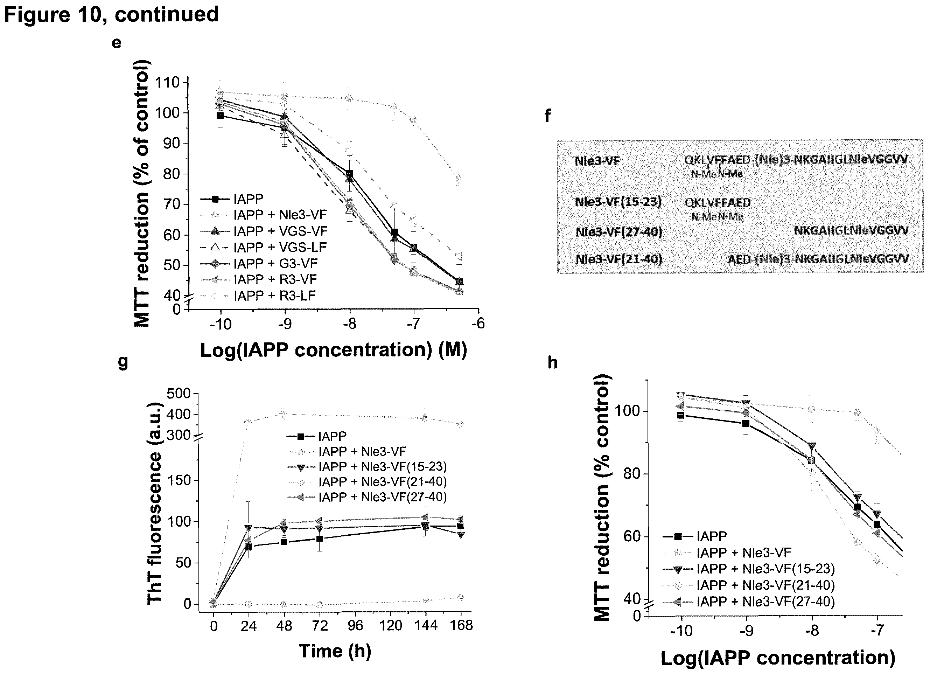

**Figure 10** shows an identification of best-suited sequence positions for N-methylations **(a-e)** and effects of partial segments of Nle3-VF on IAPP amyloid self-assembly **(f-g). a** Sequences of the four designed N-methylated Nle3 analogs (red: (Nle)3; blue: hot segments; purple: Met35Nle substitution); numbers indicate positions of N-methylated residues (for numbering the residues of all analogs, their numbers in the Aβ40 sequence were used). **b** Effects of the Nle3 analogs on IAPP fibrillogenesis. Fibrillogenesis of IAPP alone (16.5 µM) or its mixtures with the different analogs was studied via the ThT binding assay (IAPP/peptide 1/2). Dashed boxes indicate incubation time points at which parts of the solutions were used for the MTT reduction assay shown in **c.** Of note, data of Nle3-VF and Nle3-LF are also shown in Fig. id,f. Data are means ± SD from 3 assays. **c** Effects of Nle3 analogs on formation of cytotoxic IAPP assemblies. Solutions **of b** (24 h or 7 days aged as indicated) were added to RIN5fm cells; cell damage was determined via MTT reduction (means ± SD, 3 assays, n=3 each). Of note, data of Nle3-VF and Nle3-LF is also shown in Fig. 1e,g. **d** Effects of Val18Phe20- (-VF) or Leu17Phe19-(-LF) N-methylated analogs of the non-inhibitors VGS, R3, and G3 on IAPP fibrillogenesis as compared to inhibitor Nle3-VF. Fibrillogenesis of IAPP alone (16.5 µM) or its mixtures with the analogs was studied via ThT binding (LAPP/peptide 1/2). Dashed boxes indicate incubation time points at which parts of the solutions were used for the MTT reduction assay shown in **e.** Of note, data of Nle3-VF are also shown in Fig. id. Data are means ± SD from 3 assays. **e** Effects of peptides studied under **d** on IAPP cytotoxicity. Solutions of **d** were added to RIN5fm cells; cell damage determined via MTT reduction (means ± SD, 3 assays, n=3 each). Of note, data of Nle3-VF are also shown in Fig. 1e. **f** Sequences and abbreviations of synthesized and tested partial segments of Nle3-VF (see **g,h)** (color code: same as in **a**). **g** Effects of the partial segments of Nle3-VF shown in **f** on IAPP fibrillogenesis as compared to Nle3-VF. Fibrillogenesis of IAPP alone (16.5 µM) or its mixtures with each of the segments was studied via ThT binding (LAPP/segment 1/2). Data are means ± SD from 3 assays. **h** Effects of peptides studied under **g** on IAPP cytotoxicity. Solutions of **g** (at 24 h) were added to RIN5fm cells; cell damage determined via MTT reduction (means ± SD, 3 assays, n=3 each).

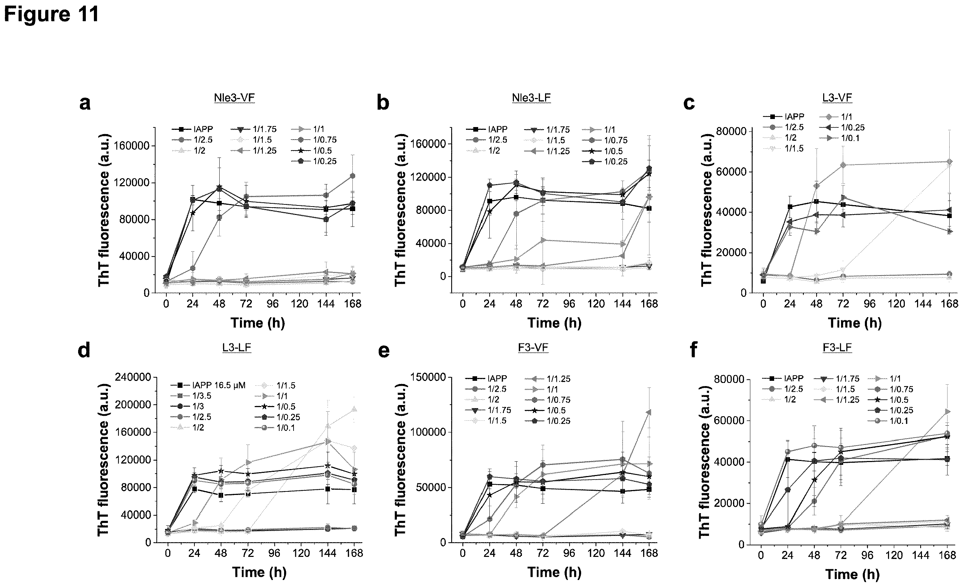

**Figure 11** shows concentration-dependence of inhibitory effects of ACMs on IAPP amyloid self-assembly. Fibrillogenesis of IAPP (16.5 µM) alone or in presence of Nle3-VF (a), Nle3-LF (b), L3-VF (c), L3-LF (d), F3-VF (e), and F3-LF (f) at the indicated IAPP/ACM ratios was followed by the ThT binding assay. Data are means ± SD from 3-8 assays.

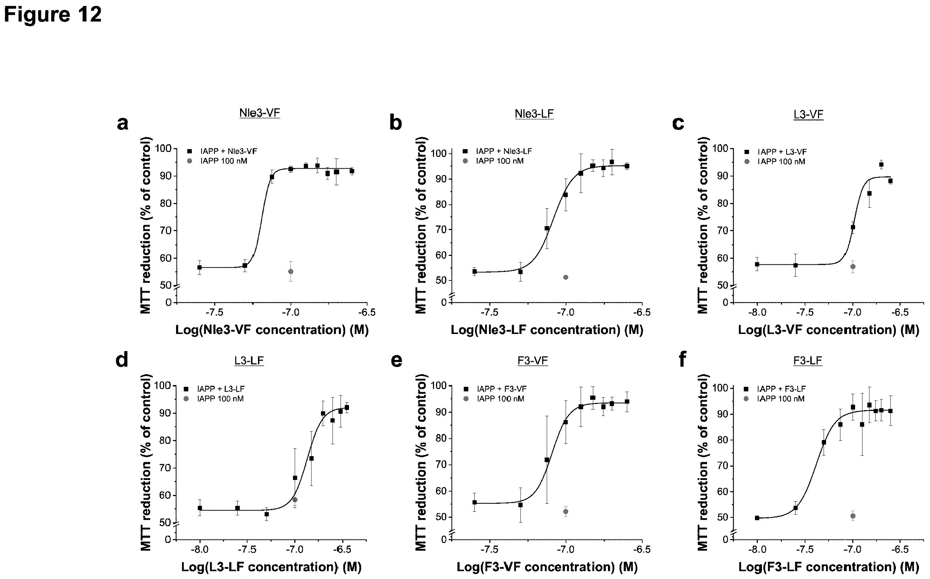

**Figure 12** shows a determination of IC₅₀ values of inhibitory effects of ACMs on formation of cell-damaging IAPP assemblies. Aged solutions (24 h) of IAPP alone (100 nM) or its mixtures with different amounts of ACMs were added to RIN5fm cells. Cell damage was determined by the MTT reduction assay for mixtures of IAPP with Nle3-VF (**a**), Nle3-LF (**b**), L3-VF (**c**), L3-LF (**d**), F3-VF (**e**) and F3-LF (**f**) as indicated; cytotoxicity of IAPP alone is included in each graph for comparison (red symbol). Data and determined IC₅₀ values are means ± SD from three assays (n=3 each).

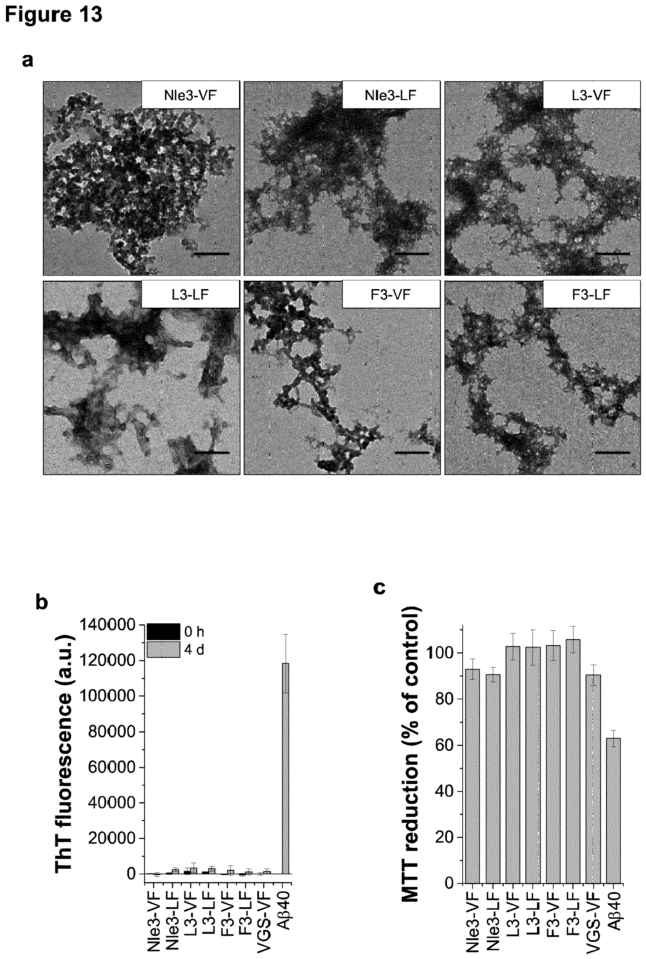

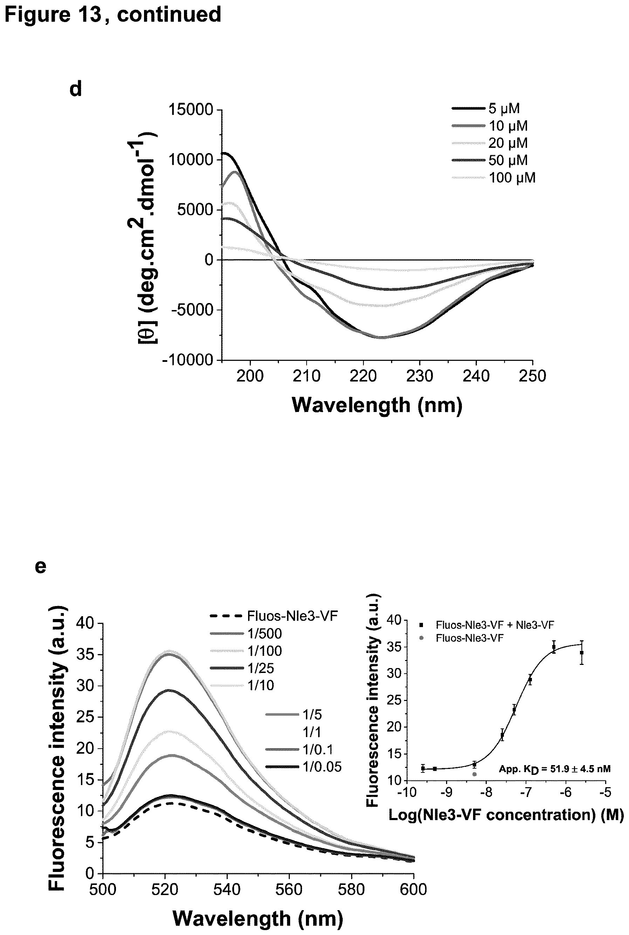

**Figure 13** shows that ACMs self-assemble into soluble, non-fibrillar, β-sheet rich, and non-toxic aggregates. **a** TEM examination of aged solutions of the six ACMs (100 µM; 4 days) reveals amorphous aggregates as main species; scale bars: 100 nm. **b** ACM aggregates do not bind ThT. ThT binding properties of the aged solutions of ACMs used in a and the non-inhibitor VGS-VF are shown (100 µM; 4 days). The ThT binding properties of an aged Aβ40 solution (100 µM; 4 days) is shown for comparison. Data are means ± SD from 3 assays. **c** ACM aggregates are not cytotoxic. Effects of aged solutions of ACMs and VGS-VF (4 days aged solutions from **a;** at 20 µM) on PC12 cell viability as determined via the MTT reduction assay. For comparison, effects of an aged fibrillar Aβ40 (solution from **b**; at 20 µM) are shown. Data are means ± SD from 3 assays, n=3 each. **d** Nle3-VF oligomerization studied by far-UV CD spectroscopy. CD spectra at different peptide concentrations as indicated (aq. solution, pH 7.4) are shown. Loss of signal was indicative of oligomerization; however, no turbidity or precipitation was observed. **e** Self-assembly of Nle3-VF studied by fluorescence spectroscopic titrations. Emission spectra of Fluos-Nle3-VF (5 nM) alone and with various Nle3-VF amounts as indicated (Fluos-Nle3-VF/Nle3-VF) (pH 7.4); spectra are from one representative assay out of three. Inset, binding curve (data means ± SD from 3 binding curves); determined app. K_{D} = 51.9 ± 4.5 nM (mean ± SD from 3 binding curves).

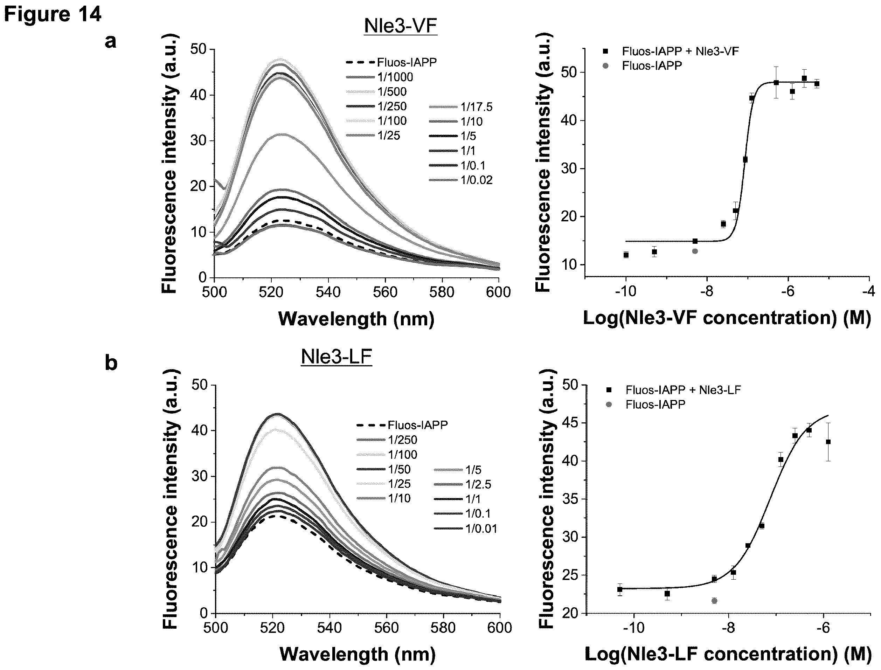

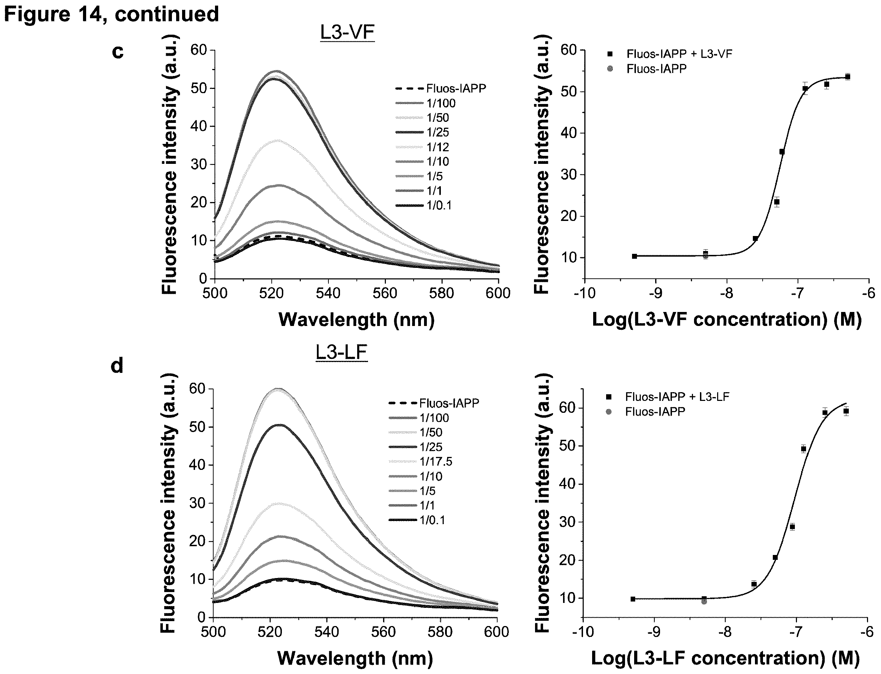

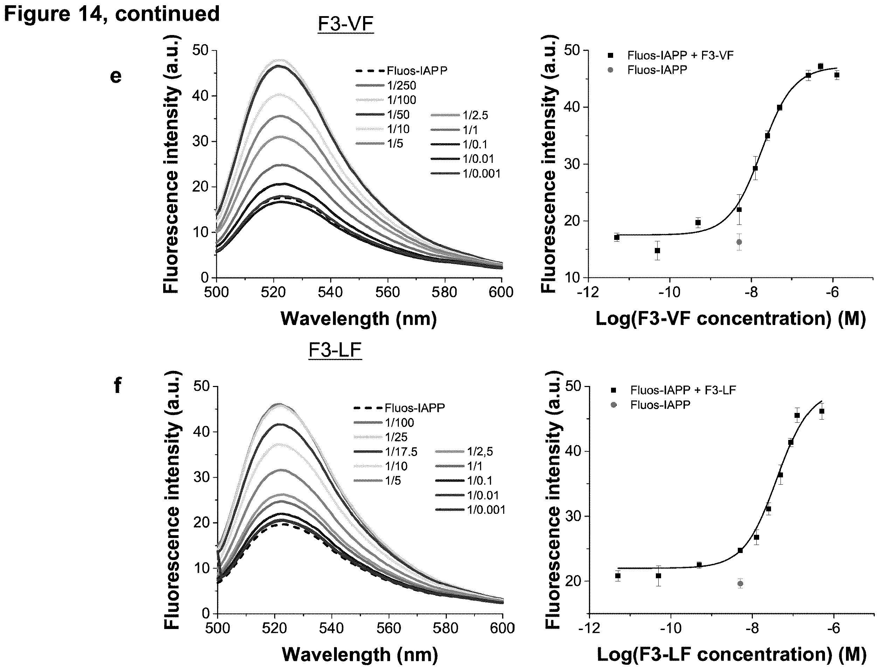

**Figure 14** shows the determination of binding affinities of IAPP/ACM interactions by fluorescence spectroscopic titrations. Fluorescence emission spectra of Fluos-IAPP (5 nM) alone or in the presence of different molar ratios of ACMs as indicated (Fluos-IAPP/ACM) (pH 7.4) are shown on the left side of each figure panel; spectra are from one assay out of three. Data on the interactions of Fluos-IAPP with **(a)** Nle3-VF, **(b)** Nle3-LF, **(c)** L3-VF, **(d)** L3-LF, **(e)** F3-VF, and **(f)** F3-LF are shown as indicated. On the right side of each figure panel, the corresponding binding curves are shown; data are means ± SD from 3 binding curves. Determined app. K_{D}s (means ± SD from 3 binding curves) are in Table 1.

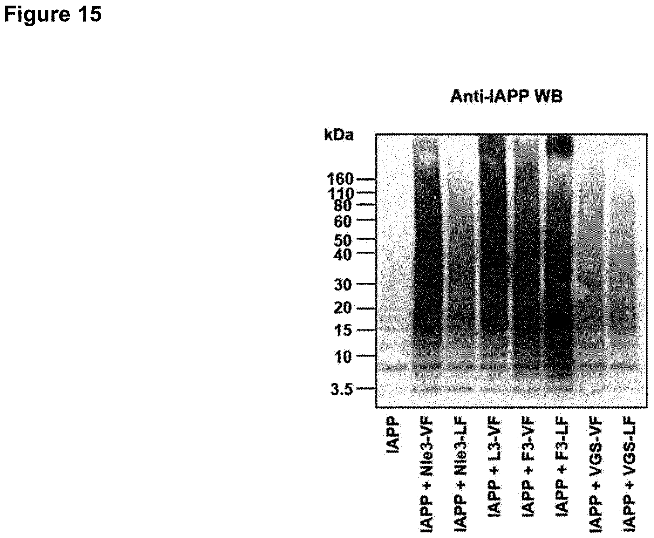

**Figure 15** shows that IAPP/ACM interactions yield hetero-di-/-tri-/-tetramers and large amounts of poorly resolved medium-to-high MW hetero-assemblies. Characterization of hetero-complexes in aged mixtures of IAPP with 5 ACMs (IAPP/peptide, 1/2; IAPP, 30 µM; pH 7.4; 7 days) via cross-linking, NuPAGE, and Western blot with anti-IAPP antibody. For comparison, assemblies present in aged IAPP (30 µM; pH 7.4; 7 days) and its mixtures with non-inhibitors VGS-VF and VGS-LF (IAPP/peptide, 1/2; IAPP, 30 µM; pH 7.4; 7 days) are also shown.

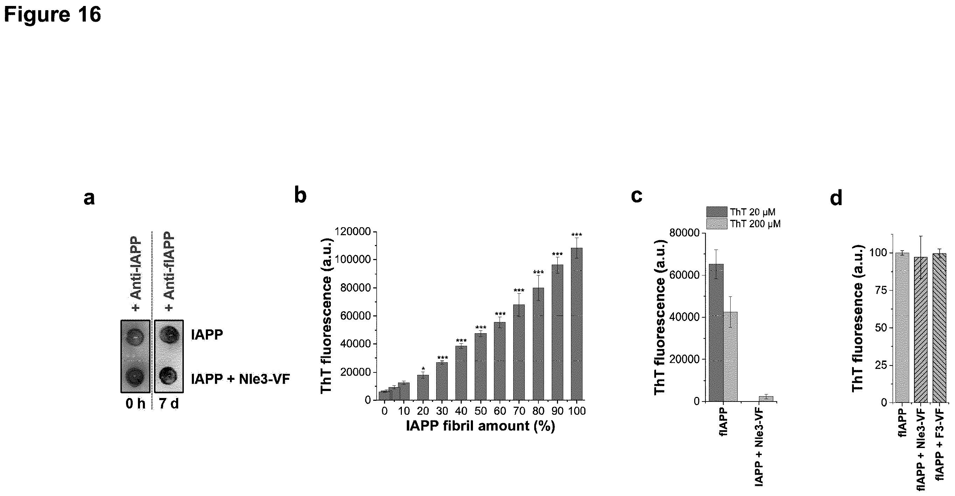

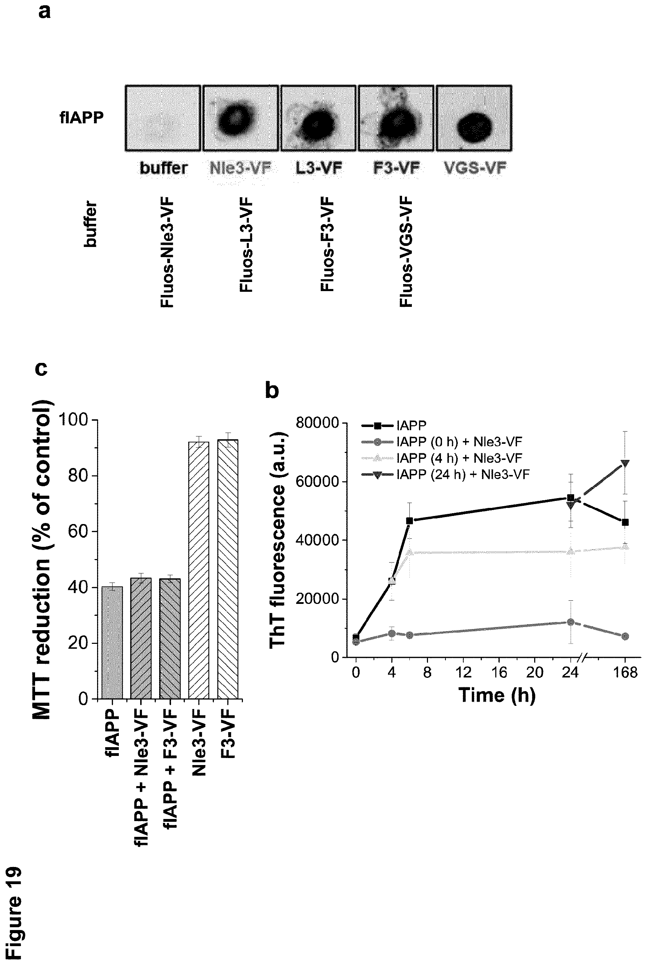

**Figure 16** shows the evidence that "ThT invisible" fibrils found in IAPP/ACM mixture are neither fIAPP rests which were not detected by ThT nor fIAPP with non-specifically bound ACM. a Dot blot analysis shows that same amounts of IAPP were present in aliquots of IAPP alone or IAPP/Nle3-VF mixtures used for ThT binding, MTT reduction assays, and TEM. These results excluded the possibility that the lack of ThT binding of the fibrils found in aged IAPP/Nle3-VF mixtures might be due to the presence of less amounts of fIAPP in the aliquots of the mixtures than in the aged IAPP alone solutions, e.g. caused by fIAPP sticking to microtube walls in the presence of Nle3-VF. Aliquots (equal volumes) from freshly made (0 h) or 7 days aged solutions of IAPP (fIAPP; 16.5 µM) or IAPP/Nle3-VF (1/2) mixtures were spotted onto nitrocellulose membrane. IAPP (1.3 µg) in freshly made (0 h) solutions was quantified with an anti-IAPP antibody whereas in 7 days aged solutions (consisting mostly of fibrils) by a fibril-specific anti-fIAPP antibody4. b The applied ThT assay had a high fIAPP detection sensitivity. Aged IAPP (16.5 µM; 7 days) consisting mostly of fIAPP (based on TEM and ThT binding (Fig. id & 2f) was serially diluted as indicated and fibrils were quantified by the ThT binding assay. ThT signals that differed significantly from the buffer were found for fIAPP concentrations >3.3 µM corresponding to 20% of total fIAPP amount. Data are means ± SD (n=3); ^{∗∗∗}P<0.01, ^{∗}P<0.05 (1-way ANOVA & Bonferroni). c Lack of ThT reactivity of fibrils in IAPP/Nle3-VF mixtures is not due to competition between ThT and Nle3-VF for binding to fIAPP. ThT binding of fIAPP (16.5 µM; 96 h aged) and an IAPP/Nle3-VF (1/2) mixture (96 h aged) was determined using 20 and 200 µM ThT and no differences were observed (data are means ± SD from 3 assays; buffer values were subtracted). d fIAPP does not lose its ThT binding potential after co-incubation ("coating") with ACMs. fIAPP (IAPP 16.5 µM, 9 days aged) before and 1 day after co-incubation with Nle3-VF or F3-VF (33 µM). Data are means ± SD from 3 assays; see also related assay in Fig. 19b.

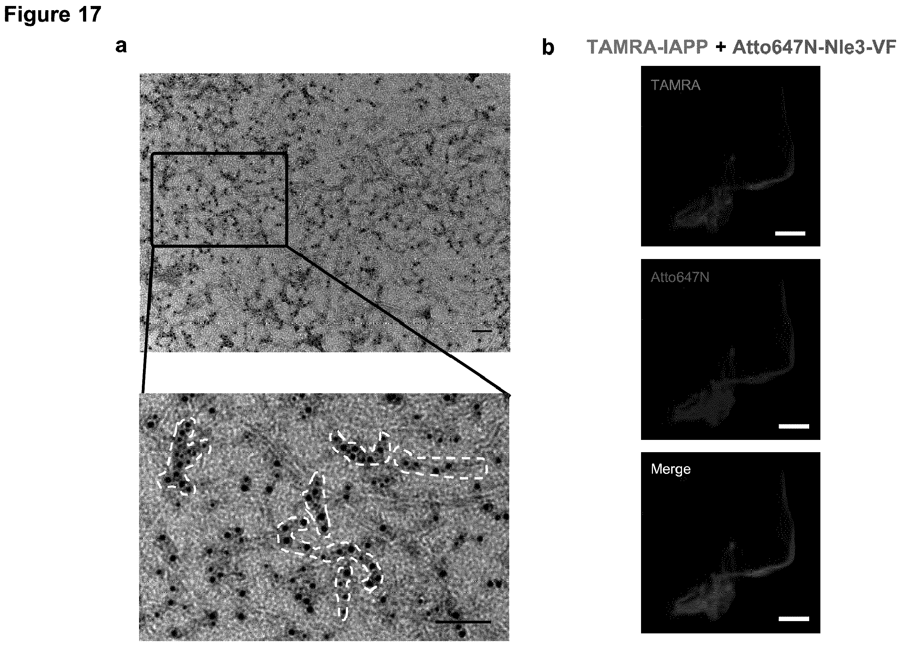

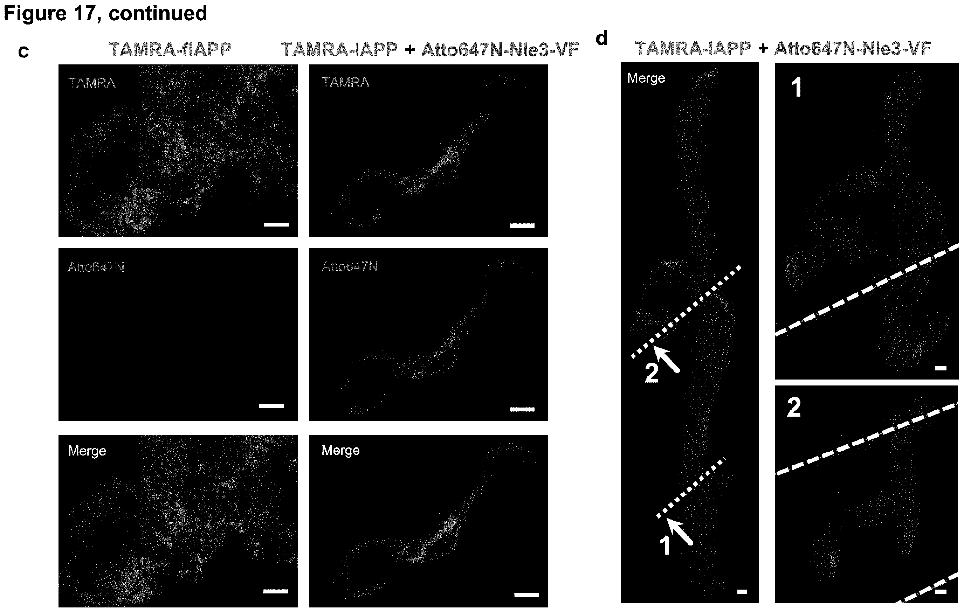

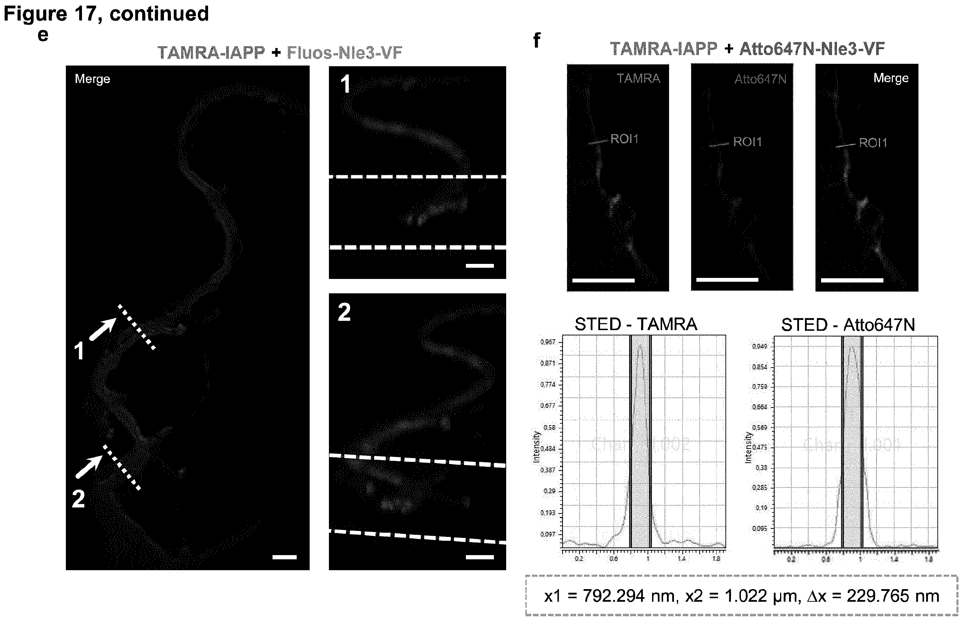

**Figure 17** shows additional TEM, CLSM, and STED evidence for IAPP/ACM co-assembly into nanofibers and supramolecular nanofiber bundles. **a** Immunogold TEM image of fibrils in aged IAPP/Nle3-VF mixture (IAPP, 16.5 µM; 1/2, 7 days aging) reveals fibrils which bind both the anti-fIAPP (IAPP fibril specific; 5 nm gold) and the anti-Aβ antibody (Nle3-VF; **10** nm gold). Highlighted areas depict fibrils which bind to both antibodies. Scale bars, 100 nm. **b** Confocal laser-scanning microscopy (CLSM) images of nanofiber co-assemblies in aged IAPP/Nle3-VF (1/2) mixture containing TAMRA-IAPP and Atto647N-Nle3-VF (10%) (IAPP(total) 16.5 mM, 7 day-aging). Scale bars, 5 µm. **c** STED images of nanofiber co-assemblies in aged IAPP or IAPP/Nle3-VF (1/2) mixture as indicated containing TAMRA-IAPP and Atto647N-Nle3-VF (10%) (IAPP(total) 16.5 mM, 7 days aging). Scale bars, 1 µm. **d** 3D reconstructions of z-stacks/still images of TAMRA-IAPP/Atto647N-Nle3-VF nanofiber co-assemblies shown in Fig. 3d. Arrows and dashed lines in the left panel indicate view of the sections shown in the right panel. Scale bars, 1 µm. **e** 3D reconstructions of z-stacks/still images of TAMRA-IAPP/Fluos-Nle3-VF nanofiber co-assemblies found in the 2PM studies of Fig. 3d. Arrows and dashed lines in the left panel indicate view of the sections shown in the right panel. Scale bars, 5 µm. **f** Example of the estimation of the width of a TAMRA-IAPP/Atto647N-Nle3-VF heteromeric nanofiber bundle (prepared as in **c)** determined with the "full-width-at half-maximum" values of an intensity based line-profile plot. Top panel, STED image; green line indicates region of interest (ROI) chosen for measurement; scale bar, 5 µm. Bottom panel, intensity plots of Atto647N and TAMRA channels; Dx, heteromeric nanofiber bundle width measured at half-maximum of the peak height.

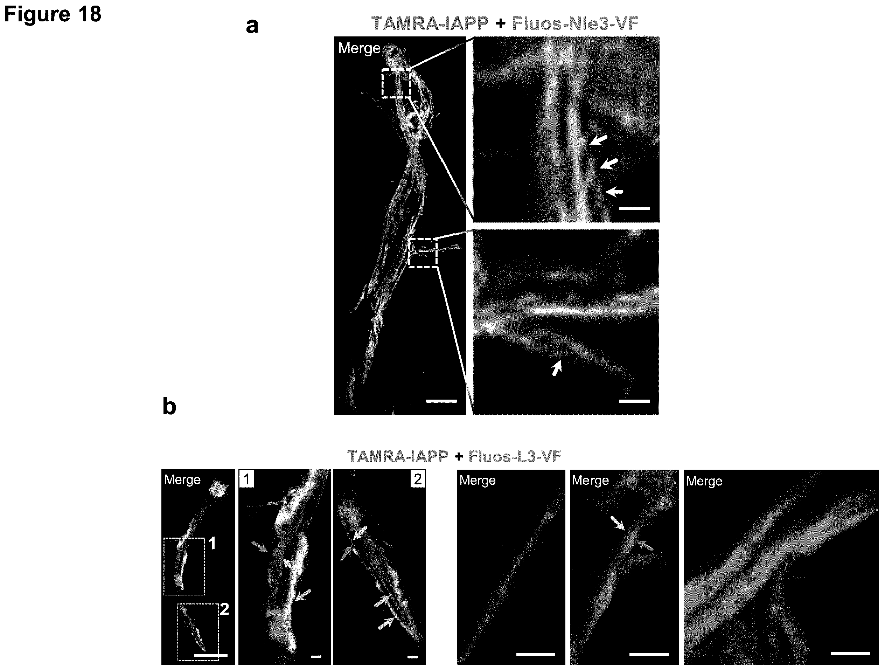

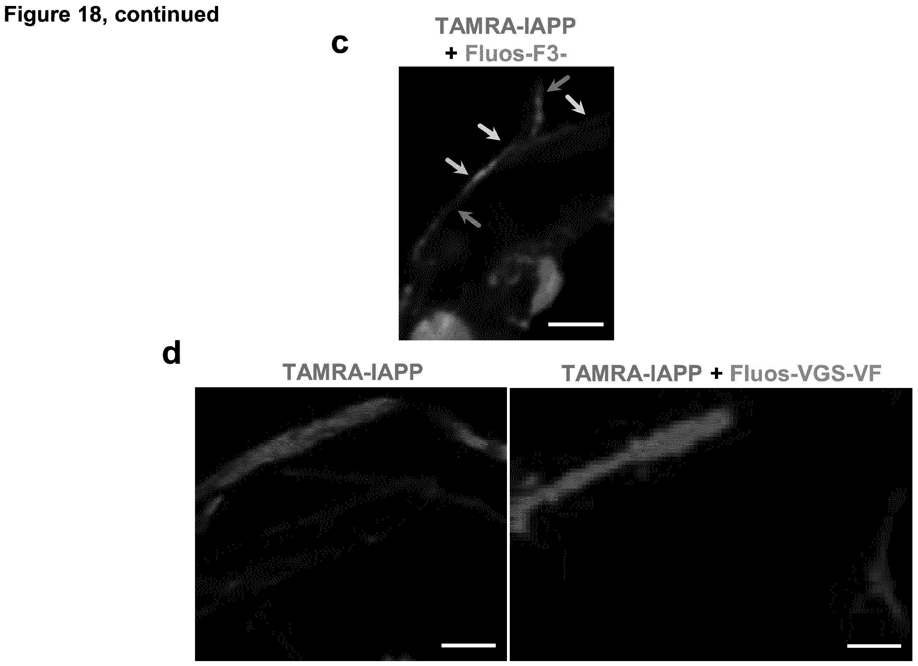

**Figure 18** shows additional 2PM evidence for supramolecular IAPP/ACM nanofiber co-assemblies. a 2PM image of a huge loop-like co-assembly (shown in Fig. 3g) found in aged TAMRA-IAPP/Fluos-Nle3-VF (1/2) mixtures (TAMRA-IAPP 16.5 µM; aging 6 days). In the insets, magnified image parts show µm-sized bicolored rods (widths ~1.7 µm, lengths ~5 µm) (short white arrows) as potential "building blocks" of braided parts of the co-assembly. Scale bars: 50 µm (insets 5 µm). **b** 2PM images of fibrous superstructures including tape-like heteromeric nanofiber bundles (right panel) found in aged TAMRA-IAPP/Fluos-L3-VF mixtures (TAMRA-IAPP 16.5 µM, 1/2, 7 day-aging). Colored arrows in the insets indicate fibrillar stacks of the two peptides arranged in parallel (red arrows, TAMRA-IAPP; green arrows, Fluos-L3-VF); or overlaying (yellow arrows). Scale bars: 50 µm in the image of the left panel and 5 µm for the magnified areas 1 and 2 and all images of the right panel. **c** 2PM images of heteromeric fibrous nanofiber bundles found in aged TAMRA-IAPP/Fluos-F3-VF mixtures (TAMRA-IAPP 16.5 µM, 1/2, 7 day-aging); color code for arrows as in **a;** scale bars, 5 µm. **d** 2PM images of fibrillar assemblies found in aged TAMRA-IAPP (16.5 µM, 7 day-aged) (left) and its aged mixture with the non-inhibitor Fluos-VGS-VF (1/2, 7 days aged) (right). These latter mixtures consisted mostly of fIAPP bundles. Scale bars, 5 µm.

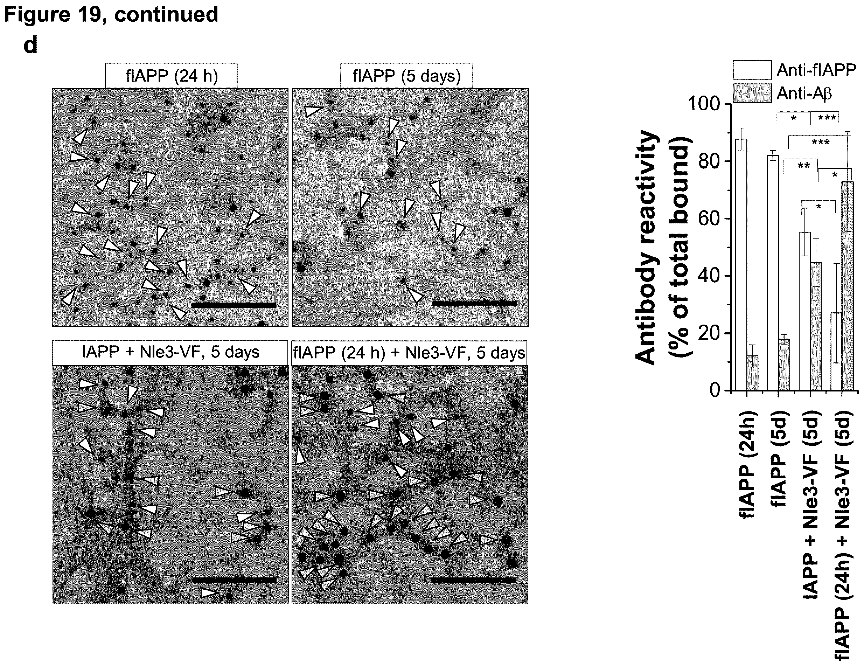

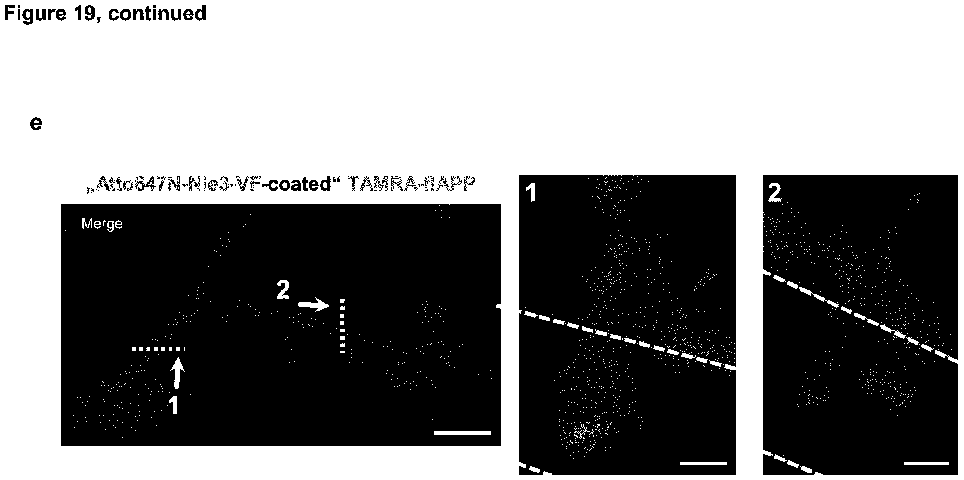

**Figure 19** shows that "ACM-coated" fIAPP are distinct from "ThT-invisible" and non-toxic hf-IAPP/ACM and their formation is not a major reason for ACM inhibitory activity on IAPP amyloid self-assembly. **a** Dot blot analysis reveals that both ACMs and the non-inhibitor VGS-VF bind fIAPP. Membranes containing spotted fIAPP (40 mg) were probed with N-terminal fluorescein-labeled peptides (Fluos-ACMs and Fluos-VGS-VF, 0.2 µM). Representative results from 2-3 assays are shown. **b** Addition of Nle3-VF to already nucleated IAPP fibrillogenesis does not affect the amount of ThT-reactive fibrils. ThT binding was measured in IAPP (16.5 µM) alone or its mixtures with Nle3-VF (33 mM) at the indicated time points before and after Nle3-VF addition. Data are means ± SD from 3 assays. **c** Addition of ACMs to preformed fIAPP does not affect fIAPP cytotoxicity. ACMs (Nle3-VF and F3-VF; 33 µM) were added to preformed fIAPP (16.5 µM, 7 days aged). Following co-incubation for 1 day, fIAPP alone, its mixtures with the ACMs, and ACMs alone were added to RIN5fm cells (fIAPP, 500 nM). Cell damage was determined via MTT reduction. Data of fIAPP and its mixtures are means ± SD of 6 wells from 2 assays (n=3 each). Data of ACMs alone are from 1 assay (n=3); additional data on the lack of cytotoxic effects of the ACMs are in Fig. 13c. **d** Immunogold TEM reveals significant differences between the antibody binding ability of fibrils in aged IAPP/Nle3-VF mixtures (hf-IAPP/Nle3-VF) and the fibrils in "Nle3-VF-coated" fIAPP solutions. Shown are immunogold TEM images of fIAPP solutions (24 h or 5 day-aged), IAPP/Nle3-VF mixtures (1/2; IAPP 16.5 µM, 5 day-aged), and Nle3-VF-coated fIAPP as indicated. Nle3-VF-coated fIAPP was made by adding Nle3-VF (33 µM) to preformed fIAPP (IAPP (16.5 µM) aged for 24 h) and co-incubating for 5 days. fIAPP was detected by anti-fIAPP specific antibody (5 nm gold nanoparticles; white arrowheads) and Nle3-VF by anti-Aβ antibody (10 nm gold nanoparticles; orange arrowheads) exhibiting a 10-20% NSB to fIAPP (see antibody binding quantification graph; right side). Scale bars: 100 nm. On the right side, the quantification of antibody binding of the four different incubations expressed as antibody reactivity (% of total bound) is presented ("d", days). Nle3-VF-coated fIAPP bound significantly more anti-Aβ antibody than fibrils in IAPP/Nle3-VF mixtures; ^{∗∗∗}P<0.001, ^{∗∗}P<0.01, ^{∗}P<0.05 by one-way ANOVA & Bonferroni. **e** 2PM examination of Nle3-VF-coated fIAPP reveal a distinct morphology that is different from hf-IAPP/Nle3-VF. In contrast to hf-IAPP/Nle3-VF, Nle3-VF-coated fIAPP consisted of fIAPP bundles randomly covered by large amorphous Fluos-Nle3-VF aggregates. 3D reconstructions of z-stacks/still images are shown. Arrows and dashed lines in the left panel indicate view of the sections shown in right panel. Scale bars, 20 µm for the image in the left panel and 5 µm for image sections 1 and 2.

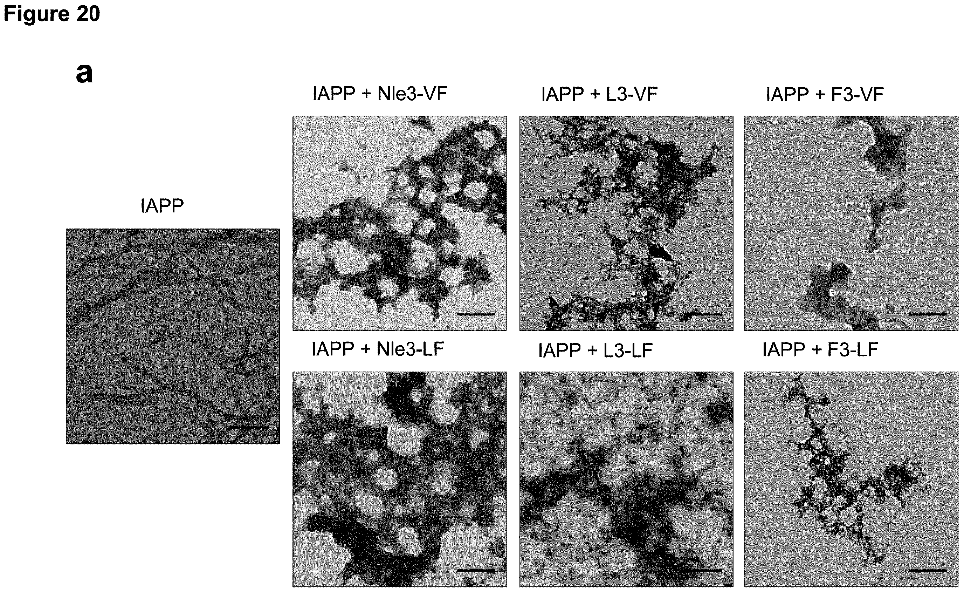

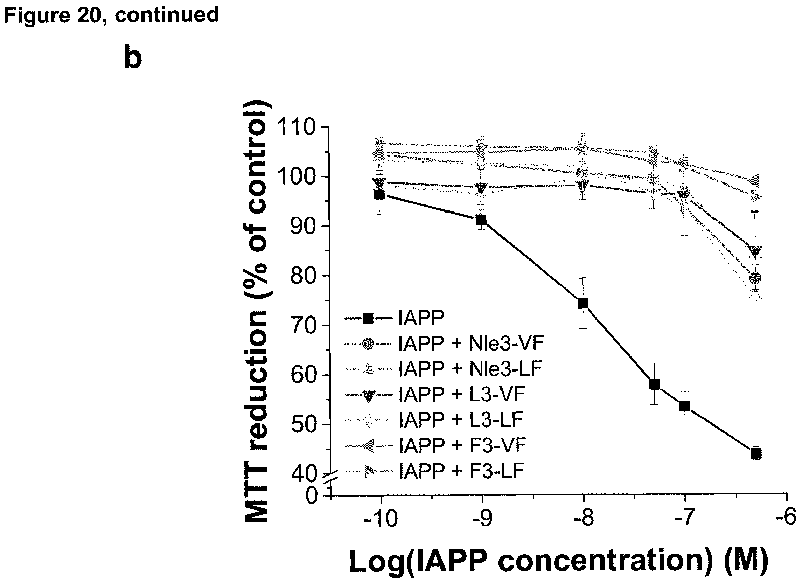

**Figure 20** shows that early IAPP/ACM co-assemblies are amorphous and non-cytotoxic. **a** Early IAPP/ACM co-assemblies consist mostly of amorphous aggregates. TEM analysis of 24 h aged incubations of IAPP (16.5 µM) and its mixtures with ACMs (33 µM). Scale bars: 100 nm. **b** Early non-fibrillar IAPP/ACM co-assemblies are non-cytotoxic. Incubations of IAPP (16.5 µM) and its mixtures with ACMs (1/2; 24 h-aged) were added to RIN5fm cells and cell damage was determined via MTT reduction (means ± SD, 3 assays, n=3 each).

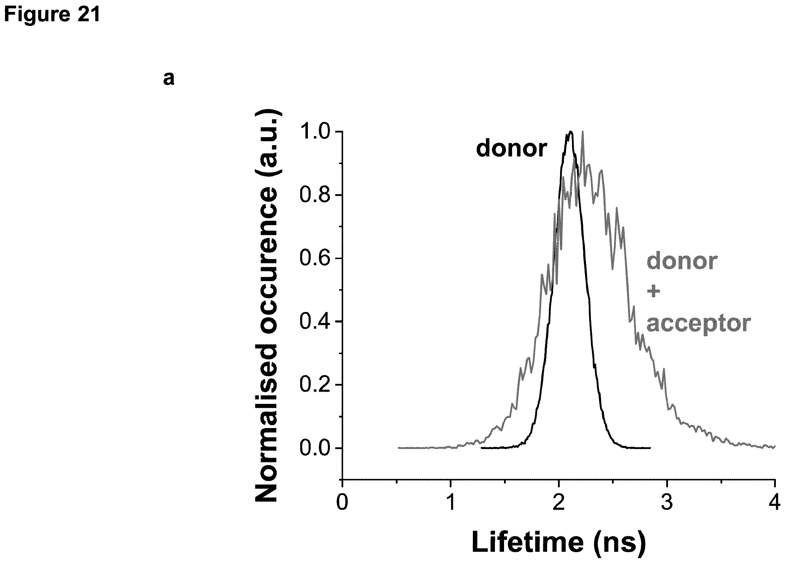

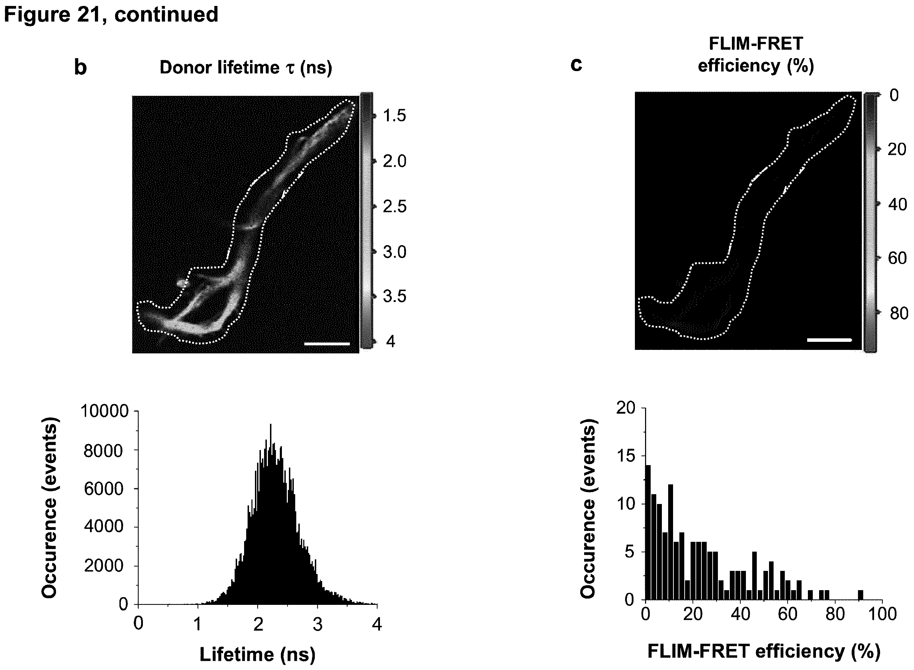

**Figure 21** shows that FLIM-FRET analysis of fibrillar co-assemblies found in Fluos-Nle3-VF (33 µM) following addition of preformed TAMRA-fIAPP (10%) reveal no significant FLIM-FRET events. **a** Fluorescence lifetime of donor (Fluos-Nle3-VF) without or with acceptor (TAMRA-fIAPP) reveals no changes in donor lifetime in the presence of acceptor. **b** Upper part, FLIM image of the fibrillar co-assembly showing donor life time; life time as indicated; scale bar, **10** µm. Lower part, donor fluorescence lifetime distributions (~2 ns) in the fibrillar co-assembly. **c** Upper part, FLIM-FRET efficiency (%) observed in the fibrillar co-assembly; efficiency distribution as indicated; scale bar, 10 µm. Lower part, FLIM-FRET efficiency distribution in the fibrillar co-assembly (<40%).

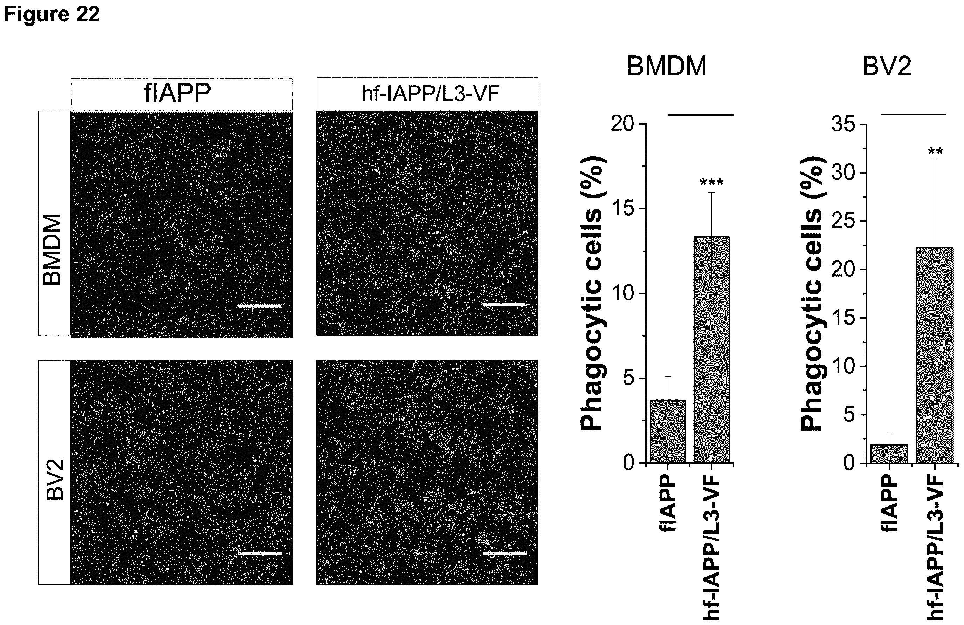

**Figure 22** shows that IAPP/L3-VF-nanofibers are much more efficiently phagocytosed than fIAPP by primary murine BMDMs and cultured murine BV2 microglia. Left panel, representative microscopic images of BMDMs or BV2 cells as indicated following incubation (6 h, 37°C) with TAMRA-fIAPP (3.3 µM) (left side) or hf-TAMRA-IAPP/L3-VF (3.3 µM) (right side) as indicated (compare with Fig. **4h**); red dots indicate TAMRA-IAPP; scale bars, 100 µm. Right panel, amounts of BMDM (mid) or BV2 (right) cells (% of total) that phagocytosed fIAPP and hf-IAPP/L3-VF. Data are means ± SD from 5 independent peptide preparations studied in one cell assay with each well analyzed in 3 fields of view; ^{∗∗∗} P<0.001, ^{∗∗} P<0.01 (unpaired t-test).

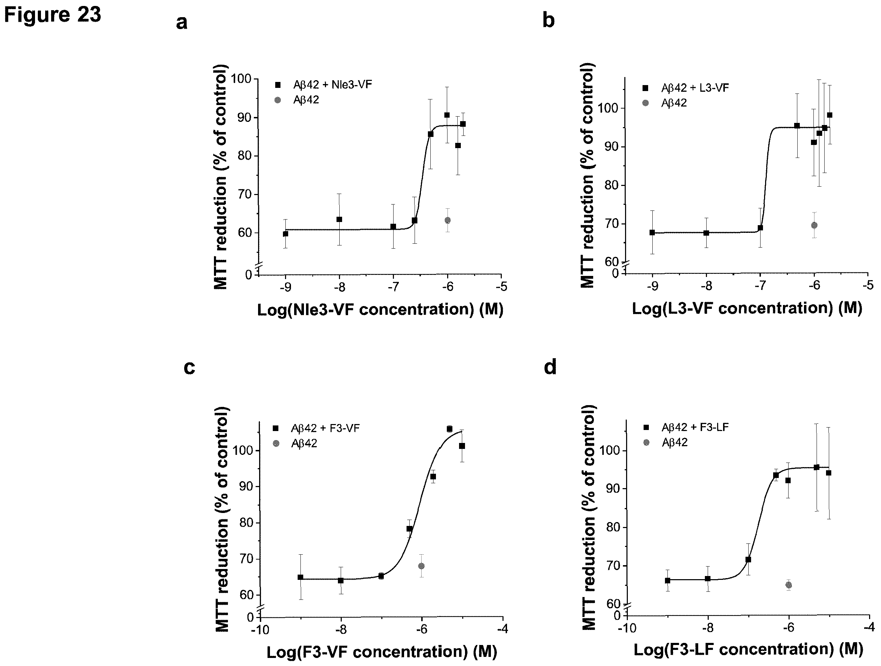

**Figure 23** shows the determination of IC₅₀ values of inhibitory effects of ACMs on formation of cell-damaging Aβ42 assemblies. PC12 cells were incubated with 6-day aged Aβ42 alone (1 µM) or its mixtures with different molar ratios of ACMs and cell damage was determined by the MTT reduction assay for mixtures of Aβ42 with Nle3-VF **(a),** L3-VF **(b),** F3-VF **(c)** and F3-LF **(d);** red symbols show effects of Aβ42 alone. Data and IC₅₀ values are means ± SD from 3 assays (n=3 each).

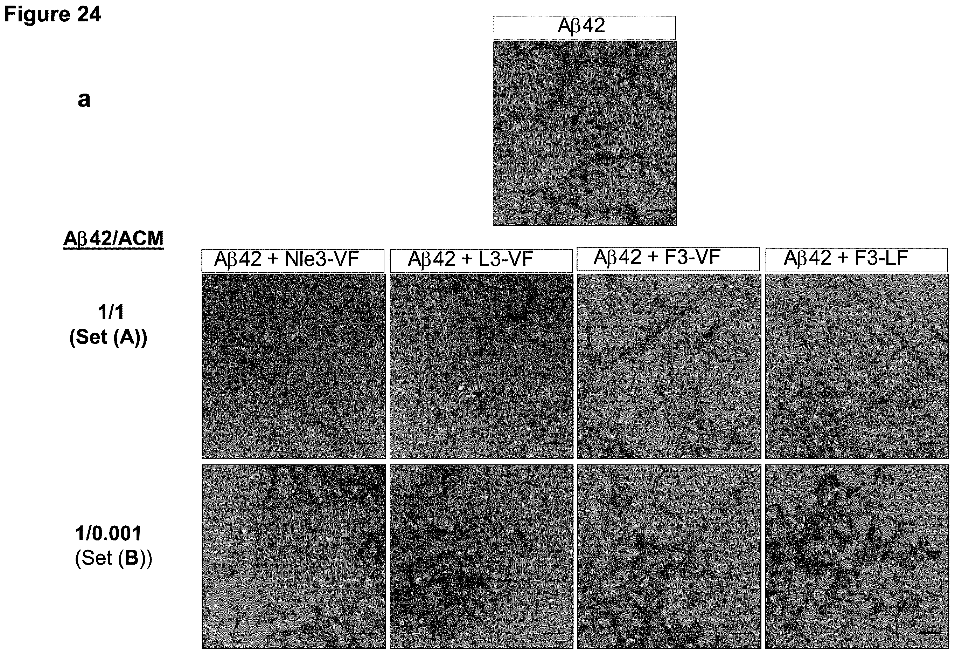

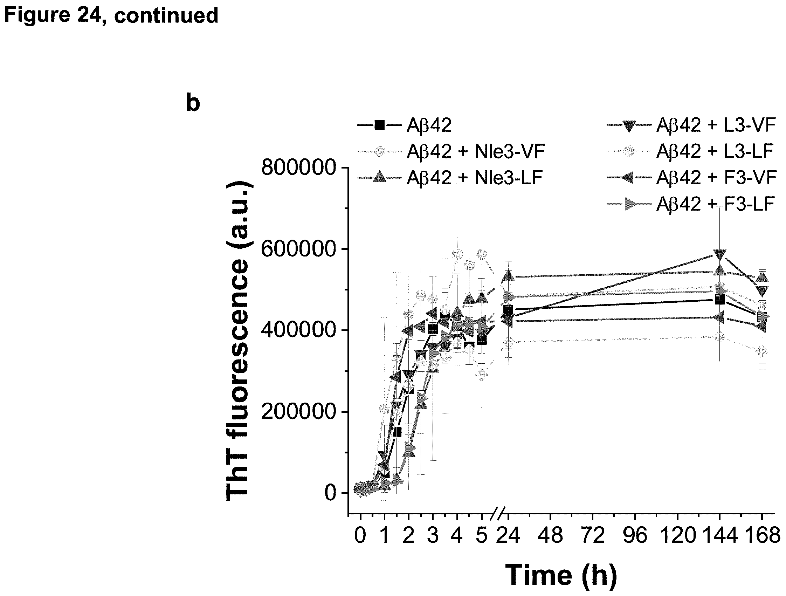

**Figure 24** shows that the formation of long fibrils in AP42/ACM mixtures is linked to ACM inhibitory effect on Aβ42 fibrillogenesis. **a** TEM images of two 6 day-aged sets of mixtures, i.e. the AP42/ACM 1/1 mixtures (set (A); full inhibition of Aβ42 fibrillogenesis according to ThT binding (Fig. **6a****))** and the AP42/ACM 1/0.001 mixtures (set (B); no inhibition of fAβ42 fibrillogenesis according to ThT binding (shown in **b)** as compared to 6 day-aged Aβ42 alone (Aβ42, 5 µM). All four "ThT-negative" mixtures of set (A) consisted of 2-4-fold longer fibrils than Aβ42 aged under the same conditions, which consisted of "ThT-positive" fAβ42(Fig. 6d). By contrast, all four ThT-positive mixtures of set (B) consisted of fibrils of identical appearance and widths to fAβ42. Scale bars: 100 nm. **b** Aβ42 amyloid self-assembly is not affected when an AP42/ACM molar ratio of 1/0.001 is used. Aβ42 fibrillogenesis (5 µM) alone or with each of the ACMs (5 nM) assessed by ThT binding (means ± SD, 3 assays). Of note, TEM images in **a** are from 6 day-aged solutions made as in **b** but w/o ThT.

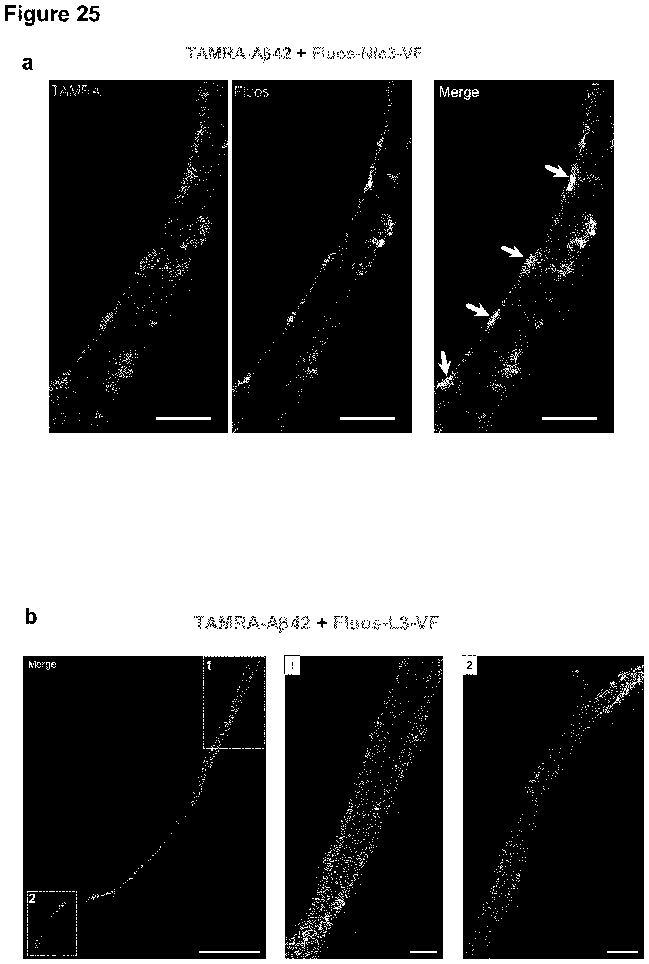

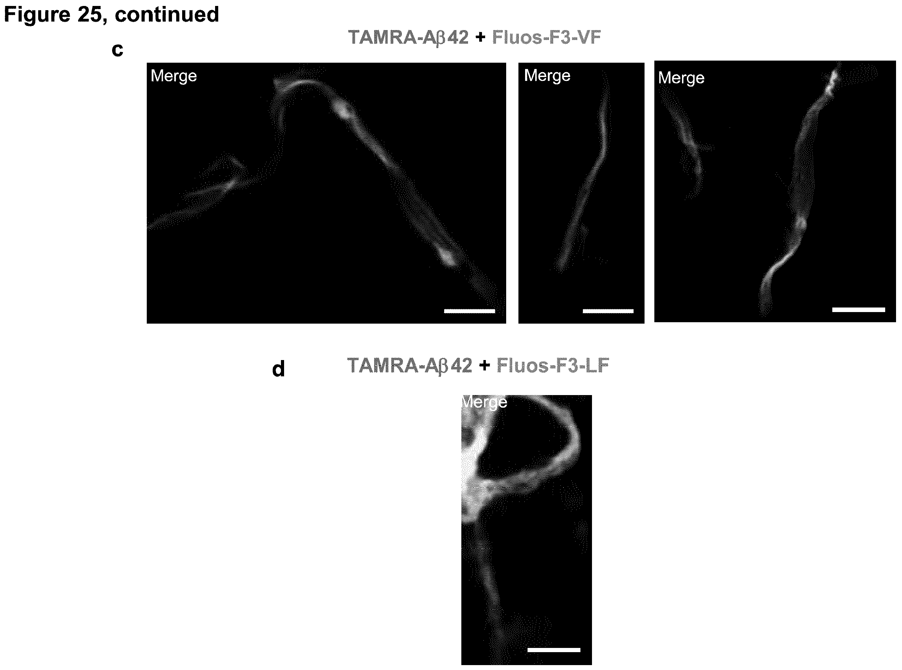

**Figure 25** shows 2PM images of diverse fibrous AP42/ACM superstructures. **a** Supramolecular Aβ42/Nle3-VF nanofiber co-assembly containing 50%TAMRA-Aβ42/Fluos-Nle3-VF (merged image presented in Fig. 6e) is shown here with each channel (CH1 (TAMRA) and CH2 (Fluos)) separately and merged. Scale bars, **10** µm. **b** Huge AP42/L3-VF ribbon-/nanotube-like nanofiber co-assembly (TAMRA-Aβ42/Fluos-L3-VF (50%); Aβ42(total), 5 mM; 1/2, 6 days). Scale bars, 100 µm and **10** µm in magnified areas 1 and 2. **c, d** Aβ42/F3-VF and Aβ42/F3-LF ribbon-like nanofiber co-assemblies (TAMRA-Aβ42/Fluos-ACM (50%); Aβ42(total) 5 µM, 1/2, 6 days) as indicated. Scale bars, **10** µm.

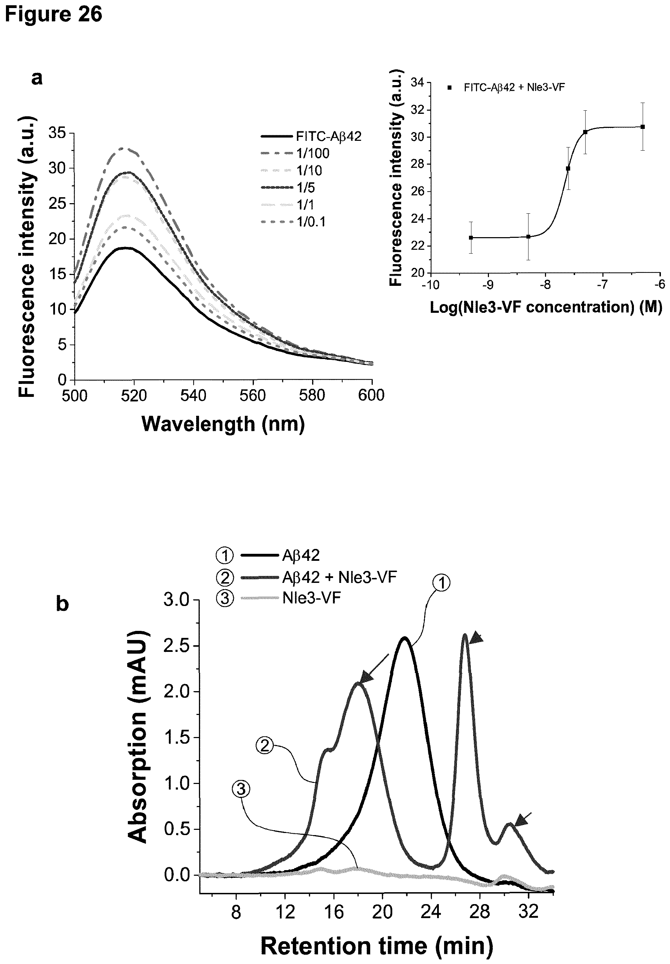

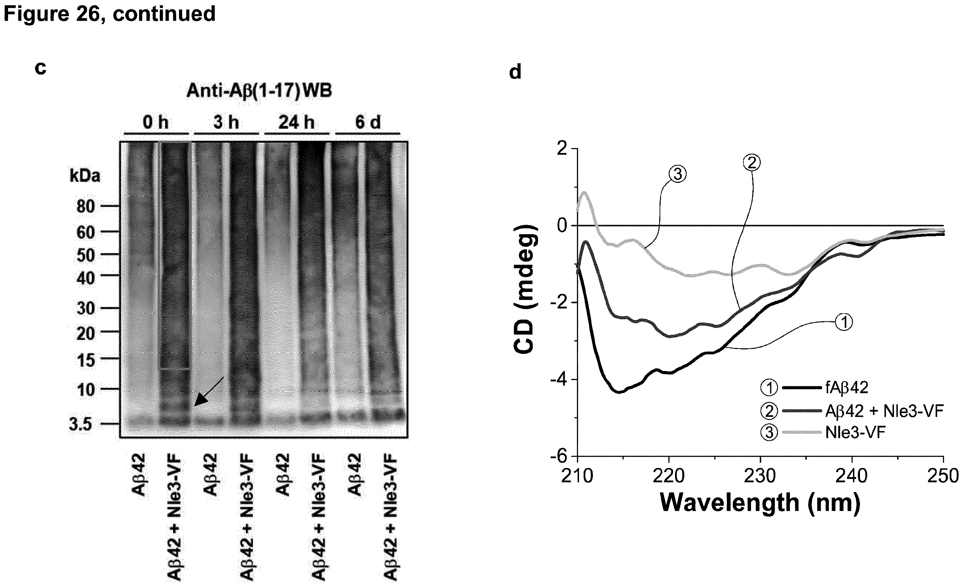

**Figure 26** shows a characterization of Aβ42/ACM interactions and hetero-complexes by fluorescence spectroscopy, size-exclusion chromatography (SEC), cross-linking, and far-UV CD spectroscopy. **a** Nle3-VF binds Aβ42 with low nanomolar affinity as determined by fluorescence spectroscopic titrations. Fluorescence spectra of FITC-Aβ42 (5 nM) and its mixtures with Nle3-VF (pH 7.4) at indicated molar ratios (data from one representative binding assay (n=3)). Inset, binding curve; data means ± SD from 3 assays; app. K_{D}, 14.5 ± 8.0 (Table 2). **b** Characterization of hf-Aβ42/Nle3-VF via SEC. Chromatograms of aged (6 days) Aβ42 (5 µM), Nle3-VF (5 µM), and their 1/1 mixture (5 µM each) consisting mostly of ThT-invisible fibrils (Fig. 6d) are shown. Arrow indicates high MW hetero-assemblies; arrowheads indicate Aβ42 and Nle3-VF monomers. **c** Kinetics of Aβ42/Nle3-VF co-assembly as followed by cross-linking with glutaraldehyde. This was performed in solutions of Aβ42, Aβ42/Nle3-VF (1/2), and Nle3-VF at different incubation time points in combination with NuPAGE and WB with anti-Aβ(1-17) antibody (6E10) which recognizes Aβ42 but not the ACM. Orange box marks smear of unresolved bands corresponding of medium-to-high MW hetero-assemblies; arrow indicates hetero-dimers. **d** hf-Aβ42/Nle3-VF have less β-sheet structure than fAβ42. Far-UV CD spectra of aged (6 days) Aβ42 (5 µM), Nle3-VF (5 µM) and their 1/1 mixture (5 µM each) are shown. Under these experimental conditions Aβ42 and Aβ42/Nle3-VF mixtures consisted mostly of fibrils (Fig. 6d).

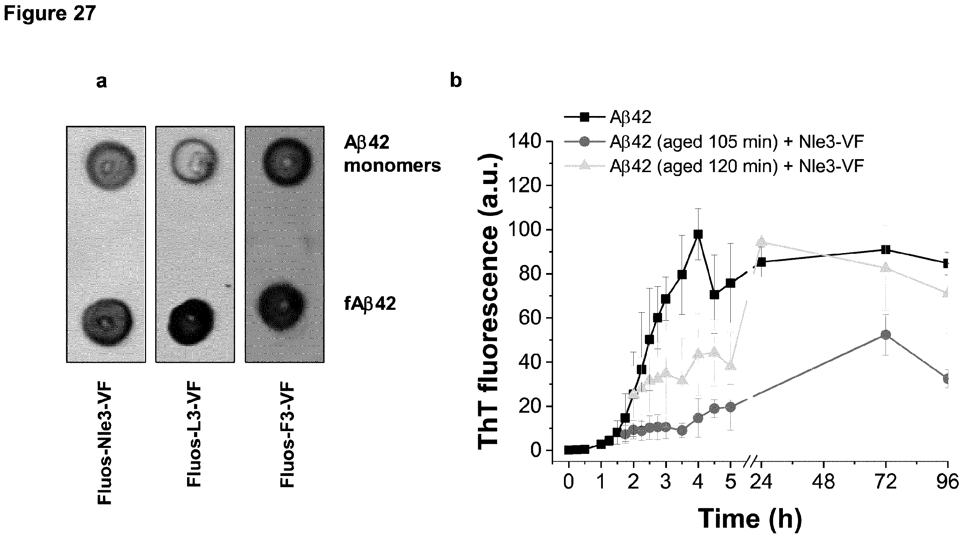

**Figure 27** shows that binding of ACMs to preformed fAβ42 does not convert fAβ42into ThT-invisible heteromeric nanofibers. **a** Dot blot analysis shows that ACMs bind to fAβ42. Membranes containing spotted fAβ42 or Aβ42 monomers (10 mg each) were probed with various Fluos-ACMs (2 µM); results are representative from 2 assays. **b** Addition of Nle3-VF to already nucleated fAβ42 fibrillogenesis does not affect amounts of already formed ThT-positive fibrils. ThT binding was measured in fAβ42(5 µM) alone or its mixtures with Nle3-VF (5 mM) at the indicated time points before and after Nle3-VF addition. Data means ± SD (3 assays except for the 24 h time point of "Aβ42 (aged 120 min) + Nle3-VF" (1 assay)).

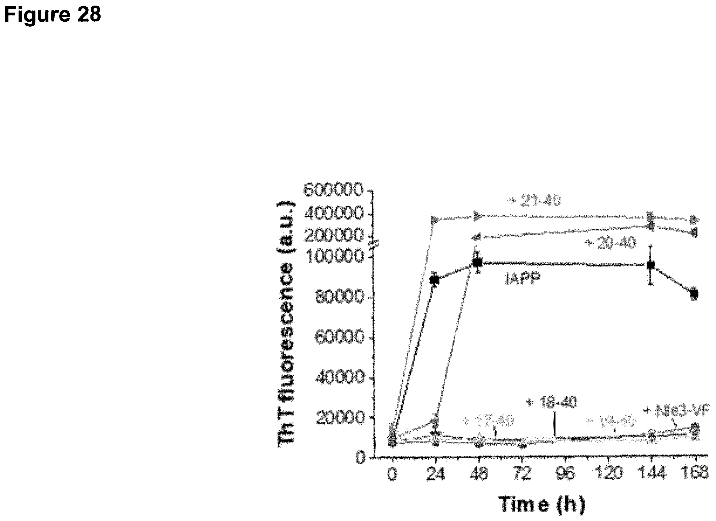

**Figure 28** shows data on the inhibitory activity of **N-terminal truncated analogs** of Nle3-VF on IAPP amyloid self-assembly. Analogs 20-40 and 21-40 cannot inhibit whereas all other analogs inhibit as full length Nle3-VF. The results indicate that N-terminal segment QKLV are not crucial for inhibitory activity. Effects of N-terminal truncated Nle3-VF analogs on IAPP amyloid self-assembly are shown. ThT binding assay of IAPP/peptide mixtures. Incubations were prepared in ThT buffer with 0.5 % HFIP containing 16.5 µM IAPP alone or its mixture with peptides (1:2); mixtures of IAPP with the corresponding analog are indicated by the "+" sign. Data are means ± SD from 3 assays.
   Applied abbreviations of the analogs as follows:
   Abbreviation **"17-40":** stands for Nle3-VF analog w/o N-terminal segment "QK"
   Abbreviation **"18-40":** stands for Nle3-VF analog w/o N-terminal segment "QKL"
   Abbreviation **"19-40":** stands for Nle3-VF analog w/o N-terminal segment "QKLV"
   Abbreviation **"20-40":** stands for Nle3-VF analog w/o N-terminal segment "QKLVF"
   Abbreviation **"21-40":** stands for Nle3-VF analog w/o N-terminal segment "QKLVFF"
**Figure 29** shows data on the inhibitory activity of **C-terminal truncated analogs** of Nle3-VF on IAPP amyloid self-assembly. C-terminal truncated analogs 15-36 does not inhibit whereas analogs 15-37 and 15-38 inhibit as full length Nle3-VF. The results indicate that C-terminal segment GVV are not crucial for inhibitory activity. Effects of C-terminal truncated Nle3-VF analogs on IAPP amyloid self-assembly are shown. ThT binding assay of LAPP/peptide mixtures. Incubations were prepared in ThT buffer with 0.5 % HFIP containing 16.5 µM IAPP alone or its mixture with peptides (1:2); mixtures of LAPP with the corresponding analogs are indicated by the "+" sign. Data are means ± SD from 3 assays.
   Applied abbreviations of the analogs as follows:
   Abbreviation "**15-38**": stands for Nle3-VF analog w/o C-terminal segment "VV"
   Abbreviation "**15-37**": stands for Nle3-VF analog w/o C-terminal segment "GVV"
   Abbreviation "**15-36**": stands for Nle3-VF analog w/o C-terminal segment "GGVV"
**Figure 30** shows data on the inhibitory activity of a **4-fold N-methylated** Nle3-VF analog termed "Nle3-LVFF" on IAPP amyloid self-assembly. The data show that Nle3-LVFF inhibits similar to the 2-fold N-methylated analogs Nle3-VF and Nle3-LF. The results indicate that N-methylation of all four residues of segment LVFF are compatible with inhibitor function. Effects of 4-fold N-methylated Nle3-VF analog termed Nle3-LVFF on IAPP amyloid self-assembly are shown. ThT binding assay of IAPP/peptide mixtures. Incubations were prepared in ThT buffer with 0.5 % HFIP containing 16.5 µM IAPP alone or its mixture with peptides (1:2) as indicated. Data are means ± SD from 3 assays.

In the following, reference is made to the examples, which are given to illustrate, not to limit the present invention.

### EXAMPLES

### Example 1: Methods

### Peptides and peptide synthesis

IAPP, IAPP-GI, rat IAPP, and their N^{a}-terminal fluorescein- or biotin-labeled analogs were synthesized by Fmoc-based solid phase synthesis (SPPS), subjected to air-oxidation, and purified by RP-HPLC. Their stock solutions were prepared in 1,1,3,3,3,3-hexafluoro-2-isopropanol (HFIP) (4°C), filtered over 0.2 µm filters (Millipore), and concentrations were determined by UV spectroscopy. TAMRA-IAPP was synthesized by overnight coupling of 5,6-carboxytetramethylrhodamine (TAMRA) (Novabiochem/Merck) to RINK-resin-bound IAPP using a 3-fold molar excess of 2-(1H-Benzotriazol-1-yl)-1,1,3,3-tetramethyluroniumhexafluorophosphate (HBTU) and a 4.5 molar excess of N,N-diisopropylethylamine (DIEA) in *N,N*-dimethylformamide (DMF). TAMRA-IAPP cleavage from the resin and RP-HPLC purification were performed as for the other labeled IAPP analogs; stocks were made in HFIP (4°C). Aβ42 was synthesized on Tentagel R PHB resin (0.18 mmol/g; Rapp Polymere) by Fmoc-SPPS. Seed-free aqueous Aβ42 stock solutions (10-20 µM) were obtained by SEC performed. Briefly, HPLC-purified Aβ42 was dissolved (1 mg/ml) in a solution of 5 M GdnHCl in **10** mM TRIS/HCl pH 6.0 and loaded onto a Superdex 75 10/300 GL column (eluent: 50 mM ammonium acetate pH 8.5, 0.5 ml/min). The monomeric Aβ42 elution peak was collected on ice, stored at 4°C and used within 1 week; peptide concentration was determined by UV spectroscopy. Fluorescein-isothiocyanate-β-Ala-labeled Aβ42 (FITC-Aβ42) and TAMRA-labeled Aβ42 (TAMPA-Aβ42) were from Bachem and HiLyte647-Aβ42 from AnaSpec; their stocks were prepared in HFIP (4°C).

All Aβ(15-40) analogs comprising ACMs, non-inhibitors, and partial segments thereof (Tables 3 and 6) were synthesized using previously described standard Fmoc-SPPS protocols and in most cases WANG-resin (0.3-0.5 mmol/g; Iris Biotech); Tentagel R PHB resin was used for Nle3, R3, and G3-VF (0.16 mmol/g; Rapp Polymere). Briefly, double couplings were usually performed using 3-fold molar excess protected amino acid and HBTU and 4.5-fold molar excess of DIEA in DMF. For difficult couplings, we applied either 2-(7-aza-1H-benzotriazole-1-yl)-1,1,3,3-tetramethyluronium-hexafluorophosphate (HATU), or 4-6-fold molar excess of protected amino acids, and/or triple couplings. N-terminal fluorescein-labeled Aβ(15-40) analogs were synthesized by coupling peptide-resins with 5,6-carboxyfluorescein (Sigma-Aldrich) using 3-fold molar excess protected amino acid and HATU and 4.5-fold molar excess of DIEA (double couplings). N-terminal Atto647N-labeled Nle3-VF was synthesized by coupling peptide-resin with Atto647N (carboxy-derivative) (ATTO-TEC) using HATU. Peptide cleavage from the resin was performed with 95% TFA/H₂O. All peptides were purified by RP-HPLC on Nucleosil 100 C18 (Grace) or Reprosil Gold 200 C18 columns (Dr. Maisch). Stock solutions were made in HFIP (4°C); peptide concentrations were determined by peptide weight or by UV spectroscopy (fluorescently labeled analogs).

All synthetic peptides were characterized by matrix-assisted laser desorption ionization (MALDI-MS) or electrospray ionization (ESI-MS) mass spectrometry (Table 6).

### Thioflavin T (ThT) binding assays

**IAPP fibrillogenesis-related studies.** Effects of the different peptides on IAPP fibrillogenesis including self- and cross-seeded fibrillogenesis were studied in combination with TEM and MTT reduction assays according to previously established ThT binding assay systems.At the indicated time points, aliquots of peptide incubations (made as described below) were gently mixed with the ThT solution (20 mM ThT in 0.05 M glycine/NaOH, pH 8.5, if not stated otherwise) in a 96-well black MTP (FluoroNunc/Thermo Fisher Scientific). ThT binding was determined immediately by measuring fluorescence emission at 486 nm following excitation at 450 nm using a 2030 Multilabel Reader VictorX3 instrument (PerkinElmer Life Sciences). ThT binding of seeds/buffer were subtracted from the data in all assays related to seeding events; in all other cases raw or normalized data are shown if not stated otherwise. All IAPP-related incubations were performed at 20°C except for the (cross-)seeded ones (RT). Preformed fIAPP were generally prepared by incubating IAPP (12 or 16.5 µM) in ThT buffer for 3-9 days (20°C), quantified by ThT binding, and verified by TEM.

Peptide incubations were performed as follows: for studying effects on IAPP fibrillogenesis, freshly made IAPP (16.5 µM) and LAPP/peptide mixtures were incubated in 50 mM sodium phosphate buffer, pH 7.4, with 100 mM NaCl containing 0.5% HFIP (abbreviated "ThT buffer") for up to 7 days. For studying effects on fIAPP-mediated seeding of IAPP fibrillogenesis, preformed fIAPP (10%) were added to freshly made IAPP (12 µM) or IAPP/peptide-mixtures (1/2) in ThT buffer; solutions were incubated for several days as indicated. To determine the detection limit of the LAPP-related ThT binding assay, fIAPP were first made by incubating IAPP (16.5 µM) in ThT buffer (7 days). Following fIAPP quantification by ThT binding and verification by TEM (Fig. id and Fig. 2f), serial fIAPP dilutions were made and ThT binding was measured as described in the first paragraph. Significance of differences between ThT binding of the various fIAPP amounts *versus* buffer alone was analyzed by one-way ANOVA and Bonferroni's Multiple Comparison test. To investigate whether ACM binding to fIAPP surfaces might compete with ThT binding, ThT binding of preformed fIAPP (16.5 µM, ThT buffer, 4 day-aged) and hf-IAPP/Nle3-VF (16.5 mM, ThT buffer, 4 day-aged) was determined using ThT solutions containing 20 or 200 µM ThT. To investigate whether binding of ACMs to fIAPP surfaces ("ACM coating" of fIAPP) might block ThT binding of fIAPP, ACM (2-fold) was added to preformed fIAPP (16.5 mM); ThT binding of ACM/fIAPP mixtures and non-treated fIAPP was determined before and following co-incubation of ACM with fIAPP (1 day) as above. To investigate effects of Nle3-VF on already nucleated IAPP fibrillogenesis, aliquots of an IAPP incubation (16.5 µM in ThT buffer) were added to the ACM at different time points of fibrillogenesis. To investigate whether hf-IAPP/ACM co-assemblies might seed IAPP fibrillogenesis, seed amounts (10%) of hf-LAPP/ACM (made by incubating IAPP (16.5 µM) with ACM (2-fold) for 7 days in ThT buffer) were added to freshly made IAPP (12 µM in ThT buffer); incubations were performed for 48 h. Seeding effects of preformed fIAPP (10%) made under the same conditions as hf-IAPP/ACM were studied in parallel. Dot blot analysis (Fig. 16a) confirmed that similar amounts of fIAPP and hf-IAPP/ACM were used for seeding or other assays. To determine the effects of ACMs on fAβ42-mediated cross-seeding of IAPP fibrillogenesis, seed amounts (10%) of fAβ42(made by incubating Aβ42 (88 µM) in ThT buffer containing 1% HFIP for 19 days at 37°C; fibril formation confirmed by ThT binding and TEM) were added to freshly made IAPP (12 µM in ThT buffer) or LAPP/peptide mixtures (1/2); incubations were performed for 48 h.

**ACM fibrillogenesis-related studies.** To study the fibrillogenic potential of ACMs, peptides and Aβ40 (positive control) (100 µM) were incubated in **10** mM aqueous sodium phosphate buffer, pH 7.4 (1% HFIP) for 4 days. ThT fluorescence was measured at 0 h and 4 days by mixing an aliquot with a ThT containing solution (121 µM ThT, 0.05 M glycine/NaOH, pH 8.5); buffer values were subtracted from the data shown in Fig. 13b. The absence of fibrils and cytotoxic aggregates from these solutions was confirmed by TEM (Fig. 13a) and MTT reduction assays (Fig. 13c).

**Aβ42 fibrillogenesis-related studies.** To study effects of the different peptides on Aβ42 fibrillogenesis, synthetic Aβ42 isolated from SEC (see "Peptides & peptide synthesis") was used. Peptide incubations were performed in the presence of ThT in 96-well black MTPs (FluoroNunc, Thermo Fisher Scientific). Incubation conditions for all assays were (if not stated otherwise): Aβ42 (5 µM) alone or its mixture with the peptide (at the indicated molar ratios) in 45 mM ammonium acetate, pH 8.5, containing **10** µM ThT (37°C); MTPs were shaken (500 rpm; orbital shaker (CAT S20)) for the first 5 h of the fibrillogenesis. ThT fluorescence was measured with a 2030 Multilabel Reader VictorX3 instrument at the indicated time points as under IAPP-related assays. Values of seeds or buffer alone were subtracted from the data in self-/cross-seeding assays; all other data shown are raw data except for data in Fig. 27b which were normalized).

For studying effects of ACMs on fAβ42-mediated seeding of Aβ42, preformed fAβ42(made by incubating Aβ42 (5 µM) as above but w/o ThT for 6 days (TEM, Fig. 6d)) were added to freshly made Aβ42 (5 µM) or AP42/ACM mixtures (5 µM each; made on ice) just before the addition of ThT (10 µM). fAβ42seed concentration was 0.5 µM (10%) and incubations (37°C) were performed as above. Of note, fAβ42 were quantified/verified by ThT binding, dot blots, and TEM. For studying whether hf-Aβ42/ACM might seed Aβ42 fibrillogenesis, Aβ42 (5 µM) and AP42/ACM mixtures (5 µM each) were first incubated for 6 days w/o ThT as described in the top section to obtain fAβ42 and hf-Aβ42/ACM; fibrils were quantified/verified by ThT binding (fAβ42), TEM (fAβ42 and hf-Aβ42/ACM), and dot blots. Seed amounts of fAβ42or hf-AP42/ACM (10%, 0.5 mM) were then mixed (on ice) with freshly made Aβ42 (5 µM) in 45 mM ammonium acetate, pH 8.5, and following addition of ThT (10 µM) incubations (37°C) were performed as described above. To study effects of ACMs added at post-nucleation time points of Aβ42 fibrillogenesis, Aβ42 (5 µM) was incubated in the presence of ThT as described in the top section and mixed with the ACM (1/1) at the indicated time points of fibrillogenesis. Effects of ACMs on fIAPP-mediated cross-seeding of Aβ42 were studied as follows: Aβ42 (10 µM) alone and AP42/ACM mixtures (1/2) were prepared as above on ice. Preformed fIAPP (2 µM) (made by incubating IAPP (128 µM) in ThT buffer for 9-12 days; fIAPP quantified/verified by ThT and TEM) were added to the above solutions just prior to the addition of ThT (10 mM). Final composition of the assay buffer was: 45 mM ammonium acetate, pH 8.5, containing **10** µM ThT and <2% of ThT buffer resulting from the fIAPP seed or the buffer alone solution (in the Aβ42 w/o seed (control) solution). Incubations (37°C) and determination of ThT fluorescence were performed as for all other Aβ42-related studies.

### Assessment of cell damage via the MTT reduction assay

Studied on the effects of peptides on formation of cell damaging IAPP assemblies were performed in cultured RIN5fm cells in combination with the ThT binding assay and TEM. Briefly, cells were cultivated and platted in 96-well plates. Aliquots of solutions used for the ThT binding assays (see "ThT binding assays") were diluted with cell medium at the indicated incubation time points (24 h or 7 days) and added to the cells at the indicated final concentrations. Following incubation with the cells for ~20 h (37°C, humidified atmosphere, 5% CO₂) cell damage was assessed by measuring cellular MTT reduction. IC₅₀ values were determined. Briefly, IAPP (16.5 mM) was incubated with different molar ratios of the ACMs in ThT buffer (see under ThT binding assay) for 24 h and solutions added to the cells (IAPP, 100 nM); cell viability was assessed as above. To determine cell damaging effects of ACM-coated fIAPP, preformed fIAPP (16.5 µM) was co-incubated with the ACM (33 mM) for 1 day as under "ThT-binding assays". Solutions of ACM-coated fIAPP *versus* fIAPP alone were diluted with cell medium and incubated with the cells (fIAPP, 500 nM) as described above.

The studies on effects of ACMs on formation of cell-damaging Aβ42 assemblies were performed in combination with the ThT binding assay and TEM using PC12 cells cultured and plated. Incubations of Aβ42 alone and its mixtures were made in MTPs as described for the ThT binding assays (parallel to the incubations made for the ThT binding assay) but w/o ThT; 6 day-aged solutions (37°C) were diluted with cell medium and added to the PC12 cells at the indicated final concentrations. Following incubation with the cells for ~20 h (37°C, humidified atmosphere, 5% CO₂), cell damage was assessed by measuring cellular MTT reduction. To determine IC₅₀ values of the effects of ACMs, incubations of Aβ42 (5 µM) or its mixtures with various amounts of the ACMs were performed as for the ThT binding assay (37°C) but w/o ThT in MTPs. 6 day-aged incubations were diluted with medium and added to the PC12 cells (Aβ42, 1 mM) and cell damage was assessed following 20 h incubation with the cells as above. On note, anomalous concentration-dependence profiles were found for mixtures of Aβ42 with L3-LF and Nle3-LF most likely due to aggregation; therefore, IC₅₀ values were not determined.

To study effects of ACMs on fIAPP-mediating cross-seeding of formation of cell-damaging Aβ42 assemblies, incubations were made as for the corresponding ThT binding assays but w/o ThT in MTPs. Solutions were aged for 1.5 h (37°C, shaking 500 rpm). Aliquots were diluted with cell medium, incubated with PC12 cells at the indicated final concentrations for ~20 h and cellular MTT reduction was measured as above.

Effects of ACMs on PC12 cell viability were studied using the 4 day-aged solutions applied in the ThT binding assays which were performed to determine their amyloidogenic potential (see under "ThT binding assays). Following incubation with the cells (at 20 mM) for ~20 h, cell damage was assessed by MTT reduction; data were corrected for buffer effects. For comparison, effects of aged Aβ40 were also studied and cytotoxicity was as expected.

### Transmission electron microscopy (TEM)

Aliquots of solutions used for ThT binding, MTT reduction, or other assays were applied on formvar/carbon-coated grids at the indicated incubation time points. Grids were washed with ddH₂O and stained using aqueous 2% (w/v) uranyl acetate. Examination of the grids was done with a JEOL 1400 Plus electron microscope (120 kV). For Aβ42-related studies, solutions made as for the ThT binding assay but w/o containing ThT were used for TEM and the MTT reduction assays. Kinetics of evolution of IAPP homo- and IAPP/Nle3-VF hetero-fibrils from amorphous aggregates was followed by TEM in solutions made in 10 mM sodium phosphate buffer, pH 7.4 (Fig. 4a) and also in solutions made in ThT buffer and very similar results were found.

### Immunogold-TEM

Immunogold-TEM was performed. Briefly, peptide solutions made as described for the corresponding ThT binding assays were applied onto the grids at the indicated incubation time points. Grids were blocked with 0.1% BSA in 1xPBS. fIAPP was detected with a fibril-specific mouse anti-fIAPP antibody (Synaptic Systems; Cl. 91E7). Nle3-VF was revealed by a rabbit anti-Ap40 polyclonal antibody (Sigma-Aldrich) exhibiting 10-20% NSB to IAPP. The two antibodies (in 0.1% BSA in 1xPBS; dilution 1/10) were deposited simultaneously onto the grid and incubated for 20 min. Following washing with 1xPBS, grids were incubated (20 min) with secondary antibodies goat anti-rabbit gold-conjugate (10 nm) and goat anti-mouse gold-conjugate (5 nm) (Sigma-Aldrich) (in 0.1% BSA in 1xPBS, dilution 1/10) as above. Following 1xPBS and ddH₂O washings, uranyl acetate staining and grid examination were performed as described under "TEM". To quantify IAPP and Nle3-VF contents of fibrils, 5 and 10 nm gold particles were counted; "antibody reactivity" is expressed as % of total number of gold particles bound. Significance was analyzed by one-way ANOVA and Bonferroni's Multiple Comparison test.

### Far-UV CD spectroscopy

CD spectra were recorded using a Jasco 715 spectropolarimeter. CD spectra (average of 3 spectra) were measured between 195-250 nm, at 0.1 nm intervals, a response time of 1 sec, and at RT. The spectrum of the buffer was always subtracted from the spectra of the peptide solutions. Peptide incubations related to ACM alone or ACM/IAPP interactions were performed. Briefly, to study peptide conformations and oligomerization propensities, CD spectra of freshly made solutions in 10 mM sodium phosphate buffer, pH 7.4, containing 1% HFIP were measured at 5 mM or at the indicated concentrations in concentration-dependence studies. For studying hf-IAPP/ACM, IAPP (16.5 µM) was incubated with Nle3-VF or VGS-VF (33 µM) in ThT buffer as for the ThT binding assay for 7 days and spectra were measured at the indicated time points. For comparison, spectra of IAPP, Nle3-VF and VGS-VF alone were also measured. For studying the structure of hf-Aβ42/Nle3-VF, incubations were performed as for the ThT binding assays but in the absence of ThT. Briefly, Aβ42 alone (5 µM), Nle3-VF alone (5 µM), and their mixtures (5 µM each) in 45 mM ammonium acetate (pH 8.5) were incubated for 6 days at 37°C and CD spectra were measured.

### Fluorescence spectroscopic titration assays

Fluorescence spectroscopic studies were performed with a Jasco FP-6500 fluorescence spectrophotometer. Briefly, excitation was at 492 nm and spectra were measured between 500 and 600 nm. All titrations were performed in freshly made solutions of synthetic N-terminal fluorescently labeled peptide (5 nM) and various amounts of unlabeled peptide in 10 mM sodium phosphate buffer, pH 7.4 (1% HFIP) within 2-5 min following solution preparation. Under these experimental conditions, freshly made solutions of Fluos-IAPP and FITC-AP42 (5 nM) consist mostly of monomers and the same was found for Fluos-ACMs (5 nM) (Fig. 13e and data not shown). Apparent binding affinities (app. K_{D}s) were estimated by using 1/1 binding models. However, due to the high self-assembly propensities of involved peptides more complex models might also apply. Determined app. K_{D}s are means (±SD) from three binding curves derived from three independent titration assays.

### Cross-linking. NuPAGE. and Western blot (WB)

Hetero-complex cross-linking studies in combination with NuPAGE and WB were performed with a previously developed assay system used for the characterization of Aβ and IAPP homo- and hetero-assemblies. Briefly, for characterizing IAPP homo-/hetero-assemblies, IAPP (30 µM), IAPP/ACM mixtures (1/2) and ACMs alone (60 µM) were incubated in 10 mM sodium phosphate buffer (pH 7.4) for up to 7 days (20°C). In the case of Aβ42 homo-/hetero-assemblies, Aβ42 (30 µM) and Aβ42/ACM mixtures (1/2) were incubated in 10 mM sodium phosphate buffer (pH 7.4) for up to 6 days. At the indicated time points (0 h, 24 h, and 7 days (IAPP studies) or 0 h, 3 h, 24 h, and 6 days (Aβ42 studies)) aliquots were cross-linked (2 min) with 25% aqueous glutaraldehyde (Sigma-Aldrich) and treated with a 2 M NaBH₄ solution (in 0.1 M NaOH, 20 min). Following precipitation with trichloroacetic acid (10%) (4°C) and centrifugation (10 min, 12000 g), pellets were dissolved in reducing NuPAGE sample buffer, boiled (5 min, 95°C) and subjected to NuPAGE gel electrophoresis as described using 4-12% Bis-Tris gels and MES running buffer (Thermo Fisher Scientific). Equal amounts of IAPP or Aβ42 were loaded in all lanes. Peptides were transferred onto nitrocellulose membranes (XCell II Blot Module, Thermo Fisher Scientific). Membranes were blocked overnight (10°C) with 5 % milk in TBS-T (20 mM Tris/HCl, 150 mM NaCl and 0.05 % Tween-20). To reveal homo-/hetero-assemblies, membranes were incubated (2 h) with one of the following primary antibodies (in 5% milk in TBS-T): rabbit polyclonal anti-LAPP (Peninsula; 1:1000) for IAPP-containing assemblies, rabbit polyclonal anti-Aβ40 (Sigma-Aldrich; 1:2000) for ACM-containing assemblies, or mouse monoclonal anti-Aβ(1-17) (6E10, BIOZOL; 1:2000) for Aβ42-containing assemblies (no cross-reactivity with ACMs). Primary antibodies were combined with suitable peroxidase (POD)-coupled secondary antibodies (donkey anti-rabbit-POD (1:5000) or goat anti-mouse-POD (1:1000)) and homo-/hetero-assemblies were revealed with SuperSignal West Dura Extended Duration Substrate (Thermo Fisher Scientific). Membranes were stripped by incubating in stripping buffer (2% SDS, 100 mM b-mercaptoethanol, 50 mM TRIS, pH 6.8) for 20 min at 60°C and at RT for 45 min. Prestained protein size markers ranging from 3.5 to 260 kDa (Invitrogen) were electrophoresed in the same gels.

### Size exclusion chromatography (SEC)

SEC was performed with a Superdex 75 10/300 GL column (GE Healthcare); flow rate was 0.5 ml/min and detection was at 214 nm. For IAPP-related studies, elution buffer was 50 mM sodium phosphate buffer, pH 7.4, containing 100 mM NaCl. IAPP (16.5 µM) or IAPP/ACM (or IAPP/VGS-VF) mixtures (1/2) were incubated in ThT buffer as for the ThT binding assays and at the indicated incubation time points centrifuged (1 min, 20000 g) and loaded onto the column. For Aβ42-related studies, elution buffer was 50 mM ammonium acetate, pH 8.5.Aβ42 (5 µM) or Aβ42/ACM (1/1) mixtures were incubated under ThT binding assay conditions (w/o ThT) and loaded onto the column at indicated time points. The column was calibrated with proteins/peptides of known molecular weights.

### ANS binding fluorescence spectroscopy

ANS binding studies were performed with a Jasco FP-6500 fluorescence spectrophotometer. Briefly, excitation was at 355 nm and fluorescence emission spectra were recorded between 355 and 650 nm. Solutions of ANS alone (8 µM) and its mixtures with IAPP (2 µM) or IAPP/Nle3-VF (1/2) mixtures were freshly made in 10 mM sodium phosphate buffer, pH 7.4, containing 1% HFIP and spectra were recorded at the indicated time points.

### Hetero-complex pull-down assays

Pull-down assays were performed using streptavidin-coupled magnetic beads (Dynabeads M-280 Streptavidin, Dynal). Briefly, solutions of Biotin-IAPP (16.5 µM), Biotin-IAPP/Nle3-VF-mixtures (1/2), and Nle3-VF (33 µM; control for non-specific binding (NSB) to beads) in 10 mM sodium phosphate buffer, pH 7.4 were aged for 7 days (20°C) and subsequently incubated with the beads for 4 h at RT. Bead-bound complexes were isolated by magnetic affinity. Following washing, beads were boiled with reducing NuPAGE sample buffer (5 min, 95°C) and supernatants subjected to NuPAGE electrophoresis and WB as described under "Cross-linking, NuPAGE and WB". Equal amounts were loaded in all lanes; lane "Nle3-VF (control)", freshly dissolved Nle3-VF without incubation with the beads.

### Confocal laser scanning microscopy (CLSM) and 3D stimulated emission depletion (STED) imaging

IAPP or IAPP/ACM mixtures (1/2) containing 10% N-terminal fluorescently-labeled analogs TAMRA-IAPP and Atto647N-ACM (IAPP(total), 16.5 µM; ACM(total), 33 mM) were incubated in 10 mM sodium phosphate buffer, pH 7.4 for 7 days (20°C). Aliquots (30-40 µl) were pipetted onto SuperFrost Plus adhesion slides (Thermo Fisher Scientific), air-dried, covered with a high precision covershlip (#1.5; Ibidi), and embedded using Prolong Diamond Antifade Mountant (Thermo Fisher Scientific). CLSM and STED were performed using a Leica SP8 STED 3X microscope (HC PL APO 93x/1-30 GLYC CORR STED objective) with a tunable white light laser source to excite fluorophores. Depletion power (660 nm (TAMRA), 775 nm (Atto647N)) and time-gated detection of excited light were chosen to minimize sample damage while optimizing xyz-resolutions. Images were collected in a sequential scanning mode (hybrid-diode detectors) to maximize signal collection while minimizing channel cross-talk (TAMRA: excitation 552 nm/emission 557-645 nm; Atto647N: excitation 646 nm/emission 651-700 nm). 3D reconstructions/ fibril measurements were performed using Leica's LAS-X software package (v1.2). Datasets were deconvoluted using Leica's Lightning application.

### Two-photon microscopy (2PM) and FLIM-FRET studies

Solutions analyzed by 2PM or FLIM-FRET consisted of either N-terminal fluorescently-labeled peptides (100%) or mixtures of labeled with non-labeled peptides (when indicated) and were prepared as follows: for most IAPP-related studies, hf-IAPP/ACM were made by incubating TAMRA-IAPP (16.5 µM) with synthetic N-terminal fluorescein- or Atto647N-labeled ACMs (33 µM) in 10 mM sodium phosphate buffer, pH 7.4 (abbreviated "1xb") for 6-7 days (20°C). For comparison, aged TAMRA-IAPP (16.5 µM) was also examined and consisted mostly of fibrillar assemblies (TAMRA-fIAPP; Fig. 18d). In some cases (i.e. samples examined by both STED and 2PM), IAPP/ACM mixtures (6-7 days aged, 1xb (20°C)) consisting of 16.5 mM IAPP(total) and 33 mM ACM(total) with each of them containing 10% of labeled peptide were used as indicated.

For 2PM and FLIM-FRET studies related to ACM-coated fIAPP, first fIAPP were made by incubating an IAPP/TAMRA-IAPP mixture (16.5 µM IAPP(total) containing 10% TAMRA-IAPP) in 1xb for 48 h. fIAPP were then co-incubated with a mixture of Nle3-VF/Atto647-Nle3-VF (33 µM Nle3-VF(total) for 1 day (20°C)) to yield ACM-coated fIAPP. For 2PM and FLIM-FRET studies regarding the role of monomeric/prefibrillar IAPP on formation of IAPP/Nle3-VF nanofiber co-assemblies, aliquots from a freshly made mixture of Fluos-Nle3-VF (33 mM) with TAMRA-IAPP (1.65 mM) in 1xb (20°C) were examined at the indicated incubation time points. For the corresponding studies on the role of fIAPP, preformed TAMRA-fIAPP seeds (3.3 mM); TAMRA-fIAPP was made by incubating TAMRA-IAPP (16.5 mM) in 1xb for 5 days (20°C). For all Aβ42-related studies, solutions consisted of 1/1 mixtures of unlabeled/labeled peptides (as indicated) and were prepared as for the ThT binding assays (w/o ThT) (45 mM ammonium acetate, pH 8.5, 37 °C; aging as indicated) as follows: for the studies on fAβ42 *versus* hf-Aβ42/ACM, Aβ42 solutions (Aβ42(total) 5 µM) consisted of 50% TAMPA-Aβ42 and 50% Aβ42 (6 day-aging); Aβ42/ACM mixtures (1/2) contained, in addition to Aβ42/TAMPA-Aβ42 (1/1; Aβ42(total), 5 µM), ACM/Fluos-ACM (1/1; ACM(total), 10 µM) (4-6 day-aging). For studies on fIAPP-mediated cross-seeding of Aβ42, the Aβ42 alone solution (Aβ42(total), 10 µM) contained HiLyte647-Aβ42 (50%); the Aβ42/ACM (1/2) mixtures consisted of Aβ42/HiLyte647-Aβ42 (1/1) (Aβ42(total), 10 µM) and Nle3-VF/Fluos-Nle3-VF (1/1) (Nle3-VF(total), 20 µM); solutions were aged for 1.5 h. TAMRA-fIAPP seeds were made by incubating TAMRA-IAPP (128 µM) in ThT buffer for 6 days (20°C)); their concentration in the crossseeded solution was 2 µM. Aliquots from the above solutions were applied onto SuperFrost Plus adhesion slides, air-dried, washed (Aβ42-related studies) and embedded with Prolong Diamond Antifade Mountant as for CLSM and STED.

Samples were imaged on a two(multi)-photon Leica TCSPC SP8 DIVE FALCON LIGHTNING microscope (Leica) equipped with extended IR spectrum tunable laser (680-1300 nm) (New InSight^{®} X₃^{™}, Spectra-Physics) and fixed IR laser (1045 nm), advanced Vario Beam Expander (VBE), ultra-high-speed resonance scanner (8kHz), HC PL IRAPO 25x/1.0 WATER objective, and FLIM-FRET modality. Images were collected in sequential scanning mode (hybrid-diode detectors; TAMRA: excitation 1100 nm/emission 560-630 nm; fluorescein (Fluos): excitation 920 nm/emission 480-550 nm; HiLyte647: excitation 1280 nm/emission 635-715 nm) and handled using Leica's LAS-X software package. Deconvolutions were performed using Huygens Professional or Leica's Lightning application.

For fluorescence lifetime imaging (FLIM), up to 1000 photons/pixel were captured (timecorrelated single-photon counting (TCSPC) mode). Samples were prepared as described above. Fluorescence decays were fit using Leica's FALCON software applying multi-exponential models. Quality of fits was assessed by randomly distributed residuals/low Chi-square values. The number of components (n) used for fittings was manually fixed to values (n=2-4) that minimized Chi-square statistic. In control experiments, fluorescence lifetime of the donor fluorescent molecule (Fluos-Nle3-VF) (33 µM, 1xb, aging 6 days) in absence of acceptor was acquired similarly (a multi-exponential model was applied). Amplitude-weighted average lifetime was calculated as τ = Σ(αᵢτᵢ)/Σαᵢ (αᵢ: amplitude of each lifetime τᵢ). FLIM-FRET efficiency was calculated by FRET eff = 1-(τ_{DA}/τ_{D}); (τ_{DA}=lifetime donor in presence of acceptor; τ_{D}=lifetime donor alone).

### Dot blot assays to assess binding ofACMs to IAPP and Aβ42 fibrils and monomers

IAPP (128 µM) and Aβ42 (11 µM) incubations were made as for ThT binding assays (Aβ42 w/o ThT) and deposited onto nitrocellulose membranes (IAPP: 40 µg, Aβ42: 10 µg) either directly following their preparation (for monomers) or after 2 days of aging (for fibrils; confirmed by ThT binding and TEM). After blocking (5 % milk in TBS-T, 2 h, RT) and several washing steps (with TBS-T and ThT buffer), membranes were incubated with N-terminal fluorescein-labeled ACMs (Fluos-ACMs) at 0.2 µM for IAPP-related membranes or 2 µM for Aβ42-related membranes; incubation was overnight (10°C) in ThT buffer (containing 1% HFIP). To control for fibril autofluorescence, similar membranes were incubated in parallel with buffer only. Bound peptides were visualized using a LAS-400mini instrument (Fujifilm) equipped with a suitable fluorescence filter.

### Dot blot analysis for quantification of fibrils

Dot blot analysis was used to verify the presence of equal amounts of homo- and heteromeric fibrillar assemblies in the aliquots of solutions examined by the various different assays, e.g. the ThT binding assay, the MTT reduction assay, or the PK digestion assay. For example, in the case of the solutions used for ThT binding and MTT reduction assays, 7 day-aged IAPP (16.5 µM) or IAPP/ACM (1/2) mixtures were prepared as described under "ThT binding assays"; TEM showed that fibrils were major species in both kinds of solutions (Fig. 2f). Aliquots (1.3 µg IAPP) were spotted onto 0.2 µm-nitrocellulose membranes. For comparison, freshly made solutions (containing no fLAPP or hf-LAPP/ACM according to ThT binding (IAPP) and TEM) were made as above and spotted immediately. IAPP present in o h aged solutions of IAPP alone and solutions containing non-fibrillar IAPP/ACM co-assemblies (TEM data Fig. 4a) was revealed using a rabbit polyclonal anti-LAPP antibody (Peninsula; 1:1000) whereas for fLAPP present in 7 day-aged solutions the fIAPP-specific mouse anti-fLAPP antibody (Synaptic Systems; Cl. 91E7, 1:500) was used. Incubations with antibodies and membrane development were done as under "PK digestion assays".

### Assessment of fibril thermostability by TEM and ThT binding

Solutions consisting mainly of fLAPP, hf-IAPP/Nle₃-VF, fAβ42 or hf-Aβ42/Nle3-VF were prepared as described under "ThT binding assays" (fIAPP 16.5 µM, 7 day-aged; hf-IAPP/Nle3-VF, IAPP 16.5 mM, Nle3-VF 33 µM, 7 day-aged; fAβ42 5 µM, 6 day-aged; hf-Aβ42/Nle3-VF, 5 µM each, 6 day-aged; Aβ42 incubations w/o ThT) and boiled (95°C) for 5 min except for fAβ42 which was boiled for 15 min. TEM grids were loaded, stained, and analyzed as under "TEM". ThT binding of fIAPP and hf-IAPP/Nle₃-VF solutions was assessed by mixing aliquots before or after boiling with a ThT solution as described under "ThT binding assays"; buffer values were subtracted from the data.

### Proteinase K (PK) fibril digestion assay in combination with Dot Blot

The PK digestion assay was performed based on protocols by Ladiwala et al. and Cho et al. Briefly, PK stocks (100 µg/ml) were prepared in 50 mM TRIS/HCl pH 8.0 containing 10 mM CaCl₂; the final PK concentration in the assay was 0.5 µg/ml. fIAPP (16.5 µM) or hf-IAPP/ACM (1/2) were prepared by incubating the peptides in 10 mM sodium phosphate buffer, pH 7.4 for 7 days (20°C); fibril formation was confirmed by ThT binding (fIAPP) and TEM. fAβ42 (5 µM) and hf-Aβ42/ACM (1/1) were made as described under "ThT binding assays" (w/o ThT; 7 day-aging). For IAPP-related assays, solutions were made by mixing 60 µl of the fIAPP or hf-IAPP/ACM solutions with 0.3 µl of the PK stock solution. For Aβ42-related assays, solutions were made by mixing 200 µl of fAβ42 or hf-Aβ42/ACM solutions with 1 µl PK stock. Solutions made as above but w/o PK were used as controls for 100% undigested fibrils. Solutions were incubated at 37°C and at indicated time points aliquots were dotted onto nitrocellulose membranes and spots were quickly dried by air. Membranes were washed (TBS-T) and blocked (5% milk in TBS-T, overnight (10°C)). The following primary antibodies were used for membrane development (2 h, in 5% milk in TBS-T (RT)): mouse anti-fIAPP (Synaptic Systems, Cl. 91E7; 1:500) for fIAPP; mouse anti-Aβ(1-17) (6E10, BIOZOL; 1:2000) for Aβ42); rabbit anti-Aβ40 (Sigma-Aldrich; 1:2000) for ACMs. Primary antibodies were combined with goat anti-mouse-POD (1:1000) or donkey anti-rabbit-POD (1:5000); detection was as under "Cross-linking, NuPAGE and WB".

### Phagocytosis assay

Phagocytosis of fIAPP and fAβ42 *versus* hf-IAPP/ACM and hf-Aβ42/ACMs was studied in primary murine BMDMs and cultured murine BV2 microglia using TAMRA-IAPP and TAMPA-Aβ42 and essentially following an established protocol. Briefly, BV2 cells were maintained in GlutaMAX-supplemented RPMI1640 medium containing 10% FBS and 1% penicillin/streptomycin on poly-L-ornithine-coated flasks. For the phagocytosis assay, BV2 cells were seeded into 24-well plates containing coverslips in serum-free RPMI1640-GlutaMAX and further incubated for 24 h (5% CO₂, 37°C) to reach 10000 cells/well. Primary BMDMs were obtained from bone-marrow monocytes isolated from wildtype C57BL/6 mice, plated in 24-well plates at a density of 10000 cells/well, and differentiated with L929 cellconditioned medium (RPMI1640, 10% FBS, 1% penicillin/streptomycin) for 7 days. Thereafter, cells were incubated with aged peptide solutions at 37°C for 6 h. Peptide solutions were prepared as follows: for IAPP-related studies, TAMRA-fIAPP (16.5 µM) and hf-TAMRA-IAPP/ACM (16.5 mM) were made by incubating peptides/peptide mixtures (1/2) in ThT buffer for 7 days as for the ThT binding assay. Solutions were diluted with cell medium and added to the cells at a final homo-/hetero-nanofiber (IAPP) concentration of 3.3 µM. For Aβ42-related cell uptake studies, TAMRA-fAβ42 (5 µM) and hf-TAMRA-Aβ42/ACM (5 µM) were prepared by incubating the peptides/peptide mixtures (1/1) under ThT assay conditions (w/o ThT) for 6 days. Following centrifugation (20 min, 20000 g), pellets were resuspended in cell medium and incubated with the cells for 6 h at a final homo-/hetero-fibril concentration of 1 µM. Of note, dot blot analysis and BCA showed that the main peptide fraction was present in the pellet. Following incubation of the cells with the peptides, supernatants were removed and cells on the coverslips were washed 5 times with ice-cold 1xPBS, fixed with 4% paraformaldehyde, washed with 1xPBS, permeabilized with 0.2% Triton-X 100, and rinsed three times with cold 1xPBS. Coverslips were mounted with Vectashield Antifade mounting medium containing DAPI (Vector Laboratories). Images were acquired using a Leica DMi8 fluorescence microscope. The percentage of cells that had taken up peptides was calculated by dividing the number of BV2 cells or BMDMs that phagocytosed TAMRA-labeled peptide by the total cell count, multiplied by 100. Significance was analyzed by unpaired student's t-test.

### Hippocampal long-term potentiation (LTP) measurements

LTP measurements were performed. Briefly, sagittal hippocampal slices (350 µm) were obtained from C57BL/6N male mice (6-8 weeks) in ice-cold Ringer solution bubbled with a mixture of 95% O₂ and 5% CO₂ and according to protocols approved by the ethical committee on animal care and use of the government of Bavaria Germany. Extracellular recordings were performed using artificial cerebrospinal fluid (ACSF)-filled glass microelectrodes (2-3 MW) at RT. ACSF consisted of 125 mM NaCl, 2.5 mM KCl, 25 mM NaHO₃, 2 mM CaCl₂, 1 mM MgCl₂, 25 mM D-glucose, and 1.25 mM NaH2PO₄ (pH 7.3) and was bubbled with 95% O₂ and 5% CO₂. Field excitatory postsynaptic potentials (fEPSPs) were evoked in the hippocampal CA1 dendritic region via two independent inputs by stimulating the Schaffer collateral commissural pathway (Seep). For LTP induction, high-frequency stimulation (HFS; 100 Hz/100 pulses) conditioning pulses were delivered to the same Seep inputs. Both stimulating electrodes were used to utilize the input specificity of LTP, thus allowing for the measurement of an internal control within the same slice. Aβ42 (50 nM), AP42/ACM mixtures (1/10) or ACMs alone (500 nM) were freshly dissolved in ACSF and applied 60-90 min before HFS. Responses were measured for 60 min after HFS. fEPSP slope measurements (20-80% of peak amplitude) are presented as % fEPSP slope of baseline (the 20 min control period before tetanic stimulation was set to 100%). Data analysis by 1-way ANOVA and Bonferroni's multiple comparison test.

### X-ray fiber diffraction

fIAPP and hf-IAPP/Nle3-VF were made by aging IAPP (1024 µM) or a mixture of IAPP (1024 µM) and Nle3-VF (2048 µM) in ddH₂O for 3 days. A droplet of each solution was placed between glass rods supported by plasticine balls and allowed to dry (humidified atmosphere, 2-4 days, RT). X-ray diffraction data were collected at the facility Single-Crystal X-Ray Diffractometry of the TUM Catalysis Research Center (CRC) using a Bruker D8 Venture diffractometer equipped with a CPAD detector (Bruker Photon II), an IMS micro source with CuKa radiation (λ = 1.54178 Å) and a Helios optic using the APEX3 software package (Version 2019-1.0, Bruker AXS Inc., Madison, Wisconsin, USA, 2019).

### Example 2: Inhibitor design and concept evaluation

For inhibitor design, Aβ(15-40) was used as a template in the context of the fAβ40 fold; this features a β-strand-loop-β-strand motif with Aβ(12-22) and Aβ(30-40) forming the β-strands and Aβ(23-29) the loop (Fig. 1a,b). A minimum number of chemical modifications was made aiming at (a) distorting the loop, (b) stabilizing β-sheet structure, and (c) suppressing intrinsic amyloidogenicity of Aβ(15-40) while maintaining its pronounced self-/cross-assembly propensity in analogy to the ISM concept (Fig. 1b). The modifications were: (a) substitution of loop tripeptide Aβ(24-26) (Val-Gly-Ser) by β-sheet propagating tripeptides consisting of identical large hydrophobic residues, to strengthen β-sheet interaction surfaces while being incompatible with localization in turns/β-arcs and (b) selective amide bond N-methylation of two alternate residues within one of the two Aβ β-strand segments, to suppress intrinsic amyloidogenicity of ACMs and their co-assemblies (Fig. 1b). Positions of N-methylations were based on fAβ40 models and previous SAR studies. Finally, Met35 was replaced by Nle to avoid Met(O)-related side effects.

To evaluate the concept, 13 Aβ(15-40) analogs containing various different loop tripeptide segments (LTS), comprising (Nle)3, (Leu)3, (Phe)3, (Arg)3, (Gly)3, or Val-Gly-Ser (control LTS) and one pair of two N-methylated residues were designed, synthesized and studied (Fig. 1c, Table 3). In addition, to identify best suited LTS, various non-N-methylated analogs were synthesized and screened in initial studies (Table 3): First, the effect of unmodified Aβ(15-40) (abbreviated VGS) on IAPP fibrillogenesis was studied by using the amyloid-specific thioflavin T binding assay and was found unable to inhibit (Fig. 9a,b). However, non-N-methylated analogs Nle3 and L3 containing LTS (Nle)3 or (Leu)3 instead of Aβ(24-26) led to some delay of fibrillogenesis (Fig. 9a,b). By contrast, analogs R3 and G3 containing LTS (Arg)3 and (Gly)3, respectively, did not inhibit and far-UV CD spectroscopy indicated less b-sheet structure than in Nle3 and L3 (Fig. 9a-c). These findings suggested that Nle3 or L3 might be suitable candidates for further modifications.

Peptide Nle3 was then used as a template to identify best-suited positions for N-methylations. The four Nle3 analogs Nle3-LF, Nle3-VF, Nle3-GI, and Nle3-GG were synthesized, each of them containing two N-methylations placed at specific residues either within the N-terminal region corresponding to Aβ(15-23) (analogs Nle3-VF and Nle3-LF) or within the C-terminal region corresponding to Aβ(27-40) (analogs Nle3-GI & Nle3-GG) (Fig. 10a, Table 3). Peptides Nle3-VF and Nle3-LF (Fig. 1c) carrying N-methylations at Val18/Phe20 and at Leu17/Phe19, respectively, fully suppressed IAPP fibrillogenesis and cytotoxicity as determined by ThT binding in combination with the 3-[4,5dimethylthiazol-2-yl]-2,5-diphenyltetrazolium bromide (MTT) reduction assay in cultured rat insulinoma (RIN5fm) cells (Fig. 1d-g, Fig. 10b,c and Fig. 11a,b). By contrast, Nle3-GI and Nle3-GG, carrying N-methylations at Gly29/Ile31 and at Gly29/Ile33, respectively, did not inhibit (Fig. 10b,c). Titrations of cytotoxic IAPP with Nle₃-VF or Nle3-LF revealed nanomolar IC₅₀ values consistent with highly potent inhibitory activities (Table 1, Fig. 12a,b). Of note, introduction of the N-methylations of Nle3-VF and Nle3-LF into the non-inhibitory peptides VGS, G3 or R3 did not convert them into inhibitors (Fig. 10d,e). Also, partial Nle3-VF segments Nle3-VF(15-23), Nle3-VF(27-40), and Nle3-VF(21-40) did not inhibit (Fig. 10f-h). These results showed that both the loop tripeptide (Nle)3 and one of the two N-methylation patterns within Aβ(17-20) Val18Phe20 and Leu17Phe19 are optimal to convert Aβ(15-40) into a nanomolar inhibitor of IAPP.

To further evaluate the concept, we next synthesized and tested the four peptides L3-VF, L3-LF, F3-VF, and F3-LF containing loop tripeptides (Leu)3 or (Phe)3 and each of the two identified N-methylation patterns (Fig. 1c, Table 3). All of them fully suppressed IAPP fibrillogenesis and related cytotoxicity (Fig. 1d-g, Fig. 11c-f) and nanomolar IC₅₀ values were obtained (Table 1, Fig. 12c-f).

Far-UV CD spectroscopy revealed significant amounts of b-sheet structure in all six inhibitory ACMs whereas non-inhibitors VGS-VF and VGS-LF were less structured (Fig. 1h,i). In addition, ACMs exhibited strong self-assembly propensities; however, they were soluble, non-amyloidogenic and non-cytotoxic up to at least 500-fold higher concentrations than their IC₅₀ values (Fig. 13). Importantly, in the presence of ACMs both self- and cross-seeding of IAPP amyloid formation by preformed fIAPP or fAβ42 were strongly suppressed as found by the ThT binding assay (Fig. ij,k).

Together, the studies identified the six ACMs Nle3-VF, Nle3-LF, L3-VF, L3-LF, F3-VF, and F₃-LF (Fig. 1c, Table 3) as highly potent inhibitors of self-/cross-seeded IAPP amyloid self-assembly.

### Example 3: ACMs co-assemble with IAPP into amyloid-like but non-toxic nanofibers and their diverse highly ordered superstructures

To obtain insight into the inhibition mechanism, the inventors next studied ACM interactions and co-assemblies. First, fluorescence spectroscopic titrations of N-terminal fluorescein-labeled IAPP (Fluos-IAPP) (5 nM) with ACMs revealed high affinity interactions. In fact, most app. K_{D}s were <100 nM and in very good agreement with the determined IC₅₀ values (Fig. 2a, Fig. 14, Table 1). As freshly made solutions of Fluos-IAPP at 5 nM consist mainly of monomers, these results suggested that ACMs bind IAPP monomers/prefibrillar species with high affinity. IAPP/ACM hetero-assemblies were then cross-linked at various incubation time points with glutaraldehyde, separated by NuPAGE, and visualized by Western blot (WB) (Fig. 2b & Fig. 15). IAPP alone contained monomers, clearly resolved low MW oligomers (2-6-mers), and fibrils which did not enter the gel (Fig. 2b). By contrast, a strong smear between ~15 kDa and the upper end of the gel was observed in IAPP/ACM mixtures already at o h indicative of large amounts of medium-to-high MW co-assemblies (Fig. 2b, Fig. 15). In addition, bands corresponding to hetero-dimers and hetero-tri-/-tetramers or IAPP mono-/dimers were also present in the IAPP/ACM mixtures, whereas the pattern of IAPP/non-inhibitor mixtures was as for IAPP alone (Fig. 2b, Fig. 15). Formation of hetero-dimers and large MW aggregates was further confirmed by size exclusion chromatography (SEC) (Figure 2c).

Far UV-CD spectroscopy revealed that IAPP/Nle3-VF co-assemblies exhibited a mixture of disordered and b-sheet structure (Fig. 2d). By contrast, the CD spectra of aged IAPP and IAPP/non-inhibitor (VGS-VF) mixtures were typical for β-sheet-rich aggregates (Fig. 2d). Furthermore, anilino-naphthalene 8-sulfonate (ANS) binding studies indicated that IAPP/Nle3-VF co-assembly fully suppressed surface-exposure of hydrophobic clusters which occurs at early steps of IAPP amyloid self-assembly and is likely related to cytotoxic oligomer formation (Fig. 2e).

To characterize the morphology of the IAPP/ACM co-assemblies, solutions used for ThT binding and MTT reduction assays were examined with transmission electron microscopy (TEM). As expected, fibrillar assemblies were major species in aged IAPP and its mixtures with the non-inhibitor VGS-VF (Fig. 2f). However, surprisingly, the aged mixtures of IAPP with all six ACMs exclusively consisted of fibrillar assemblies as well; these fibrils were indistinguishable from fIAPP fibrils by TEM (6-10 nm widths and 100-200 nm lengths) (Fig. 2f, Table 4). Notably, in contrast to aged IAPP and IAPP/non-inhibitor mixtures, no turbidity, gelation, or precipitation, was observed in the above IAPP/ACM mixtures. X-ray fiber diffraction revealed then that fibrils in IAPP/Nle3-VF mixtures exhibited the cross-β pattern, which is typical for amyloid fibrils (Fig. 2g). Because fIAPP strongly binds ThT and ACMs were non-amyloidogenic up to 100 mM at least (Fig. 1d, Fig. 13a,b), it seemed reasonable to speculate that the fibrils in the IAPP/ACM mixtures could be: (a) fIAPP which escaped detection by the ThT binding assay or (b) fIAPP which was "covered" with non-specifically bound ACMs; competition of ACMs and ThT for the same fIAPP binding sites might have blocked ThT binding to fIAPP. However, these possibilities were excluded by a series of experiments (Fig. 16).

The ThT-invisible and non-cytotoxic fibrils found in the IAPP/ACM incubations (termed "hf-IAPP/ACM") might thus be heteromeric. To obtain more evidence for this hypothesis, the inventors first applied immunogold TEM. In fact, aged IAPP/ACM mixtures contained fibrils which bound both the anti-IAPP and the anti-Aβ (anti-ACM) antibody (Fig. 3a, Fig. 17a). Additional support was obtained by hetero-complex pull-down assays. Here, ThT-invisible fibrils present in aged mixtures of N-terminal biotin-labeled IAPP (Biotin-IAPP) with Nle3-VF were captured by streptavidin-coated magnetic beads and their components revealed by WB (Fig. 3b).

High-resolution advanced laser-scanning microscopy provided further unequivocal evidence for diverse supramolecular IAPP/ACM nanofiber co-assemblies (Fig. 3c-i, Fig. 17b-f, Fig. 18). Confocal laser scanning (CLSM), stimulated emission depletion (STED), and two-photon microscopy (2PM) visualization of aged IAPP/Nle3-VF mixtures containing N-terminal TAMRA-labeled IAPP (TAMRA-IAPP) and N-terminal Atto647-labeled Nle3-VF (Atto647N-Nle3-VF) or N-terminal fluorescein-labeled Nle3-VF (Fluos-Nle3-VF) revealed large amounts of mm-long heteromeric nanofiber bundles (Fig. 3c,d, Fig. 17b-e). Their widths were 232±76 nm (n=33) whereas fIAPP homomeric nanofiber assemblies formed under identical conditions were less broad (124±23 nm (n=19), p<0.001) as estimated by the more accurate STED nanoscopy (Fig. 3c, Fig. 17f). 3D reconstructions of z-stacks of 2PM pictures suggested that heteromeric nanofiber bundles consist of laterally co-assembled, parallel arranged/in part intertwined stacks of IAPP and ACM molecules (Fig. 17d,e). Additional 2PM studies revealed diverse highly ordered fibrous superstructures including huge macromolecular loops (~500 µm long) (Fig. 3g,h, Fig. 18a) and ribbon- or nanotube-like co-assemblies (widths 5-20 mm, lengths >50 mm) (Fig. 3h,i). Interestingly, parts of the ribbon-like co-assemblies were reminiscent of giant DNA double helixes (Fig. 3h,i). Here, IAPP assemblies seemed to "wrap" and "link" two parallel-running heteromeric nanofiber bundles and similar observations were made in the nanotube-like co-assemblies. Twisted heteromeric nanofiber bundles were also observed (Fig. 3h). Notably, IAPP mixtures with other ACMs but not with the non-inhibitor VGS-VF contained similar heteromeric nanofiber superstructures as the IAPP/Nle3-VF mixtures (Fig. 18b-d).

At this stage, detailed studies on the interaction of ACMs with fIAPP were performed. Dot blots showed that ACMs and the non-inhibitor VGS-VF bind fIAPP. However, ACM/fIAPP co-assemblies (termed "ACM-coated" fIAPP) consisted of fIAPP bundles which were randomly covered by amorphous Nle3-VF aggregates and maintained the ThT binding and cytotoxic properties of fIAPP (Fig. 19; see also Fig. 16). These results further supported the notion that hf-LAPP/ACM were distinct from ACM-coated fIAPP.

To learn more about the molecular architecture of the IAPP/ACM nanofibers, we used fluorescence lifetime imaging/Förster resonance energy transfer (FLIM-FRET). Pronounced FLIM-FRET events were observed in TAMRA-IAPP/Fluos-Nle3-VF nanofiber co-assemblies (Fig. 3j). The faster donor (Fluos-Nle3-VF) fluorescence decay, its strongly reduced lifetime (~0.8 ns) in presence of the acceptor (TAMRA-IAPP), and the appreciable FLIM-FRET efficiency (~85%) were consistent with a very close donor-acceptor proximity i.e. <5.5 nm corresponding to the Förster radius of the TAMRA/Fluos pair (Fig. 3i). The FLIM-FRET data supported the notion that IAPP and the ACM might be part of the same fibril.

Together, these results suggested that the potent inhibitory effect of ACMs is mediated by nanomolar affinity co-assembly with IAPP monomers/prefibrillar species into amyloid-like but ThT-invisible and non-cytotoxic nanofibers and their diverse highly ordered superstructures.

### Example 4: ACM/IAPP nanofibers evolve from amorphous co-assemblies and IAPP may act as a template

The inventors next asked at which stage of the co-assembly process the fibrillar co-assemblies form. The cross-linking and SEC studies indicated that large hetero-assemblies were present already at the begin of the co-incubation (Fig. 2b,c). TEM only detected amorphous aggregates between o and 48 h whereas fibrils were the most abundant species at later time points (7 days) (Fig. 4a, Fig. 20a). Notably, early amorphous aggregates in the IAPP/ACM mixtures were non-cytotoxic as also found for the fibrils (Fig.20b). Thus, non-toxic hf-IAPP/ACM likely evolve via structural rearrangements of non-toxic amorphous co-aggregates.

Because ACMs were non-amyloidogenic in isolation but co-assembled with IAPP into amyloid-like nanofibers, the inventors hypothesized that the amyloidogenic character of IAPP could play a role. In fact, TEM showed that no fibrils formed in mixtures of Nle₃-VF with the natively occurring (human) IAPP analog rat IAPP or the earlier designed double N-methylated IAPP analog IAPP-GI, which have high sequence identity to IAPP but are weakly or non-amyloidogenic (Fig. 4b). Moreover, "cross-nucleation" studies using 2PM and FLIM-FRET suggested a templating role for IAPP monomers/prefibrillar species (Fig. 4c,d). Addition of seed amounts (5%) of TAMRA-IAPP monomers to Fluos-Nle3-VF yielded within 48 h nanofiber co-assemblies of similar appearance and identical FLIM-FRET properties to the nanofiber co-assemblies present in the 7 day-aged TAMRA-IAPP/Fluos-Nle3-VF (1/2) mixtures (Fig. 4c, 4d, Fig. 3f,j). Notably, no appreciable FLIM-FRET events were detected when TAMRA-fIAPP seeds were used (Fig. 21).

### Example 5: Additional properties of hf-LAPP/ACM

The inventors next studied whether the non-toxic hf-IAPP/ACM may differ from fIAPP regarding other properties as well. First, the inventors asked whether hf-IAPP/ACM can seed IAPP fibrillogenesis. However in contrast to fIAPP or fibrils present in IAPP/non-inhibitor mixtures, seed amounts of hf-IAPP/ACM were unable to accelerate IAPP fibrillogenesis as assessed by ThT binding (Fig. 4e).

Pathogenic amyloid fibrils are usually characterized by an extraordinary high stability. Therefore, the inventors compared the thermostabilities of IAPP/ACM nanofibers and fIAPP by using ThT binding and TEM. In contrast to fIAPP, hf-IAPP/Nle3-VF were fully converted into amorphous aggregates after heating to 95°C for 5 min (Fig. 4f). Furthermore, most pathogenic amyloids are resistant toward proteolytic degradation including PK degradation. fLAPP and hf-IAPP/ACM were therefore incubated with PK and degradation kinetics followed by dot blot analysis using anti-fIAPP and anti-Aβ (anti-ACM) antibodies (Fig. 4g). Remarkably and in contrast to fIAPP, which was stable to PK digestion for at least 30 h, hf-IAPP/ACM were fully degraded in <6 h with their ACM component being degraded within few minutes. Finally, the inventors studied the cellular uptake efficiency of hf-IAPP/ACM in direct comparison to fIAPP. In fact, the uptake of amyloid assemblies by macrophages and microglia is a major mechanism of amyloid clearance in both AD or T2D. Uptake of fIAPP versus hf-IAPP/ACM was studied in primary murine bone marrow-derived macrophages (BMDMs) and the wellestablished murine microglial cell line BV2 by fluorescence microscopy using TAMRA-IAPP as tracer. The inventors found that 3- to 10-fold higher amounts of hf-TAMRA-IAPP/ACM were phagocytosed by both cell types as compared to TAMRA-fIAPP (Fig. 4h, Fig. 22).

Together, the results revealed several potentially beneficial properties of non-toxic hf-IAPP/ACM, i.e. seeding incompetence, thermolability, high PK sensitivity, and an efficient cellular clearance profile, which clearly distinguished them from pathogenic fIAPP.

### Example 6: Proposed hypothetical models of IAPP/ACM nanofiber co-assembly

The structure of Aβ/IAPP hetero-amyloids has not been yet elucidated. Based on suggested structures of fIAPP and fAβ40(42) and the polymorphic nature of self-/cross-amyloid assembly, various different interfaces could be involved in hf-IAPP/ACM formation (Fig. 5). The inventors data suggested that single IAPP/ACM nanofibers and basic units of their superstructures may form by lateral co-assembly of two or more "protofilament"-like stacks of IAPP and ACM molecules (Fig. 5). Thereby, b-sheet-prone segments of the ACM part corresponding to Aβ(21-40) would become incorporated into the b-sheet H-bond network of the ACM "protofilament" yielding diverse cross-interaction surfaces with both IAPP and ACM stacks (Fig. 5a,b). The proposed arrangement of Aβ(21-40) is also supported by the finding that Aβ(15-40) analogs with N-methylations within Aβ(21-40) did not inhibit (Fig. 10a-c). IAPP "protofilament" cross-interactions with the ACM could be mediated via previously suggested IAPP segments and fIAPP folds or yet unknown variants thereof. The lack of a 2^{nd} IAPP "protofilament" and a non-ideal IAPP/Aβ(ACM) side chain interdigitation in the hetero-nanofiber as compared to fIAPP and/or intrinsic instability of involved IAPP and/or ACM folds could account for hetero-nanofiber lability. Notably, axial IAPP/ACM "protofilament" co-assembly could also occur (Fig. 5c). However, the STED and 2PM data, the expected higher instability of such a "protofilament" due to the N-methylations, and the observed lack of inhibitory effects of partial ACM segments (Fig. 10f-h) make this scenario less likely.

### Example 7: ACMs inhibit Aβ42 amyloid self-assembly via co-assembly into ThT-invisible and non-toxic nanofibers and their diverse superstructures

In analogy to other Aβ-derived Aβ inhibitors, ACMs also interfere with Aβ amyloid self-assembly. In fact, ThT binding and MTT reduction assays in PC12 cells showed that all six ACMs (Aβ42/ACM 1/1) effectively suppressed formation of Aβ42 fibrils and cytotoxic assemblies (Fig. 6a,b). Titrations of cytotoxic Aβ42 with ACMs yielded mostly nanomolar IC₅₀ values consistent with potent inhibitory activity (Table 2, Fig. 23). Furthermore, ACMs strongly suppressed seeding of Aβ42 fibrillogenesis by preformed fAβ42 (Fig. 6c). Remarkably, TEM examination of the aged "ThT-negative" and non-cytotoxic Aβ42/ACM mixtures revealed that they exclusively consisted of fibrils (Fig. 6d). These fibrils were 2-4 times longer than fAβ42 while their widths were identical to the widths of fAβ42 (7-8 nm) (Fig. 6d, Table 5). Additional TEM and ThT studies suggested that formation of long fibrils was linked to inhibitory activity (Fig. 24a,b). Because the fibrils in the Aβ42/ACM mixtures were significantly longer than fAβ42, non-toxic, and could not solely consist of fAβ42, which strongly binds ThT, or the non-amyloidogenic ACMs, we assumed that they might be heteromeric (termed hf-Aβ42/ACM).

2PM examination of aged Aβ42/ACM mixtures containing N-terminal TAMRA-labeled Aβ42 (TAMRA-Aβ42) and Fluos-ACMs revealed diverse heteromeric fibrous superstructures. These comprised several µm-long heteromeric nanofiber bundles with widths between 0.5-2 mm and related heterogeneous superstructures, i.e. ribbons, tapes, or nanotube-like ones with widths between 3-14 µm (Fig. 6e and Fig. 25). The 2PM images and 3D reconstructions of z-stacks indicated that both axial and lateral co-assembly might underlie their formation, likely enabled by the high degree of sequence identity between ACMs and Aβ(15-40). In hf-Aβ42/Nle3-VF, we observed thick "nodes" periodically arranged along a long "cable"-like part (Fig. 6e). Pronounced FLIM-FRET events were detected which were consistent with close distances between the two peptides, i.e. <5.5 nm (Fig. 6f). Notably, a stronger reduction of Fluos-Nle3-VF lifetime in the presence of TAMEA-Aβ42, i.e. from ~2.1 to ~0.8 ns, and a higher FLIM-FRET efficiency (>80%) were observed in the node regions of interest (ROI-1) than in the cable-like ones (ROI-2) (Fig. 6f). A closer donor-acceptor distance or more donor molecules in nodes might account for this finding.

ACM/Aβ42 interactions and hetero-complexes were then studied by fluorescence spectroscopy, SEC, cross-linking in combination with NuPAGE and WB, and far-UV CD spectroscopy (Fig. 26). Fluorescence spectroscopic titrations of N-terminal FITC-labeled Aβ42 (FITC-Aβ42, 5 nM) with ACMs yielded nanomolar app. K_{D}s (Table 2, Fig. 26a). SEC and cross-linking studies revealed large MW hetero-assemblies in Aβ42/Nle3-VF mixtures consistent with the TEM and 2PM findings (Fig. 26b,c). Cross-linking also identified Aβ42/Nle3-VF hetero-dimers; their formation might underlie hetero-nanofiber co-assembly. Far-UV CD spectroscopy indicated less b-sheet structure in hf-Aβ42/Nle3-VF as compared to fAβ42 (Fig. 26d).

Together, the results suggested that the potent inhibitory effect of ACMs on Aβ42 amyloid self-assembly is mediated by nanomolar affinity interactions of ACMs with Aβ42 monomers/prefibrillar species which redirect them into long ThT-invisible and non-toxic hetero-nanofibers and their diverse mm-scaled superstructures. Further studies suggested that binding of ACMs to preformed fAβ42 does not result in this kind of co-assemblies (Fig. 27).

### Example 8: Additional properties of Aβ42/ACM co-assembly

Hippocampal synaptic plasticity is regarded as a key mediator of learning and memory processes; its damage by toxic Aβ42 aggregates is a major responsible factor in AD pathogenesis. The inventors *ex vivo* electrophysiological studies in mouse brains revealed that in the presence of various different ACMs Aβ42-mediated inhibition of hippocampal long-term potentiation (LTP) was fully ameliorated (Fig. 7a). As inhibition of LTP by Aβ42 is linked to loss of memory and cognitive functions in AD, this data supported the potential physiological relevance of the *in vitro* determined inhibitory effects.

We then investigated whether hf-Aβ42/ACM might differ from fAβ42 with respect to their seeding competence. In fact, the ThT binding assay showed that, in contrast to fAβ42, hf-Aβ42/Nle3-VF and hf-Aβ42/L3-VF were seeding-incompetent (Fig. 7b). Furthermore, we asked whether hf-Aβ42/ACM might exhibit similar proteolytic degradation, thermolability, and cellular clearance features as hf-IAPP/ACM. Kinetics of PK-mediated degradation of fAβ42 *versus* hf-Aβ42/Nle3-VF were studied by dot blot analysis and the 6E10 antibody which specifically recognizes Aβ42 (Aβ(1-17)) but not the ACMs. hf-Aβ42/Nle3-VF were degraded within ~30 min whereas degradation of fAβ42 took ~2 h, i.e. was ~4 times slower (Fig. 7c). Thermostability studies using TEM then showed that hf-Aβ42/Nle3-VF were fully converted into amorphous aggregates after 5 min at 95°C; by contrast, fAβ42 were stable at 95°C for at least 15 min (Fig. 7d). Finally, phagocytosis of TAMRA-fAβ42 and hf-TAMRA-Aβ42/Nle3-VF(L3-VF) by cultured BV2 microglia cells was quantified by applying fluorescence microscopy. Significantly higher amounts of hf-TAMRA-Aβ42/ACM became phagocytosed as compared to TAMRA-fAβ42 (Fig. 7e).

Together, these findings revealed that ThT-invisible and non-toxic hf-Aβ42/ACM were seeding-incompetent and less thermostable than fAβ42 and that they became more efficiently degraded by PK and phagocytosed by BV2 microglia than fAβ42.

### Example 9: Inhibition of fIAPP-mediated cross-seeding of Aβ42 amyloid self-assembly by ACMs

Cross-seeding of Aβ42 amyloid self-assembly by fIAPP accelerates Aβ42 amyloid self-assembly and could link onset and pathogenesis of T2D with AD. In a simplified mechanistic scenario, fIAPP seeds will template formation of IAPP/Aβ42 hetero-amyloids which will template further cytotoxic Aβ42 self-/cross-assembly events. Thereby, polymorphic cross-interactions between amyloid core regions may play an important role.

Because ACMs contain the Aβ amyloid core, bind with high affinity both IAPP and Aβ42, incl. fIAPP and fAβ42, and inhibit their amyloid self-assembly, the inventors assumed that they might also interfere with cross-seeding of Aβ42 by fIAPP. In fact, ACMs effectively suppressed fIAPP-mediated cross-seeding of Aβ42 fibrillogenesis and cytotoxicity (Fig. 7f). Involved supramolecular (co-)assemblies were then studied by 2PM (Fig. 7g-j). First, preformed TAMRA-fIAPP seeds were added to Aβ42 containing N-terminal HiLyte647-labeled Aβ42 (HiLyte647-Aβ42). 2PM at the fibrillogenesis plateau revealed large Aβ42 clusters, consisting of apparently amorphous aggregates or fibrils, bound to/branching out from fIAPP surfaces (Fig. 7g,h). This data was consistent with secondary (cross-)nucleation. However, a completely different picture was obtained when TAMRA-fIAPP seeds were added to a mixture of Aβ42 with Nle3-VF containing HiLyte647-Aβ42 and Fluos-Nle3-VF (Fig. 7i,j). Major species were: (a) large fibrous Aβ42/Nle3-VF/IAPP co-assemblies (many µm long; widths ~1-5 µm) and (b) diverse roundish/elliptical Aβ42/Nle3-VF/IAPP or Aβ42/Nle3-VF co-assemblies (up to ~10 µm) (Fig. 7h-j). Analysis of 2PM images and 3D-reconstructions suggested that fibrous co-assemblies consisted of Aβ42, Nle3-VF, and Aβ42/Nle3-VF bound to fIAPP bundles (Fig. 7i,j). Thus, suppression of Aβ42 cross-seeding likely occurs *via* a dual mechanism (Fig. 8): (1) sequestration of Aβ42 into non-toxic ACM/Aβ42 co-assemblies (both fibrillar and amorphous ones) and (2) binding of non-toxic ACM and ACM/Aβ42 co-assemblies to fIAPP yielding cross-seeding-incompetent and non-toxic ternary nanofiber co-assemblies.

### Example 10: Tables

**Table 1 IC₅₀ values of inhibitory effects of ACMs on IAPP-mediated cell damage^{[a]} and app. K_{D}s of ACM/IAPP interactions^{[b][c]}.**

| **ACM** | **IC₅₀ (±SD) (nM)^{[a]}** | **app. KD (±SD) (nM)^{[b][c]}** |
|---|---|---|
| **Nle3-VF** | 65.0 (±5.2) | 69.5 (±1.4) |
| **Nle3-LF** | 82.1 (±10.2) | 55.4 (±5.9) |
| **L3-VF** | 112.5 (±8.1) | 77.3 (±2.9) |
| **L3-LF** | 133.2 (±29.0) | 143.2 (±5.0) |
| **F3-VF** | 78.5 (±13.6) | 15.0 (±1.9) |
| **F3-LF** | 41.7 (±4.1) | 37.6 (±2.9) |

| | | |
|---|---|---|
| [a] IC₅₀ values, means (±SD) from 3 titration assays (n=3 each) [b] Determined by titrations of N-terminal fluorescein-labeled IAPP (5 nM; pH 7.4) with ACMs [c] App. K_{D}s are means (±SD) from 3 binding curves | | |

**Table 2 IC₅₀ values of inhibitory effects of ACMs on Aβ42-mediated cell damage^{[a]} and app. K_{D}s of ACM/Aβ42 interactions^{[b][c]}.**

| **ACM** | **IC₅₀ (±SD) (nM)^{[a]}** | **app. K_{D} (±SD) (nM)^{[b][c]}** |
|---|---|---|
| **Nle3-VF** | 367 (±79) | 14.5 (±8.0) |
| **Nle3-LF** | n.d. | 11.1 (±6.0) |
| **L3-VF** | 261 (±140) | 38.0 (±2.0) |
| **L3-LF** | n.d. | 2.6 (±1.4) |
| **F3-VF** | 1032 (±297) | 160.8 (±12.9) |
| **F3-LF** | 262 (±115) | 430.6 (±7.1) |

| | | |
|---|---|---|
| [a] IC₅₀ values, means (±SD) from 3 titration assays (n=3 each); n.d.: not determined [b] Determined by titrations of N-terminal FITC-labelled Aβ42 (5 nM; pH 7.4) with ACMs [c] App. K_{D}s, means (±SD) from three binding curves | | |

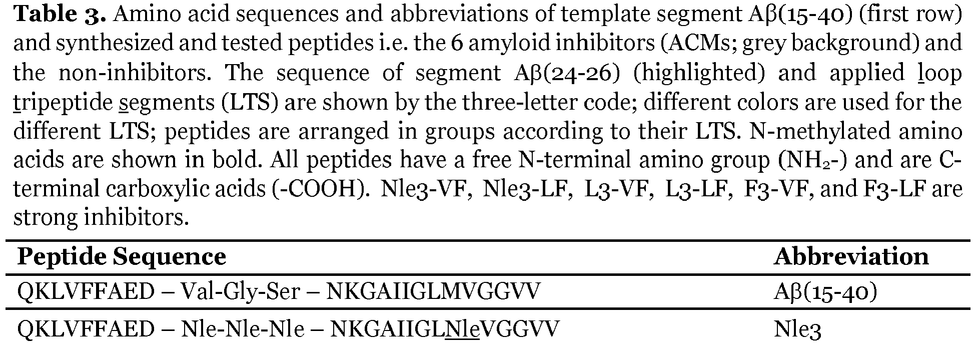

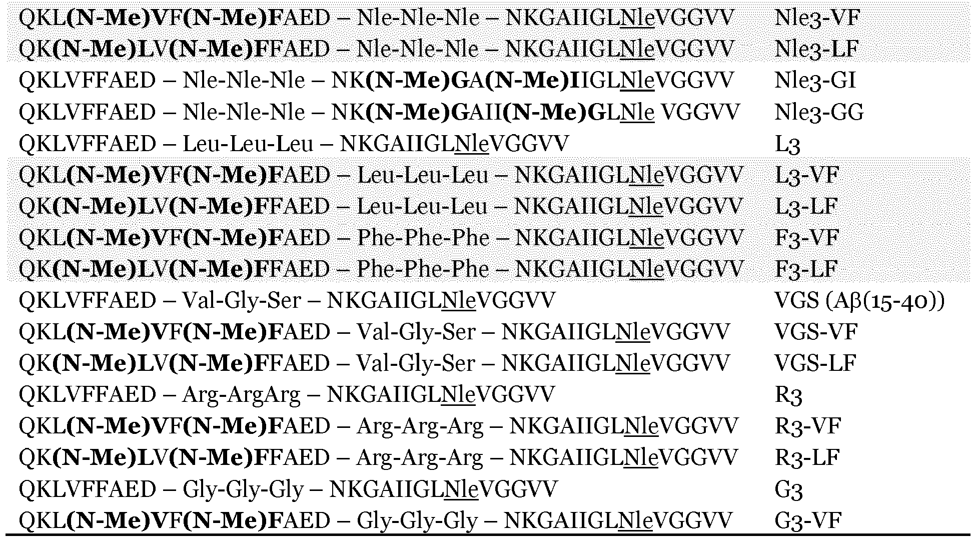

**Table 4. Widths of fIAPP and fibrils found in aged IAPP, IAPP/ACM and IAPP/VGS-VF mixtures as determined by TEM.**

| **Peptide or peptide mixture** | **Fibril width (nm)^{[a]}** |
|---|---|
| **IAPP** | 9.7 (±2.3) |
| **IAPP + Nle₃-VF** | 8.3 (±1.9) |
| **IAPP + L3-VF** | 7.7 (±1.5) |
| **IAPP + F3-VF** | 8.7 (±1.8) |
| **IAPP + Nle3-LF** | 6.1 (±1.1) |
| **IAPP + L3-LF** | 7.9 (±1.8) |
| **IAPP + F3-LF** | 6.6 (±1.2) |
| **IAPP + VGS-VF** | 8.5 (±2.0) |

| | |
|---|---|
| ^{[a]} Measured in 7 days aged IAPP and IAPP/peptide mixtures (from Fig. 1d,f; TEM images Fig. 2f). Data are means (±SD) from 21-47 fibrils | |

**Table 5. Lengths and widths of fAβ42 and fibrils found in Aβ42/ACM mixtures as determined by TEM.**

| **Peptide or peptide mixture** | **Fibril length (nm)^{[a]}** | **Fibril width (nm)^{[a]}** |
|---|---|---|
| **Aβ42** | 154 (±58) | 7.8 (±1.6) |
| **Aβ42 + Nle₃-VF** | 472 (±138) | 7.3 (±1.7) |
| **Aβ42 + Nle3-LF** | 590 (±268) | 7.5 (±1.1) |
| **Aβ42 + L3-VF** | 458 (±106) | 6.9 (±1.4) |
| **Aβ42 + L3-LF** | 347 (±72) | 7.0 (±2.2) |
| **Aβ42 + F3-VF** | 354 (±117) | 6.9 (±1.3) |
| **Aβ42 + F3-LF** | 358 (±118) | 7.3 (±1.5) |

| | | |
|---|---|---|
| ^{[a]} Measured in 6 days aged solutions of Aβ42 and its mixtures (1/1) with ACMs (from Fig. 6b; TEM images Fig. 6d). Data are means (±SD) from 15-23 (lengths) or 21-23 (widths) fibrils (see also bar diagram in Fig 6d) | | |

**Table 6. Molecular weights (M) of synthesized peptides which were used in this study as determined by MALDI-TOF-MS or ESI-MS (^{∗}).**

| **Peptide** | **[M+H]⁺ ([M+Na]⁺) calculated (g/mol)** | **[M+H]^{+[a]} or [M+Na]^{+[b]} found (g/mol)** |
|---|---|---|
| Nle3 | 2726.61 (2748.59) | 2748.71^{[b]} |
| Nle3-VF | 2754.67 (2776.65) | 2776.89^{[b]} |
| Nle3-LF | 2754.67 (2776.65) | 2776.83^{[b]} |
| Nle3-GI | 2754.67 (2776.65) | 2776.50^{[b]} |
| Nle3-GG | 2754.67 (2776.65) | 2777.13^{[b]} |
| L3 | 2726.61 (2748.59) | 2727.00^{[a]∗} |
| L3-VF | 2754.67 (2776.65) | 2776.99^{[b]} |
| L3-LF | 2754.67 (2776.65) | 2757.00 ^{[a]∗} |
| F3-VF | 2856.63 (2878.61) | 2878.92^{[b]} |
| F3-LF | 2856.63 (2878.61) | 2878.73^{[b]} |
| VGS | 2630.48 (2652.46) | 2652.35^{[b]} |
| VGS-VF | 2658.54 (2680.52) | 2681.37^{[b]} |
| VGS-LF | 2658.54 (2680.52) | 2680.47^{[b]} |
| R3 | 2855.67 (2877.65) | 2855.97^{[a]} |
| R3-VF | 2883.73 (2905.71) | 2884.80^{[a]∗} |
| R3-LF | 2883.73 (2905.71) | 2883.99^{[a]} |
| G3 | 2558.43 (2580.41) | 2580.42^{[b]} |
| G3-VF | 2586.49 (2608.47) | 2609.00^{[b]} |
| Nle3-VF(15-23)^{[c]} | 1124.62 (1146.60) | 1146.62^{[b]} |
| Nle3-VF(27-40)^{[c]} | 1309.81 (1331.79) | 1331.93^{[b]} |
| Nle3-VF(21-40)^{[c]} | 1964.17 (1986.15) | 1986.21^{[b]} |
| Fluos-Nle3-VF | 3112.97 (3134.95) | 3134.93^{[b]} |
| Fluos-Nle3-LF | 3112.97 (3134.95) | 3135.16^{[b]} |
| Fluos-L3-VF | 3112.97 (3134.95) | 3134.94^{[b]} |
| Fluos-L3-LF | 3112.97 (3134.95) | 3134.75^{[b]} |
| Fluos-F3-VF | 3214.93 (3236.91) | 3237.05^{[b]} |
| Fluos-F3-LF | 3214.93 (3236.91) | 3236.59^{[b]} |
| Fluos-VGS-VF | 3016.84 (3038.82) | 3038.84^{[b]} |
| Aβ42 | 4512.28 (4534.26) | 4512.83^{[a]∗} |
| rat IAPP | 3918.96 (3940.94) | 3923.54^{[a]} |
| IAPP-GI | 3929.92 (3951.90) | 3930.47^{[a]} |
| IAPP | 3901.86 (3923.84) | 3902.41^{[a]} |
| Biotin-IAPP | 4240.19 (4262.17) | 4241.13^{[a]} |
| TAMRA-IAPP | 4314.29 (4336.27) | 4313.95^{[a]} |
| Fluos-IAPP | 4260.16 (4282.14) | 4260.48^{[a]} |
| Atto647N-Nle3-VF | 3382.57 (3404.55) | 3381.85^{[a]} |

| | | |
|---|---|---|
| M, monoisotopic mass; ^{[a]} [M+H]⁺; ^{[b]} [M+Na]⁺;^{[c]} numbering of residues/segments of partial Aβ(15-40) or ACM analogs according to their sequence numbers in Aβ40. | | |

### Example 11: Discussion

The inventors exploited Aβ/IAPP cross-interactions to design Aβ amyloid core mimics (ACMs) as inhibitors of amyloid self-assembly of both IAPP and Aβ42. Collectively, the inventors identified six 26-residue peptides as effective amyloid inhibitors of both IAPP and Aβ42. All six ACMs bound IAPP with nanomolar affinity and blocked its cytotoxic amyloid self-assembly with nanomolar IC₅₀ values. In addition, all six ACMs bound Aβ42 with nanomolar affinity and blocked its cytotoxic self-assembly, three of them with nanomolar IC₅₀ values. Moreover, *ex vivo* electrophysiology in murine brains showed a full amelioration of Aβ42-mediated damage of synaptic plasticity by ACMs. Importantly, ACMs also inhibited reciprocal cross-seeding of IAPP and Aβ42 amyloid self-assembly. ACMs thus belong to the most effective inhibitors of *in vitro* amyloid self-assembly of IAPP, Aβ42, or both polypeptides.

The inventors most remarkable finding was that ACMs, which were non-amyloidogenic in isolation, exerted their potent amyloid inhibitor function *via* a novel mechanism, i.e. by co-assembling with IAPP or Aβ42 into amyloid-like but ThT-invisible and non-toxic nanofibers and their diverse highly ordered fibrous superstructures. The latter ones comprised large heteromeric nanofiber bundles and several mm-sized loops, ribbons, and nanotube-like superstructures. Furthermore, non-toxic ternary fibrous co-assemblies consisting of fIAPP, Aβ42, and ACM formed when Aβ42 cross-seeding by fIAPP was performed in the presence of ACMs.

Unexpectedly, IAPP(Aβ42)/ACM nanofiber co-assembly was efficiently inhibited by the peptides of the invention. In fact, ACMs contained all three Aβ hot segments for high affinity interactions with IAPP, Aβ42, and themselves, and combined inbuilt β-sheet extension blocking (N-methylations) with β-sheet stabilization/extension enabling (LTS and Aβ(21-40)) elements.

The identified IAPP/ACM nanofibers were indistinguishable from fIAPP by TEM and had the cross-β amyloid core signature by XRD, but were less ordered than fIAPP according to CD spectroscopy. Our TEM, STED, 2PM, and FLIM-FRET studies suggested that nanofibers and basic parts of their fibrous superstructures consisted of laterally co-assembled, parallel arranged or intertwined/twisted, "protofilament-like" IAPP and ACM stacks. In addition, the inventors' studies showed that hf-IAPP/ACM evolve from large amorphous co-assemblies and suggested that IAPP monomers/prefibrillar species might template this process likely *via* hetero-dimers. Although the mechanistic steps are yet unclear, IAPP/ACM nanofiber co-assembly could proceed in analogy to proposed mechanisms of self- and co-assembly of IAPP and Aβ.

The identified Aβ42/ACM nanofibers had similar widths but 2-4 times greater lengths than fAβ42. The inventors' imaging results were consistent with both axial and lateral co-assembly, the former likely underlying hetero-nanofiber elongation.

Another notable finding of the inventors' study relates to the potentially beneficial properties of IAPP/ACM and Aβ42/ACM nanofibers. These clearly distinguished them from pathogenic fIAPP and fAβ42 and, in addition to their non-toxic nature and seeding incompetence, comprised thermolability, proteolytic degradability, and a more efficient phagocytosis than fIAPP and fAβ42. Such features are reminiscent of labile/reversible functional amyloids, in which they may serve to control their formation/storage/ disassembly related to their diverse biological functions. Examples are amyloids from certain secreted peptide hormones or from proteins forming reversible subcellular condensates. By contrast, most "pathological amyloids" are linked to cell damage and characterized by high stability and resistance to proteolysis. Furthermore, increasing evidence suggests that amyloid fold polymorphism underlies amyloid pathogenicity and functional diversity. The inventors' results suggest that, in addition to pathogenic IAPP/Aβ hetero-amyloids generated by fibril-mediated cross-seeding, potentially beneficial IAPP/Aβ hetero-amyloids, such as those mimicked by IAPP/ACM nanofibers, might also exist and this could be also the case for other cross-interacting amyloids. The structural characterization of IAPP/ACM nanofibers should help in identifying molecular factors that may diverge cytotoxic amyloid self-assembly into non-toxic and labile hetero-amyloids and enable the exploitation of amyloid fold versatility to designing effective anti-amyloid molecules.

In conclusion, the present invention offers a novel class of designed peptides as highly potent inhibitors of amyloid self-assembly and reciprocal cross-seeding of IAPP and Aβ42, and as highly promising leads for effective anti-amyloid drugs in both T2D and AD. In addition, the identified nanofiber co-assemblies should guide the design of novel functional (hetero-)amyloid-based supramolecular nanomaterials for biomedical and biotechnological applications.

### REFERENCES

[1] Yan, L. M. et al. Selectively N-Methylated Soluble IAPP Mimics as Potent IAPP Receptor Agonists and Nanomolar Inhibitors of Cytotoxic Self-Assembly of Both IAPP and Abeta40. Angew Chem Int Ed Engl 52 10378-10383, doi:10.1002/anie.201302840 (2013).

## Claims

1. A peptide having an amino acid sequence according to formula 1
(X₁)ₘ(X₂)ₙ(X₃)ₚ(X₄)_{q}*FₐF_{b}AE*X₅-X₆X₇X₈-*NKGAII*X₉X₁₀X₁₁*VG(G)ᵣ(V)ₛ(V)ₜ* (formula 1)
wherein,
m, n, p, q, r, s, and t are, independently at each occurrence, selected from 0 and 1;
X₁ is selected from glutamine, asparagine, alanine, glutamic acid, and aspartic acid, preferably is glutamine;
X₂ is selected from lysine, modified lysine e.g. Lys(Ac), Lys(N₃), and Lys(biotin), alanine, modified alanine, ornithine, modified ornithine e.g. Orn(Ac), diaminobutyric acid, diaminopropionic acid, arginine, and modified arginine e.g. Arg(Me)2, preferably is lysine;
X₃ is selected from leucine, N-methyl-leucine, alanine, N-methyl-alanine, isoleucine, N-methyl-isoleucine, valine, N-methyl-valine, glycine, N-methyl-glycine, norleucine, N-methyl-norleucine, phenylalanine, N-methyl-phenylalanine, tyrosine, and N-methyl-tyrosine, preferably is leucine or N-methyl-leucine;
X₄ is selected from leucine, N-methyl-leucine, alanine, N-methyl-alanine, isoleucine, N-methyl-isoleucine, valine, N-methyl-valine, glycine, N-methyl-glycine, norleucine, N-methyl-norleucine, phenylalanine, N-methyl-phenylalanine, tyrosine, and N-methyl-tyrosine, preferably is valine or N-methyl-valine;
*Fₐ* and *F_{b}* are, independently at each occurrence, selected from phenylalanine, N-methyl-phenylalanine, halogenated phenylalanine, tyrosine, and N-methyl-tyrosine, preferably selected from phenylalanine and N-methyl-phenylalanine;
*A* is, independently at each occurrence, selected from alanine, N-methyl-alanine, leucine, N-methyl-leucine, valine, N-methyl-valine, glycine, and N-methyl-glycine, preferably is alanine;
*E* is selected from glutamic acid, N-methyl-glutamic acid, aspartic acid, N-methyl-aspartic acid, glutamine, N-methyl-glutamine, asparagine, N-methyl-asparagine, alanine, N-methyl-alanine, glycine, and N-methyl-glycine, preferably is glutamic acid;
X₅ is selected from aspartic acid, glutamic acid, asparagine, aspartic acid, alanine, and glycine, preferably is aspartic acid;
X₆, X₇, and X₈ are, independently at each occurrence, selected from norleucine, leucine, phenylalanine, methionine, tryptophan, tyrosine, threonine, alanine, isoleucine, valine, and modified forms thereof e.g. N-methylated forms thereof, preferably selected from norleucine, leucine, and phenylalanine;
*N* is selected from asparagine, glutamine and alanine, preferably is asparagine;
*K* is selected from lysine, modified lysine e.g. Lys(Ac), Lys(N₃), and Lys(biotin), alanine, norleucine, ornithine, modified ornithine e.g. Orn(Ac), diaminobutyric acid, diaminopropionic acid, arginine, and modified arginine e.g. Arg(Me)2, preferably is lysine;
*G* is, independently at each occurrence, selected from glycine and alanine, preferably is glycine;
*I* is, independently at each occurrence, selected from isoleucine, leucine, norleucine, and valine, preferably is isoleucine;
X₉ is selected from glycine, alanine, and serine, preferably is glycine;
X₁₀ is selected from leucine, alanine, isoleucine, threonine, norleucine, and valine, preferably is leucine;
X₁₁ is selected from norleucine, leucine, phenylalanine, methionine, tryptophan, tyrosine, threonine, alanine, isoleucine, and valine, preferably is norleucine; and
V is, independently at each occurrence, valine;
wherein, preferably, at least one of X₃, X₄, Fₐ, and F_{b} is an N-methylated amino acid;
and pharmaceutically acceptable salts, esters, solvates, polymorphs and modified forms thereof.

2. The peptide according to claim 1, having an amino acid sequence according to formula 2
(X₁)ₘ(X₂)ₙ(*Lₐ*)ₚ(*Vₐ*)_{q}*FₐF_{b}AE*X₅-X₆X₇X₈*-NKGAII*X₉X₁₀X₁₁*VG(G)ᵣ(V)ₛ(V)ₜ* (formula 2)
wherein,
m, n, p, q, r, s, and t are, independently at each occurrence, selected from 0 and 1;
X₁ is selected from glutamine, asparagine, alanine, glutamic acid, and aspartic acid, preferably is glutamine;
X₂ is selected from lysine, modified lysine e.g. Lys(Ac), Lys(N₃), and Lys(biotin), alanine, modified alanine, ornithine, modified ornithine e.g. Orn(Ac), diaminobutyric acid, diaminopropionic acid, arginine, and modified arginine e.g. Arg(Me)2, preferably is lysine;
*Lₐ* is leucine or N-methyl-leucine;
*Vₐ* is valine or N-methyl-valine;
*Fₐ* and *F_{b}* are, independently at each occurrence, selected from phenylalanine, N-methyl-phenylalanine, halogenated phenylalanine, tyrosine, and N-methyl-tyrosine, preferably selected from phenylalanine and N-methyl-phenylalanine;
*A* is, independently at each occurrence, selected from alanine, N-methyl-alanine, leucine, N-methyl-leucine, valine, N-methyl-valine, glycine, and N-methyl-glycine, preferably is alanine;
*E* is selected from glutamic acid, N-methyl-glutamic acid, aspartic acid, N-methyl-aspartic acid, glutamine, N-methyl-glutamine, asparagine, N-methyl-asparagine, alanine, N-methyl-alanine, glycine, and N-methyl-glycine, preferably is glutamic acid;
X₅ is selected from aspartic acid, glutamic acid, asparagine, aspartic acid, alanine, and glycine, preferably is aspartic acid;
X₆, X₇, and X₈ are, independently at each occurrence, selected from norleucine, leucine, phenylalanine, methionine, tryptophan, tyrosine, threonine, alanine, isoleucine, valine, and modified forms thereof e.g. N-methylated forms thereof, preferably selected from norleucine, leucine, and phenylalanine;
*N* is selected from asparagine, glutamine and alanine, preferably is asparagine;
*K* is selected from lysine, modified lysine e.g. Lys(Ac), Lys(N₃), and Lys(biotin), alanine, norleucine, ornithine, modified ornithine e.g. Orn(Ac), diaminobutyric acid, diaminopropionic acid, arginine, and modified arginine e.g. Arg(Me)2, preferably is lysine;
*G* is, independently at each occurrence, selected from glycine and alanine, preferably is glycine;
*I* is, independently at each occurrence, selected from isoleucine, leucine, norleucine, and valine, preferably is isoleucine;
X₉ is selected from glycine, alanine, and serine, preferably is glycine;
X₁₀ is selected from leucine, alanine, isoleucine, threonine, norleucine, and valine, preferably is leucine; and
X₁₁ is selected from norleucine, leucine, phenylalanine, methionine, tryptophan, tyrosine, threonine, alanine, isoleucine, and valine, preferably is norleucine;
V is, independently at each occurrence, valine;
wherein at least one of *Lₐ, Vₐ,Fₐ*, and *F_{b}* is an N-methylated amino acid;
and pharmaceutically acceptable salts, esters, solvates, polymorphs and modified forms thereof.

3. The peptide according to any one of claims 1 and 2, having an amino acid sequence according to any one of formulae 3-5
QK*LₐVₐFₐF_{b}*AED-X₆X₇X₈-NKGAIIGLNleVGGVV (formula 3)
*FₐF_{b}*AED-X₆X₇X₈-NKGAIIGLNleVGGVV (formula 4)
QK*LₐVₐFₐF_{b}*AED-X₆X₇X₈-NKGAIIGLNleVG (formula 5)
wherein,
Q is glutamine;
K is, independently at each occurrence, lysine;
*Lₐ* is leucine or N-methyl-leucine;
*Vₐ* is valine or N-methyl-valine;
*Fₐ* and *F_{b}* are, independently at each occurrence, phenylalanine or N-methyl-phenylalanine;
A is, independently at each occurrence, alanine;
E is glutamic acid;
D is aspartic acid;
X₆, X₇, and X₈ are, independently at each occurrence, selected from norleucine, leucine, phenylalanine, methionine, tryptophan, tyrosine, threonine, alanine, isoleucine, and valine, preferably selected from norleucine, leucine, and phenylalanine;
N is asparagine;
G is, independently at each occurrence, glycine;
I is, independently at each occurrence, isoleucine;
L is leucine;
Nle is norleucine;
V is, independently at each occurrence, valine;
wherein at least one of *Lₐ, Vₐ,Fₐ*, and *F_{b}* is an N-methylated amino acid;
and pharmaceutically acceptable salts, esters, solvates, polymorphs and modified forms thereof.

4. The peptide according to any one of the foregoing claims, wherein X₆, X₇, and X₈ are the same amino acid selected from norleucine, leucine, phenylalanine, methionine, tryptophan, tyrosine, threonine, alanine, isoleucine, and valine, preferably selected from norleucine, leucine, and phenylalanine.

5. The peptide according to any one of the foregoing claims, having an amino acid sequence according to any one of formulae 6-8,
QK*LₐVₐFₐF_{b}*AEDNleNleNleNKGAIIGLNleVGGVV (formula 6),
QK*LₐVₐFₐF_{b}*AEDLLLNKGAIIGLNleVGGVV (formula 7),
QK*LₐVₐFₐF_{b}*AEDFFFNKGAIIGLNleVGGVV (formula 8),
wherein,
Q is glutamine;
K is, independently at each occurrence, lysine;
*Lₐ* is leucine or N-methyl-leucine;
*Vₐ* is valine or N-methyl-valine;
*Fₐ* and *F_{b}* are, independently at each occurrence, phenylalanine or N-methyl-phenylalanine;
A is, independently at each occurrence, alanine;
E is glutamic acid;
D is aspartic acid;
Nle is norleucine;
N is asparagine;
G is, independently at each occurrence, glycine;
I is, independently at each occurrence, isoleucine;
L is, independently at each occurrence, leucine;
V is, independently at each occurrence, valine;
F is, independently at each occurrence, phenylalanine;
wherein at least one of *Lₐ*, *V_{a,}Fₐ*, and *F_{b}* is an N-methylated amino acid;
and pharmaceutically acceptable salts, esters, solvates, polymorphs and modified forms thereof.

6. The peptide according to any one of claims 2-5, wherein two or more of Lₐ, V_{a,} Fₐ, and F_{b} are an N-methylated amino acid;
wherein, preferably, *Lₐ* and *Vₐ*; *Lₐ* and *Fₐ*; *Lₐ* and *F_{b}*; *Vₐ* and *Fₐ*; *Vₐ* and *F_{b}*; *Fₐ* and *F_{b}*; *Lₐ, Vₐ*, and *Fₐ* ; *Lₐ, Vₐ,* and *F_{b}*; *Lₐ, Fₐ,* and *F_{b}*; *V_{a,} Fₐ*, and *F_{b}*; or *Lₐ, V_{a,} Fₐ*, and *F_{b}* are N-methylated amino acids;
wherein, more preferably, at least *Lₐ* and *Fₐ*, or at least *Vₐ* and *F_{b}*, are an N-methylated amino acid.

7. The peptide according to any one of the foregoing claims, wherein said peptide has an amino acid sequence according to any one of formulae 9-17,
QK(N-methyl-L)V(N-methyl-F)FAEDNleNleNleNKGAIIGLNleVGGVV (formula 9),
QKL(N-methyl-V)F(N-methyl-F)AEDNleNleNleNKGAIIGLNleVGGVV (formula 10),
QK(N-methyl-L)V(N-methyl-F)FAEDLLLNKGAIIGLNleVGGVV (formula 11),
QKL(N-methyl-V)F(N-methyl-F)AEDLLLNKGAIIGLNleVGGVV (formula 12),
QK(N-methyl-L)V(N-methyl-F)FAEDFFFNKGAIIGLNleVGGVV (formula 13),
QKL(N-methyl-V)F(N-methyl-F)AEDFFFNKGAIIGLNleVGGVV (formula 14),
QK(N-methyl-L)(N-methyl-V)(N-methyl-F)(N-methyl-F)AEDFFFNKGAIIGLNleVGGVV (formula 15),
QK(N-methyl-L)(N-methyl-V)(N-methyl-F)(N-methyl-F)AEDLLLNKGAIIGLNleVGGVV (formula 16),
QK(N-methyl-L)(N-methyl-V)(N-methyl-F)(N-methyl-F)AEDNleNleNleNKGAIIGLNleVGGVV (formula 17),
wherein,
Q is glutamine;
K is, independently at each occurrence, lysine;
L is, independently at each occurrence, leucine;
V is, independently at each occurrence, valine;
F is, independently at each occurrence, phenylalanine;
A is, independently at each occurrence, alanine;
E is glutamic acid;
D is aspartic acid;
Nle is, independently at each occurrence, norleucine;
N is asparagine;
G is, independently at each occurrence, glycine;
I is, .3independently at each occurrence, isoleucine;
and pharmaceutically acceptable salts, esters, solvates, polymorphs and modified forms thereof.

8. The peptide according to any one of the foregoing claims, wherein said peptide consists of a sequence according to any one of formulae 1-17.

9. A pharmaceutical composition comprising a peptide according to any one of the foregoing claims and a pharmaceutically acceptable excipient.

10. A heterocomplex comprising a peptide according to any one of claims 1-8, and amyloid-β peptide and/or islet amyloid polypeptide.

11. The peptide according to any one of claims 1-8, the pharmaceutical composition according to claim 9, or the heterocomplex according to claim 10, for use in a method of preventing or treating Alzheimer's disease and/or for use in a method of preventing or treating type 2 diabetes;
wherein, optionally, said method comprises administering an effective amount of said peptide, of said composition, or of said heterocomplex to a subject in need thereof.

12. The peptide according to any one of claims 1-8, the pharmaceutical composition according to claim 9, or the heterocomplex according to claim 10, for use in a method of diagnosing Alzheimer's disease and/or for use in a method of diagnosing type 2 diabetes;
wherein, optionally, said method comprises administering an effective amount of said peptide, of said composition, or of said heterocomplex to a subject to be tested for Alzheimer's disease and/or type 2 diabetes.

13. The peptide, the pharmaceutical composition, or the heterocomplex for use according to any one of claims 11-12, wherein said peptide is linked to or administered together with a suitable reporter molecule that allows detection of Aβ40(42), islet amyloid polypeptide (IAPP), and/or amyloid aggregates or co-aggregates thereof by a suitable detection methodology, such as positron emission tomography (PET), nuclear magnetic resonance (NMR), magnetic resonance imaging (MRI), and PET-MRI and wherein said subject, after administration of said peptide, is subjected to said suitable detection methodology, such as PET, NMR, MRI, PET-MRI.

14. A kit for the *in vitro* or *in vivo* detection of amyloid fibrils or aggregates, or for the diagnosis of Alzheimer's disease and/or type 2 diabetes in a subject, said kit comprising the peptide according to any one of claims 1-8, the pharmaceutical composition according to claim 9, or the heterocomplex according to claim 10, in a freeze-dried form in a suitable container, a buffered solvent in a separate container for reconstitution of said peptide in solution, and, optionally, means to dispense said peptide once reconstituted in solution, such as a syringe or pipette.

15. Use of the peptide according to any one of claims 1-8 or of the heterocomplex according to claim 10, in an *in vitro* assay, such as an enzyme linked immunosorbent assay (ELISA), a radioimmuno assay (RIA), or a Dot/Slot blot assay, for the detection of monomeric islet amyloid polypeptide (IAPP), monomeric Aβ40(42), amyloid fibrils, amyloid aggregates, and/or amyloid co-aggregates.
